# EUROPÄISCHE PATENTSCHRIFT

(11) **EP 2 176 416 B1**
(45) Veröffentlichungstag und Bekanntmachung des Hinweises auf die Patenterteilung: **16.03.2016**
(21) Anmeldenummer: 08786637.2
(22) Anmeldetag: 30.07.2008
(51) Int. Cl.: C12N 15/82, C12N 9/10

(54) **ELONGASEN UND VERFAHREN ZUR HERSTELLUNG MEHRFACH UNGESÄTTIGTER FETTSÄUREN IN TRANSGENEN ORGANISMEN**
ELONGASES AND METHODS FOR PRODUCING POLYUNSATURATED FATTY ACIDS IN TRANSGENIC ORGANISMS
ÉLONGASES ET PROCÉDÉS DE PRODUCTION D'ACIDES GRAS POLYINSATURÉS DANS DES ORGANISMES TRANSGÉNIQUES

(30) Priorität: 31.07.2007 EP 07113547
(43) Veröffentlichungstag der Anmeldung: 21.04.2010
(73) Patentinhaber: BASF Plant Science GmbH, 67056 Ludwigshafen (DE); Bioriginal Food & Science Corporation, Saskatoon Saskatchewan S7J OR1 (CA)
(72) Erfinder: BAUER, Jörg, 67117 Limburgerhof (DE); QIU, Xiao, Saskatoon Saskatchewan S7N 5A8 (CA); VRINTEN, Patricia, Saskatoon Saskatchewan S7J 4H5 (CA)
(74) Vertreter: Herzog, Fiesser & Partner Patentanwälte PartG mbB
(86) Internationale Anmeldenummer: PCT/EP2008/060007
(87) Internationale Veröffentlichungsnummer: WO 2009/016208

(56) Entgegenhaltungen:
- WO-A-2006/100241
- WO-A-2008/022963
- WO-A2-01/59128
- WO-A2-02/08401
- WARUDE DNYANESHWAR ET AL: "POLYUNSATURATED FATTY ACIDS: BIOTECHNOLOGY" CRC CRITICAL REVIEWS IN BIOTECHNOLOGY, CRC PRESS, BOCA RATON, FL, US, Bd. 26, Nr. 2, 1. Januar 2006 (2006-01-01), Seiten 83-93, XP008070892 ISSN: 0738-8551
- TRUKSA MARTIN ET AL: "Metabolic engineering of plants to produce very long-chain polyunsaturated fatty acids" TRANSGENIC RESEARCH, LONDON, GB, Bd. 15, Nr. 2, 1. April 2006 (2006-04-01), Seiten 131-137, XP002416460 ISSN: 0962-8819
- O'BRIEN D J ET AL: "PRODUCTION OF EICOSAPENTAENOIC ACID BY THE FILAMENTOUS FUNGUS PYTHIUM IRREGULARE" APPLIED MICROBIOLOGY AND BIOTECHNOLOGY, SPRINGER VERLAG, BERLIN, DE, Bd. 40, Nr. 2/03, 1. Januar 1993 (1993-01-01), Seiten 211-214, XP009014029 ISSN: 0175-7598

## Beschreibung

Die vorliegende Erfindung betrifft Polynukleotide aus *Pythium irregulare, Rhizopus oryzae* und *Euglena gracilis,* die Elongasen kodieren und zur rekombinanten Herstellung von mehrfach ungesättigten Fettsäuren eingesetzt werden können. Weiterhin betrifft die Erfindung Vektoren, Wirtszellen und transgene nicht-humane Organismen, die die erfindungsgemäßen Polynukleotide enthalten, sowie die von den Polynukleotiden kodierten Polypeptide. Die Erfindung betrifft zudem Antikörper gegen die erfindungsgemäßen Polypeptide. Schließlich betrifft die Erfindung noch Herstellungsverfahren für die mehrfach ungesättigten Fettsäuren und für Öl-, Lipid- und Fettsäurezusammensetzungen und deren Verwendung als Arzneimittel, Kosmetik, Nahrungsmittel, Futtermittel, vorzugsweise Fischfutter oder Nahrungsergänzungsmittel.

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfach ungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar.

Mehrfach ungesättigte langkettige ω-3-Fettsäuren wie Eicosapentaensäure (= EPA, C20:5^{Δ5,8,11,14,17}) oder Docosahexaensäure (= DHA, C22:6^{Δ4,7,10,13,16,19}) sind wichtige Komponenten der menschlichen Ernährung aufgrund ihrer verschiedenen Rollen in der Gesundheit, die Aspekte wie die Entwicklung des kindlichen Gehirns, die Funktionalität des Auges, die Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) der Babynahrung zur Erhöhung des Nährwertes zugesetzt. Der ungesättigten Fettsäure DHA wird dabei ein positiver Effekt in Bezug auf die Entwicklung und Aufrechterhaltung von Gehirnfunktionen zugeschrieben.
Im folgenden werden mehrfach ungesättigte Fettsäuren als PUFA, PUFAs, LCPUFA oder LCPUFAs bezeichnet (**p**oly **u**nsaturated **f**atty **a**cids, **PUFA,** mehrfach ungesättigte Fettsäuren; **l**ong **c**hain **p**oly **u**nsaturated **f**atty **a**cids, **LCPUFA,** langkettige mehrfach ungesättigte Fettsäuren).

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, C20:4^{Δ5,8,11,14}), Dihomo-γ-linolensäure (C20:3^{Δ8,11,14}) oder Docosapentaensäure (DPA, C22:5^{Δ7,10,13,16,19}) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch, oder Algen.

Je nach Anwendungszweck werden Öle mit gesättigten oder ungesättigten Fettsäuren bevorzugt. So werden z.B. in der humanen Ernährung Lipide mit ungesättigten Fettsäuren, speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche, speziell chronisch entzündliche Prozesse im Rahmen immunologischer Erkrankungen wie rheumatoider Arthritis lassen sich durch ω-3-Fettsäuren positiv beeinflussen. Sie werden deshalb speziell diätischen Lebensmitteln zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.
ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, sowie den Thromoxanen und Leukotrienen, die sich von der Arachidonsäure und der Eicosapentaensäure ableiten. Eicosanoide (sog. PG₂-Serie), die aus ω-6-Fettsäuren gebildet werden, fördern in der Regel Entzündungsreaktionen, während Eicosanoide (sog. PG₃-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben. Deren Anwendung zur Produktion in transgenen Organismen wird z. B. in WO98/46763 WO98/46764, oder WO9846765 beschrieben. Dabei wird auch die Expression verschiedener Desaturasen und Bildung polyungesättigter Fettsäuren beschrieben und beansprucht, siehe z.B. WO99/64616 oder WO98/46776. Bezüglich der Effektivität der Expression von Desaturasen und ihrem Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus w-3- und ω-6-Fettsäuren erhalten.

Besonders geeignete Mikroorganismen zur Herstellung von PUFAs sind Mikroorganismen, beispielsweise Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moose wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden wann immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Zudem fallen diese in der Regel je nach verwendetem Mikroorganismus als Fettsäuregemische aus beispielsweise EPA, DPA und ARA an.

Für die Synthese von Arachidonsäure, Eicosapentaensäure (EPA) und Docosahexaensäure (DHA) werden verschiedene Synthesewege diskutiert. So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731,1997).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453, (1999).

Warude et al., 2006, bespricht Desaturase- und Elongaseenzyme, die in der Biosynthese von PUFAs involviert sind.

Truksa et al., 2006 diskutiert verschiedene Ansätze zur Rekonstitution von Biosynthesewegen, die zur Herstellung von Arachidonsäure, Eicosapentaensäure und Docosahexaensäure in Ölpflanzen verwendet werden können.

WO 2006/100241 beschreibt ein Verfahren zur Herstellung von PUFAs in transgenen Pflanzen, wobei Polypeptide mit Δ6-Desaturase-, Δ6-Elongase-, Δ5-Desaturase-, Δ5-Elongase-, Δ4-Desaturase-, Δ12-Desaturase- und/oder ω3-Desaturaseaktivität verwendet werden.

O'Brien et al., 1993 beschreibt die Herstellung von Eicosapentaensäure durch den Fadenpilz *Pythium irregulare.*

WO 01/59128 beschreibt Elongasegene und deren Verwendung zur Herstellung von ungesättigten Fettsäuren.

WO 02/08401 beschreibt die Identifizierung von Genen, die mehrfach ungesättigte Fettsäuren elongieren können, sowie deren Verwendung.

Eine Modifikation des in Zank et al. und in Sakuradani et al. beschriebenen Weges über Δ6-Desaturase-, Δ6-Elongase, Δ5-Desaturase, Δ5-Elongase, Δ4-Desaturase ist der Sprecher-Syntheseweg (Sprecher 2000, Biochim. Biophys. Acta 1486:219-231) in Säugetieren. Anstelle der Δ4-Desaturierung erfolgt hier ein weiterer Elongationsschritt auf C₂₄, eine weitere Δ6-Desaturierung und abschliessend eine β-Oxidation auf die C₂₂-Kettenlänge. Für die Herstellung in Pflanzen und Mikroorganismen ist der sogenannte Sprecher-Syntheseweg allerdings nicht geeignet, da die Regulationsmechanismen bisher nicht aufgeklärt werden konnten.

Die mehrfach ungesättigten Fettsäuren können entsprechend ihrem Desaturierungsmuster in zwei große Klassen, in ω-6- oder ω-3-Fettsäuren eingeteilt werden, die metabolisch und funktionell unterschiedliche Aktivitäten haben.

Als Ausgangsprodukt für den ω-6-Stoffwechselweg fungiert die Fettsäure Linolsäure (1 8:2^{Δ9,12}), während der ω-3-Weg über Linolensäure (18:3^{Δ9,12,15}) abläuft. Linolensäure wird dabei durch Aktivität einer ω-3-Desaturase gebildet (Tocher et al. 1998, Prog. Lipid Res. 37, 73-117 ; Domergue et al. 2002, Eur. J. Biochem. 269, 4105-4113). Säugetiere und damit auch der Mensch verfügen über keine entsprechende Desaturaseaktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen mehrfach ungesättigten Fettsäuren Arachidonsäure (= ARA, 20:4^{Δ5,8,11,14}), eine ω-6-Fettsäure und die beiden ω-3-Fettsäuren Eicosapentaen- (= EPA, 20:5^{Δ5,8,11,14,17}) und Docosahexaensäure (DHA, 22:6^{Δ4,7,10,13,17,19}) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthritis (Cleland und James 2000, J. Rheumatol. 27, 2305-2307).

Die Verlängerung von Fettsäuren durch Elongasen um 2 bzw. 4 C-Atome ist für die Produktion von C₂₀- bzw. C₂₂-PUFAs von entscheidender Bedeutung. Dieser Prozess verläuft über 4 Stufen. Der erste Schritt stellt die Kondensation von Malonyl-CoA an das Fettsäure-Acyl-CoA durch die Ketoacyl-CoA-Synthase (KCS, im weiteren Text als Elongase bezeichnet). Es folgt dann ein Reduktionschritt (Ketoacyl-CoA-Reduktase, KCR), ein Dehydratationsschritt (Dehydratase) und ein abschliessender Reduktionsschritt (enoyl-CoA-Reduktase). Es wurde postuliert, dass die Aktivität der Elongase die Spezifität und Geschwindigkeit des gesamten Prozesses beeinflusst (Millar and Kunst, 1997 Plant Journal 12:121-131).

In der Vergangenheit wurden zahlreiche Versuche unternommen, Elongase Gene zu erhalten. Millar and Kunst, 1997 (Plant Journal 12:121-131) und Millar et al. 1999, (Plant Cell 11:825-838) beschreiben die Charakterisierung von pflanzlichen Elongasen zur Synthese von einfach ungesättigten langkettigen Fettsäuren (C22:1) bzw. zur Synthese von sehr langkettigen Fettsäuren für die Wachsbildung in Pflanzen (C₂₈-C₃₂). Beschreibungen zur Synthese von Arachidonsäure und EPA finden sich beispielsweise in WO0159128, WO0012720, WO02077213 und WO0208401. Die Synthese von mehrfach ungesättigter C24 Fettsäuren ist beispielsweise in Tvrdik et al 2000, JCB 149:707-717 oder WO0244320 beschrieben. Weitere Elongasen sind beispielsweise beschrieben in WO03078639, WO05012316 oder WO07061845.

Höhere Pflanzen enthalten mehrfach ungesättigte Fettsäuren wie Linolsäure (C18:2) und Linolensäure (C18:3). Dagegen kommen ARA, EPA und DHA im Samenöl höherer Pflanzen gar nicht oder nur in Spuren vor (E. Ucciani: Nouveau Dictionnaire des Huiles Vegetales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Ein möglicher Weg führt über gentechnische Methoden, in denen Gene kodierend für Enzyme der Biosynthese von LCPUFAs in Ölsaaten eingeführt und exprimiert werden. Dies sind Gene, die beispielsweise für Δ-6-Desaturasen, Δ-6-Elongasen, Δ-5-Desaturasen oder Δ-4-Desaturasen codieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert werden.

Erste transgene Pflanzen, die Gene kodierend für Enzyme der LCPUFA-Biosynthese enthalten und exprimieren und LCPUFAs produzieren wurden beispielsweise in DE 102 19 203 (Verfahren zur Herstellung mehrfach ungesättigter Fettsäuren in Pflanzen) beschrieben. Diese Pflanzen produzieren allerdings LCPUFAs in Mengen, die für eine Aufarbeitung der in den Pflanzen enthaltenen Öle noch weiter optimiert werden müssen.

Um eine Anreicherung der Nahrung und des Futters mit diesen mehrfach ungesättigten Fettsäuren zu ermöglichen, besteht daher ein großer Bedarf an Mitteln und Maßnahmen für eine einfache, kostengünstige Herstellung dieser mehrfach ungesättigten Fettsäuren speziell in eukaryontischen Systemen.

Die der vorliegende Efindung zugrunde liegende Aufgabe wäre also die Bereitstellung von solchen Mitteln und Maßnahme. Die Aufgabe wird durch die Ausführungsformen gelöst, die in den Patentansprüchen und im Folgenden beschrieben werden.

Die vorliegende Erfindung betrifft ein Polynucleotid umfassend eine Nukleinsäuresequenz, die ausgewählt ist aus der Gruppe bestehend aus:
(a) Nukleinsäuresequenz wie in einer der SEQ ID No. 1 oder 3 gezeigt;
(b) Nukleinsäuresequenz, die ein Polypeptid kodiert, das eine Aminosäuresequenz wie in einer der SEQ ID No. 2 oder 4 gezeigt aufweist;
(c) Nukleinsäuresequenz, die mindestens 70% identisch zu einer der Nukleinsäuresequenzen aus (a) oder (b) ist und ein Polypeptid mit einer Δ-6-Elongase Aktivität kodiert, wobei das Polypeptid die Fettsäure cis-Vakzensäure (C18:1Δ11), Arachidon¬säure (C20:4A5,8,11,14) und/oder Eicosapentaensäure (C20:5Δ5,8,11,14,17) elongieren kann; und
(d) Nukleinsäuresequenz für ein Fragment einer Nukleinsäure aus (a), (b) oder (c), wobei das Fragment ein Polypeptid mit einer Δ-6-Elongase Aktivität kodiert, wobei das Polypeptid die Fettsäure cis-Vakzensäure (C18:1Δ11), Arachidon¬säure (C20:4Δ5,8,11,14) und/oder Eicosapentaensäure (C20:5Δ5,8,11,14,17) elongieren kann.

Der Begriff "Polynucleotid" betrifft erfingdungsgemäß Polynukleotide, die Nukleinsäuresequenzen umfassen, die Polypeptide mit Elongase Aktivität kodieren.

Die Elongase Aktivitäten werden vorzugsweise für die Biosynthese von Lipiden oder Fettsäuren benötigt. Besonders bevorzugt handelt es sich um die folgenden Elongase Aktivitäten: Δ-5-Elongase, Δ-6-Elongase oder Δ-9-Elongase. Die Elongasen sind bevorzugt an der Synthese mehrfach ungesättigter Fettsäuren (PUFAs) und besonders bevorzugt an der Synthese lankettiger PUFAs (LCPUFAs) beteiligt. Geeignete Nachweissysteme für die erfindungsgemäßen Elongase Aktivitäten sind in den Beispielen oder in WO2005/083053 beschrieben. Besonders bevorzugt handelt es sich bei den zuvor genannten Aktivitäten hinsichtlich Substratspezifitäten und Umsetzungsraten um die der jeweiligen Enzyme aus *Pythium irregulare, Rhizopus oryzae* und *Euglena gracilis.* Die erfindungsgemäßen spezifischen Polynukleotide, d.h. die Polynukleotide mit einer Nukleinsäuresequertz gemäß SEQ ID Nr. 1 oder 3, wurden aus *Pythium irregulare* gewonnen. Beschrieben sind Polynukleotide aus *Rhizopus oryzae* (SEQ ID Nr. 5) oder *Euglena gracilis* (SEQ ID Nr. 7).

Es versteht sich, dass die zuvor genannten spezifischen Sequenzen angesichts des degenerierten genetischen Codes auch verändert werden können wobei von den veränderten Polynukleotiden immer noch Polypeptide mit einer Aminosäuresequenz gemäß einer der SEQ ID Nummern 2 oder 4 kodiert werden, die die zuvor genannten Elongase Aktivitäten aufweisen. Beschrieben sind Polypeptide mit einer Aminosäuresequenz gemäß SEQ ID Nummern 6 oder 8.

Der Begriff "Polynukleotid" umfasst auch Varianten der zuvor genannten spezifischen Polynukleotide. Bei diesen kann es sich um homologe, orthologe oder paraloge Sequenzen handeln. Solche Varianten umfassen Nukleinsäuresequenzen, die mindesten einen Basenaustausch, eine Basenaddition oder eine Basendeletion aufweisen, wobei die Varianten immer noch ein Polypeptid mit der zuvor genannten biologischen Aktivität der jeweiligen Ausgangssequenz kodierten sollen. Varianten umfassen Polynukleotide, die mit den zuvor genannten Polynukleotiden hybridisieren können, bevorzugt unter stringenten Bedingungen. Besonders bevorzugte stringente Bedingungen sind dem Fachmann bekannt und lassen sich in Current Protocols in Molecular Biology, John Wiley & Sons, N. Y. (1989), 6.3.1-6.3.6., finden. Ein bevorzugtes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheidet. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Harnes und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können. Alternativ können Varianten der erfindungsgemäßen spezifischen Polynukleotide auch durch Potymerasekettenreaktion (PCR) basierte Verfahren bereitgestellt werden. Hierzu können zunächst Primer von konservierten Sequenzen (z.B. Sequenzen, die funktionelle Domänen im Polypeptid kodieren) abgeleitet werden. Konservierte Sequenzen können durch Sequenzvergleiche mit Polynuklaofiden, die Polypeptide ähnlicher Aktivität kodieren, ermittelt werden. Als Matrize kann DNA oder cDNA aus Bakterien, Pilzen, Pflanzen oder Tieren vedrwendet werden. DNA Fragmente, die durch die PCR erhalten wurde, können zum Screening von entsprechenden gerromischen oder cDNA Bibliotheken verwendet werden um, - falls erforderlich - den kompletten offenen Leserahi'nen des Polynucleotids zu isolieren und durch Sequenzierung zu ermitteln.

Bevorzugte Varianten umfassen Polyrrukleotide, die eine Nukleinsäuresequenz umfassen, die mindestens 70 %, mindestens 75 %,
mindestens 80%, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% identisch mit einer der zuvor genannten spezifischen Nukleinsäuresequenzen, also einer Nukleinsäuresequenz wie in SEI ID Nr. 1 oder 3 gezeigt ist und ein Polypeptid mit der jeweiligen biologischen Aktivität kodiert.

Ebenso bevorzugt umfasst sind Polynucleotidvarianten, die Nukleinsäuresequenzen umfassen, die ein Polypeptid mit einer Aminosäuresequenz kodieren, die mindestens 70 %, mindestens 75 %, mindestens 80 %, mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% identisch mit einer der zuvor genannten spezifischen Aminosäuresequenzen, also einer Aminosäuresequenz wie in SEQ ID Nr. 2 oder 4 gezeigt ist und wobei das Polypeptid die jeweilige biologische Aktivität der Ausgangssequenz aufweist.

Der Prozentsatz identischer Nukleotide oder Aminosäuren bezieht sich vorzugsweise auf einen Sequenzabschnitt von mindestens 50% der zu vergleichenden Sequenzen und besonders bevorzugt über die gesamte Länge der zu vergleichenden Sequenzen. Eine Vielzahl von Programmen, die Algorithmen für solche Vergleiche implementieren ist im Stand der Technik beschrieben und kommerziell erhältlich. Insbesondere sei auf die Algorithmen von Needleman und Wunsch oder Smith und Waterman verwiesen, die besonders zuverlässige Ergebnisse liefern. Diese Algorithmen können vorzugsweise durch die folgenden Programme implementiert werden: PileUp (J. Mol. Evolution., 25, 351-360, 1987, Higgins et al., CABIOS, 5 1989: 151-153), Gap und BestFit (Needleman and Wunsch (J. Mol. Biol. 48; 443-453 (1970)) und Smith und Waterman (Adv. Appl. Math. 2; 482-489 (181))), als Teil der GCG software [Genetics Computer Group, 575 Science Drive, Madison, Wisconsin, USA 53711 (1991)]. Besonders bevorzugt wird der Prozentsatz (%) der Sequenzidentität im Rahmen der vorliegenden Erfindung mit dem GAP Programm über die gesamte Sequenz mit folgenden festgelegten Größen bestimmt: Gap Weight: 50, Length Weight: 3. Average Match: 10.000 und Average Mismatch: 0.000.

Ein Polynucleotid, das lediglich ein Fragment der zuvor genannten Nukleinsäuresequenzen umfasst, ist auch ein erfindungsgemäßes Polynucleotid. Das Fragment soll hierbei ein Polypeptid kodieren, das die biologische Aktivität der Ausgangssequenz bzw. des davon kodierten Polypeptids aufweist. Polypeptide, die von solchen Polynukleotiden kodiert werden, umfassen oder bestehen daher aus Domänen der zuvor genannten spezifischen Polypeptide (Ausgangspolypeptide), die die biologische Aktivität vermitteln. Ein Fragment im Sinne der Erfindung umfasst vorzugsweise mindestens 50, mindestens 100, mindestens 250 oder mindestens 500 aufeinanderfolgende Nucleotide der zuvor genannten spezifischen Sequenzen oder kodiert eine Aminosäuresequenz umfassend mindestens 20, mindestens 30, mindestens 50, mindestens 80, mindestens 100 oder mindestens 150 aufeinanderfolgende Aminosäuren einer der zuvor genannten spezifischen Aminosäuresequenzen und vermittelt biologische Aktivität, vorzugsweise Elongase Aktivität, wie zuvor beschrieben.

Die erfindungsgemäßen Polynucleotidvarianten weisen vorzugsweise mindestens 10%, mindestens 20%, mindestens 30%, mindestens 40%, mindestens 50%, mindestens 60%, mindestens 70%, mindestens 80% oder mindestens 90% der jeweiligen biologischen Aktivität des Polypeptids, das von der Ausgangssequenz kodiert wird, auf. D.h. die Polypeptide, die von den erfindungsgemäßen Polynukleotiden kodiert werden, können am Stoffwechsel von zum Aufbau von Fettsäuren, Fettsäureestern wie Diacylglyceride und/oder Triacylglyceride in einem Organismus, bevorzugt in einer Pflanze oder Pflanzenzelle, notwendigen Verbindungen oder am Transport von Molekülen über Membranen teilnehmen, wobei C₁₈-, C₂₀- oder C₂₂-Kohlenstoffketten im Fettsäuremolekül mit Doppelbindungen an mindestens zwei, bevorzugt drei, vier, fünf oder sechs Stellen gemeint sind.

Die erfindungsgemäßen Polynukleotide umfassen entweder die zuvor genannten spezifischen Nukleinsäuresequenzen oder bestehen ausschließlich daraus. D.h. die erfindungsgemäßen Polynukleotide können grundsätzlich auch noch weitere Nukleotide umfassen. Dabei kann es sich vorzugsweise um 3' oder 5' untranslatierte Bereiche der genomischen Nukleinsäuresequenz handeln. Diese bestehen vorzugsweise aus mindestens 100, 200 oder 500 Nucleotiden am 5' Terminus und mindestens 20, 50 oder 100 Nukleotiden am 3' Terminus des kodierenden Bereichs.

Weitere Polynukleotide, die zusätzliche Nukleinsäuresequenzen umfassen, sind solche, die für Fusionsproteine kodieren. Solche Fusionsproteine können zusätzlich zu den zuvor genannten Polypeptiden weitere Polypeptide bzw. Polypeptidanteile kodieren. Bei dem zusätzlichen Polypeptid bzw. Polypeptidanteil kann es sich um weitere Enzyme der Lipid- oder Fettsäure Biosynthese handeln. Auch denkbar sind Polypeptide, die als Marker für die Expression dienen können (Grün-, Gelb-, Rot-, Blau-fluoreszierende Proteine, alkalische Phosphatase u.a.) bzw. sogenannte "tags" als Marker oder Hilfe für die Aufreinigung (z.B. FLAG-tags, 6-Histidin-tags, MYC-tags u.a.).

Polynukleotid-Varianten können von verschiedenen natürlichen oder künstlichen Quellen isoliert werden. Beispielsweise können sie künstlich durch in vitro oder in vivo Mutagenese erzeugt werden. Homologe oder Orthologe der spezifischen Sequenzen können aus den verschiedensten Tieren, Pflanzen oder Mikroorganismen gewonnen werden. Vorzugsweise werden sie aus Algen gewonnen. Bevorzugt sind Algen wie Isochrysis, Euglena oder Crypthecodinium, Algen/Diatomeen wie Thalassiosira, Phaeodactylum oder Thraustochytrium, Pythium, Moose wie Physcomitrella oder Ceratodon, ganz besonders bevorzugt sind die Algen der Gattungen Euglena oder die Diatomeen aus der Klasse der Oomycota wie die Gattungen Pythium oder Phytophtora oder Pilze der Abteilung der Zygomycota aus den Gattungen Rhizopus. Die Polynukleotide können ebenfalls bevorzugt aus höheren Pflanzen gewonnen werden, wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Rhizopus, Mucor oder Mortierella, Bakterien wie Shewanella, Hefen oder Tieren wie Nematoden z.B. Caenorhabditis, Insekten oder Fische. Die Polynucleotid- Varianten stammen ebenfalls bevorzugt aus einem Tier aus der Ordnung der Vertebraten. Besonders bevorzugt stammen die Polynukleotide aus der Klasse der Vertebrata; Euteleostomi, Actinopterygii; Neopterygii; Teleostei; Euteleostei, Protacanthopterygii, Salmoniformes; Salmonidae bzw. Oncorhynchus und; ganz besonders bevorzugt, aus der Ordnung der Salmoniformes wie der Familie der Salmonidae wie der Gattung Salmo beispielsweise aus den Gattungen und Arten Oncorhynchus mykiss, Trutta trutta oder Salmo trutta fario. Die erfindungsgemäßen Polynukleotide können hierbei mittels molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lassen sich Polynukleotide oder Fragmente davon, durch Polymerasekettenreaktion (PCR) isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis der in den SEQ ID Nummern dargestellten Polynukleotid- und Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Die erfindungsgemäßen Polynukleotide können entweder als isolierte Polynukleotide (d.h. isoliert aus ihrem natürlichen Ursprung, z.B. dem genomischen Locus) oder in genetisch veränderter Form bereitgestellt werden (d.h. die Polynukleotide können auch an ihrem natürlichen genetischen Locus vorliegen, müssen dann aber genetisch verändert werden). Genetische Veränderungen der Polynukleotide an ihrem natürlichen genetischen Locus sind nicht auf Sequenzmodifikation im kodierenden Bereich beschränkt. Vielemehr umfassen solche genetischen Veränderungen auch Insertionen von regulatorischen Elementen, z.B. Promotor- oder Enhancer-Sequenzen, am oder in der Nähe des natürlichen genetischen Locus, die diesen beeinflussen, und z.B. in einem geänderten Expressionsverhalten resultieren. Ein isoliertes Polynucleotid umfasst vorzugsweise weniger als 5 kb, 4 kb, 3 kb, 2 kb, 1 kb, 0,5 kb oder 0,1 kb an Nukleinsäuresequenz, die natürlicherweise in seiner Nachbarschaft vorkommt Das erfindungsgemäße Polynucleotid kann als einzelsträngiges oder doppelsträngiges Nukleinsäuremolekül vorliegen und genomische DNA, cDNA oder RNA sein. Vorzugsweise besteht das erfindungsgemäße Polynukleotid aus RNA od DNA. Die erfindungsgemäßen Polynukleotide umfassen alle Orientierungen der in den SEQ ID Nummern aufgezeigten Sequenzen, d.h. auch komplementäre Stränge sowie reverse oder revers-komplementäre Orientterungen. Ferner umfasst der Begriff auch chemisch modifizierte Nucleinsäuren, wie die natürlich vorkommenden methylierten DNA Moleküle, oder artifizielle Nucleinsäuren z.B. biotinylierte Nukleinsäuren.

Beschrieben sind auch Oligonukleotide von mindestens 15 bp, bevorzugt mindestens 20 bp, mindestens 25 bp, mindestens 30 bp, mindestens 35 bp, oder mindestens 50 bp, die spezifisch mit einem der zuvor genannten Polynukleotide unter stringentan Bedingungen hybridisieren können. Die Oliguncleotide können aus DNA oder RNA oder beidem bestehen. Solche Oligonucleotide können als Primer für die PCR, als die Expression hemmende, antisense Oligonucleotide, für RNA Interferenz (RNAI) oder für Chimero- oder Genoplastie Ansätze verwendet werden. RNAi Verfahren sind beispielsweise in Flre et al., Nature (1998) 391:806-811; Fire, Trends Genet. 15, 358-363 (1999); Sharp, RNA interference 2001. Genes Dev. 15,485-490 (2001); Hammond et al. Nature Rev. Genet. 2, 1110-1119 (2001); Tuschl, Chem. Biochem, 2, 239-245 (2001); Hamilton et al., Science 286, 950-952 (1999); Hammond et al., Nature 404, 293-296 (2000); Zamore et al., Cell 101, 25-33 (2000); Bernstein et al., Nature 409, 363-366 (2001); Elbashir et al., Genes Dev. 15, 188-200 (2001); WO 01/29058; WO 99/32619; oder Elbashir et al., 2001 Nature 411: 494-498 beschrieben und dienen der Inhibierung der Genexpression durch Abbau der mRNA. Chimero- oder Genoplastie Ansätze dienen der in vivo Modifizierung (z.B. dem Einfügen von Punktmutationen) in Genen an deren endogenen Loci. Entsprechende Verfahren sind offenbart in USA5,565,350, US5,756,325, US5,871,984, US5,731,181, US5,795,972, US6,573,046, US6,211,351, US6,586,184, US6,271,360 und US6,479,292.

Die erfindungsgemäßen Elongasen haben gegenüber den humanen Elongasen die vorteilhafte Eigenschaft, dass sie C₂₂-Fettsäuren nicht zu den entsprechenden C24-Fettsäuren elongieren. Die erfindungsgemäßen Elongasen setzen bevorzugt nur ungesättigte C₁₈-, C₂₀- oder C₂₂-Fettsäuren um. Vorteilhaft werden durch die beschriebene Elongase
d5Elo(Eg) (SEQ ID Nr. 7 und 8) nur C₂₀-Fettsäuren mit einer Doppelbindung in Δ-5-Position umgesetzt, wobei ω-3-C₂₀ Fettsäuren bevorzugt werden (EPA). Weiterhin hat d5Elo(Eg) die Eigenschaft, dass
sie neben der Δ-5-Elongaseaktivität keine oder nur eine relativ geringe Δ-6-Elongaseaktivität aufweist Ferner setzt sie vorzugsweise in einem Hefefütterungskontext, in dem als Substrat EPA den Hefen zugesetzt wurde, mindestens 15 Gew.-% des zugesetzten EPAs zu Docosapentaensäure (DPA, C22:5^{Δ7,10.13.16.19}), stärker bevorzugt mindestens 20 Gew.-% und besonders stark bevorzugt mindestens 25 Gew.-% um. Wird als Substrat γ-Linolensäure (= GLA, C18:3^{Δ6,9,12}) gegeben, so wird diese bevorzugterweise gar nicht elongiert. Ebenfalls wird auch C18:3^{Δ5.9.12} nicht elongiert. Weniger als 60 Gew.-% des zugesetzten GLA zu Dihomo-γ-linolensäure (= C20:3^{Δ8.11.14}) wird umgesetzt, bevorzugt sogar weniger als 55 Gew.-%, besonders bevorzugt weniger als 50 Gel.-%, weniger als 45 Gew.-%, bzw. sogar weniger als 40 Gew.-%. In einer weiteren ganz bevorzugten Ausführungsform der Δ-5-Elongaseaktivität wird GLA nicht umgesetzt.

Die erfindungsgemäßen Elongasen d6Elo(Pir_1 und _2) (SEQ ID Nr. 1 bis 4) zeigen eine Substratspezifität für Δ6-desaturierte Fettsäuren. Überraschenderweise zeigt d6Elo(Pir) im Unterschied zu den bekannten Δ6-Elongasen auch eine Spezifität für C18:1Δ11 (Vakzensäure).

Vorteilhafterweise hat sich gezeigt, dass die erfindungsgemäßen Polynukleotide zur rekombinanten Herstellung mehrfach ungesättigter Fettsäuren in Wirtszellen und transgenen Organismen besonders effektiv eingesetzt weden können, Insbesondere können die von den erfindungsgemäßen Polynukleotiden kodierten Polypeptide mit Elongase Aktivität C₁₈-, C₂₀- und C₂₂-Fettsäuren mit ein, zwei, drei, vier oder fünf Doppelbindungen und bevorzugt mehrfach ungesättigte C₁₈-Fettsäuren mit ein, zwei oder drei Doppelbindungen wie C18:1^{Δ9}, C18:2^{Δ9.12}oder C18:3 ^{Δ9,12,15}, mehrfach ungesättigte C₂₀-Fettsäuren mit drei oder vier Doppelbindungen wie C20:3^{Δ8,11,14} oder C20:4^{Δ8,11,14,17} oder mehrfach ungesättigte C₂₂-Fettsäuren mit vier oder fünf Doppelbindungen wie C22:4^{Δ7,10,13,16} oder C22:5^{Δ7,10,13,16,19} umsetzen. Besonders bevorzugt können in diesem Zusammenhang die Δ-6-Desaturase mit SEQ ID Nr. 9 und 10, die Δ-5-Desaturase mit SEQ ID Nr. 11 und 12, die Δ-8-Desaturase mit SEQ ID Nr. 15 und 16, die Δ-12-Desaturase mit SEQ ID Nr. 17 und 18 und/oder die omega-3-Desaturase mit SEQ ID Nr. 13 und 14 eingesetzt werden. Je nach Fettsäure, die hergestellt werden soll, können verschiedene Kombinationen der erfindungsgemäßen Polynukleotide mit den zuvor genannten Desaturasen in den nachfolgend beschriebenen Wirtszellen oder transgenen Organismen koexprimiert oder in den erfindungsgemäßen Verfahren verwendet werden. Besonders bevorzugte Kombinationen für die Herstellung von Arachidonsäure sind in Tabelle 7, für die Herstellung von Eicosapentaensäure in Tabelle 8 und für Docosahexaensäure in Tabelle 9 nachfolgend aufgeführt.

Bevorzugt werden die Fettsäuren in Phospholipiden oder CoA-Fettsäureestern desaturiert, vorteilhaft in den CoA-Fettsäureester. Somit ist eine einfache, kostengünstige Herstellung dieser mehrfach ungesättigten Fettsäuren speziell in eukaryontischen Systemen möglich. Die mittels der erfindungsgemäßen Polynukleotide hergestellten ungesättigten Fettsäuren können dann als Öl-, Lipid- und Fettsäurezusammensetzungen formuliert und entsprechend eingesetzt werden.

Die vorliegende Erfindung betrifft weiterhin einen Vektor, der das erfindungsgemäße Polynukleotid umfasst.

Der Begriff "Vektor" bezeichnet ein Nukleinsäuremolekül, das ein anderes Nukleinsäuremolekül, wie die erfindungsgemäßen Polynukleotide, transportieren kann, an welche es gebunden ist. Ein Vektortyp ist ein Plasmid, das für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier auch als Expressionsvektoren bezeichnet.

Gewöhnlich haben Expressionsvektoren, die für DNA-Rekombinationstechniken geeignet sind, die Form von Plasmiden. In der vorliegenden Beschreibung können die Begriffe "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, künstliche Chromosomen, umfassen. Der Begriff umfasst schließlich auch Konstrukte für zielgerichtete, d.h. homologe Rekombination, oder heterologe Insertion von Polynukleotiden.

Vektoren lassen sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.-coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pB1101, pBinAR, pGPTV und pCAMBIA. Eine Übersicht über binäre Vektoren und ihre Verwendung gibt Hellens et al, Trends in Plant Science (2000) 5, 446-451. Die Vektoren mit den inserierten erfindungsgemäßen Polynukleotiden lassen sich in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen. Mittels der Klonierungsvektoren können die erfindungsgemäßen Polynukleotide in Organismen wie Mikroorganismen oder Pflanzen eingebracht werden und damit zur Pflanzentransformation verwendet werden. Geeignet Vektoren dafür sind veröffentlicht in: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)).

Bevorzugt handelt es sich bei dem Vektor um einen Expressionsvektor. Bei dem erfindungsgemäßen Expressionsvektor liegt das Polynucleotid in operativer (d.h. funktioneller) Verbindung zu einer Expressionskontrollsequenz vor. Die Expressionskontrollsequenz zusammen mit dem Polynucleotid und optional weiteren Sequenzelementen des Vektors wird auch als Expressionskassette bezeichnet. Durch die Expressionskotrollsequenz wird sichergestellt, dass das Polynucleotid nach Transformation oder Transfektion in eine Wirtszelle exprimiert werden kann. Die zu verwendende Expressionskontrollsequenz enthält vorzugsweise cis-regulatorische Elemente wie Promotor und/oder Enhancer Nukleinsäuresequenzen, die von der Transkriptionsmaschinerie der Wirtszellen erkannt werden. Der Begriff umfasst zudem andere Expressionskontrollelemente z.B. Polyadenylierungssignale und RNA stabilisierende Sequenzen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Expressionskontrollsequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann. Die erfindungsgemäßen Polynukleotide können in einer oder mehreren Kopien in der Expressionskassette oder dem erfindungsgemäßen Expressionsvektor vorliegen (z.B. in Form mehrerer Expressionskassetten). Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Weitere Expressionskontrollsequenzen im Sinne der vorliegenden Erfindung sind Translationsterminatoren am 3'-Ende der zu translatierenen Polynukleotide. Verwendet werden kann hier z.B. der OCS1 Terminator. Wie auch für die Promotoren, so sollte hier für jedes zu exprimierende Polynucleotid eine unterschiedliche Terminatorsequenz verwendet werden.

Bevorzugte Expressionskontrollsequenzen bzw. Regulationssequenzen liegen in Promotoren vor, wie dem cos-, tac-, trp-, tet-, trp-tet-, Ipp-, lac-, Ipp-lac-, laclq-, T7-, T5-, T3-, gal-, trc-, ara-, SP6-, λ-PR- oder λ-PL-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclin-induzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylprophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen lpt-2- oder lpt-1-Promotor aus Gerste (WO 95/15389 und WO 95/23230), Hordein-Promotor aus Gerste und andere, in WO 99/16890 beschriebene geeignete Promotoren. Es ist im Prinzip möglich, alle natürlichen Promotoren mit ihren Regulationssequenzen, wie die oben genannten, als Expressionskontrollsequenzen zu verwenden. Es ist ebenfalls möglich, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die Polynukleotide der vorliegenden Erfindung bevorzugt samenspezifisch in Ölsaaten exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Im Folgenden sind vorteilhafte bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und lpt1(Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der verschiedenen Biosynthesegene in die transgene Pflanze über mehrere Generation sicherzustellen, sollte jedes der erfindungsgemäßen Polynukleotide unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt (vorteilhaft in einem Polylinker) und anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle, beispielsweise im Polylinker, hinter den Promotor inseriert. Vorteilhaft hat jede Nukleinsäuresequenz ihren eigenen Promotor und gegebenenfalls ihren eigenen Terminator. Derartige vorteilhafte Konstrukte werden beispielsweise in DE 10102337 oder DE 10102338 offenbart. Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich nur einen Promotortyp in der Kassette zu verwenden. Dies kann jedoch zu unerwünschten Rekombinationsereignissen führen.

Die verwendeten rekombinanten Expressionsvektoren können zur Expression in prokaryotischen oder eukaryotischen Zellen gestaltet sein. Dies ist vorteilhaft, da häufig Zwischenschritte der Vektorkonstruktion der Einfachheit halber in Mikroorganismen durchgeführt werden. Vorzugsweise können die erfindungsgemäßen Polynukleotide in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird. Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11 d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt. Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCI, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, Span Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Polynukleotide der vorliegenden Erfindung auch in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Bevorzugte Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38. Eine Pflanzen-Expressionskassette enthält vorzugsweise Expressionskontrollsequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet. Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711). Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco. Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor. Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98145461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2- oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen). Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Der Expressionsvektor kann, wie oben beschrieben, zusätzlich zu den erfindungsgemäßen Polynukleotiden auch weitere Gene umfassen, die in die Organismen eingebracht werden sollen. Es ist möglich und bevorzugt, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Heterologe Gene oder Polynukleotide stammen aus einem Ursprungsorganismus, der sich von dem Zielorganismus unterscheidet, in den die Gene oder Polynukleofide eingebracht werden sollen. Bei homologen Gene oder Polynukieotide sind Zielorganismus und Ursprungsorganismus gleich. Vorzugsweise umfasst der Vektor daher mindestens ein weiteres Polynucleotid, das ein weiteres Enzym kodiert, das in die Biosynthese von Lipiden oder Fettsäuren eingebunden ist. Das Enzym ist vorzugsweise ausgewählt aus der Gruppe bestehend aus: Acyl-GoA-Dehydrogenase(n). Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglyceral-Lipase(n), Allenoxid-Synthase(n), Hydroperoxid-Lyase(n), Fettsäure-Elongase(n), Δ4-Desaturase(n), Δ5-Desaturase(n), Δ6-Desaturase(n), Δ8-Desaturase(n), Δ9-Desaturase(n), Δ12-Desaturase(n), Δ5-Elongase(n), Δ6-Elongase(n) und Δ9-Elongase(n), besonders bevorzugt aus der Gruppe bestehend aus: Δ5-Desaturase (vorzugsweise wie in SEQ ID Nr. 11 und 12 gezeigt), Δ6-Desaturase (vorzugsweise wie in SEQ ID Nr. 9 und 10 gezeigt), Δ8-Desaturase (vorzugsweise wie in SEQ ID Nr. 15 und 16 gezeigt), Δ12-Desaturase (vorzugsweise wie in SEQ ID Nr. 17 und 18 gezeigt) und omega-3-Desaturase (vorzugsweise wie in SEQ ID Nr. 13 und 14 gezeigt).

Die Erfindung betrifft auch eine Wirtszelle, die das erfindungsgemäße Polynucleotid oder den erfindungsgemäßen Vektor umfasst.

Wirtszeilen im Sinne der vorliegenden Erfindung können prinzipiell alle eukaryotischen oder prokaryotischen Zellen sein. Es können primäre Zellen aus Tieren, Pflanzen oder mehrzelligen Mikroorganismen sein, z.B. aus denen, die in der Beschreibung an anderer Stelle genannt sind. Ferner umfasst der Begriff auch Zeillinien, die aus diesen Organismen gewonnen werden können.

Wirtszellen im Sinne der Efindung können aber auch einzellige Mikroorganismen sein, z.B. Bakterien oder Pilze. Besonders bevorzugte Mikroorganismen sind Pilze ausgewählt aus der Gruppe der Familien Chaetomiaceae, Choanephoraceae, Cryptococcaceae, Cunninghamellaceae, Demetiaceae, Moniliaceae, Mortierellaceae, Mucoraceae, Pythlaceae, Sacharomycetaceae, Saprolegniaceae, Schizosacharomycetaceae, Sodariaceae oder Tuberculariaceae. Weitere bevorzugte Mikroorganismen sind ausgewählt aus der Gruppe: Choanephoraceae wie den Gattungen Blakeslea, Choanephora z.B. die Gattungen und Arten *Blakeslea trispora,* Choanephora *cucurbitarum, Choanephora infundibulifera* var. *cucurbitarum,* Mortierellaceae wie der Gattung Mortierella z.B. die Gattungen und Arten *Mortierella isabellina, Mortierella polycephala, Mortierella ramanniana, Mortierella, vinacea, Mortierella zonata,* der Familie der Mucorales wie die Gattungen und Arten *Rhizopus oryzae, Rhizopus stolonifer, Fusarium graminearium,* Pythiaceae wie den Gattungen Pythium, Phytophthora z.B. die Gattungen und Arten *Pythium debaryanum, Pythium intermedium, Pythium irregulare, Pythium megalacanthum, Pythium paroecandrum, Pythium sylvaticum, Pythium ultimum, Phytophthora cactorum, Phytophthora cinnamomi, Phyfophthora citricola, Phytophthora citrophthora, Phytophthora cryptogea, Phytophthora drechsleri, Phytophthora erythroseptica, Phytophthora lateralis, Phytophthora megasperma, Phytophthora nicotianae, Phytophthora nicotianae var. parasitica, Phytophthora palmivora, Phytophthora parasitica, Phytophthora syringae,* Saccharomycetaceae wie den Gattungen Hansenula, Pichia, Saccharomyces, Saccharomycodes, Yarrowia z.B. die Gattungen und Arten *Hansenula anomala, Hansenula californica, Hansenula canadensis, Hansenula capsulata, Hansenula ciferrii, Hansenula glucozyma, Hansenula henricii, Hansenula holstii, Hansenula minuta, Hansenula nonfermentans, Hansenula philodendri, Hansenula polymorpha, Hansenula saturnus, Hansenula subpelliculosa, Hansenula wickerhamii, Hansenula wingei, Pichia aicoholophila, Pichia angusta, Pichia anomala, Pichia bispora, Pichia burtonii, Pichia canadensis, Pichia capsulata, Pichia carsonii, Pichia cellobiosa, Pichia ciferrii, Pichia farinosa, Pichia fermentans, Pichia finlandica, Pichia glucozyma, Pichia guilliermondii, Pichia haplophila, Pichia henricii, Pichia holstii*, *Pichia jadinii, Pichia lindnerii, Pichia membranaefaciens, Pichia methanolica, Pichia minuta var. minuta, Pichia minuta var. nonfermentans, Pichia norvegensis, Pichia ohmeri, Pichia pastoris, Pichia philodendri, Pichia pini, Pichia polymorpha, Pichia quercuum, Pichia rhodanensis, Pichia sargentensis, Pichia stipitis, Pichia strasburgensis, Pichia subpelliculosa, Pichia toletana, Pichia trehalophila, Pichia vini, Pichia xylosa, Saccharomyces aceti, Saccharomyces bailii, Saccharomyces bayanus, Saccharomyces bisporus, Saccharomyces capensis, Saccharomyces carlsbergensis, Saccharomyces cerevisiae, Saccharomyces cerevisiae var. ellipsoideus, Saccharomyces chevalieri, Saccharomyces delbrueckii, Saccharomyces diastaticus, Saccharomyces drosophilarum, Saccharomyces elegans, Saccharomyces ellipsoideus, Saccharomyces fermentati, Saccharomyces florentinus, Saccharomyces fragilis, Saccharomyces heterogenicus, Saccharomyces hienipiensis, Saccharomyces inusitatus, Saccharomyces italicus, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, Saccharomycodes ludwigii, Yarrorvia lipolytica,* Schizosacharomycetaceae wie die Gattungen Schizosaccharomyces z.B. Spezies *Schizosaccharomyces japonicus var. japonicus, Schizosaccharomyces japonicus var. versatilis, Schizosaccharomyces malidevorans, Schizosaccharomyces octosporus, Schizosaccharomyces pombe var. malidevorans, Schizosaccharomyces pombe var. pombe,* Thraustochytriaceae z.B. die Genera Althornia, Aplanochytrium, Japonochytrium, Schizochytrium, Thraustochytrium z.B. Spezies *Schizochytrium aggregatum, Schizochytrium limacinum, Schizochytrium mangrovei, Schizochytrium minutum, Schizochytrium octosporum, Thraustochytrium aggregatum, Thraustochytrium amoeboideum, Thraustochytrium antacticum, Thraustochytrium arudimentale, Thraustochytrium aureum, Thraustochytrium benthicola, Thraustochytrium globosum, Thraustochytrium indicum, Thraustochytrium kerguelense, Thraustochytrium kinnei, Thraustochytrium motivum, Thraustochytrium multirudimentale, Thraustochytrium pachydermum, Thraustochytrium proliferum, Thraustochytrium roseum, Thraustochytrium rossii, Thraustochytrium striatum oder Thraustochytrium visurgense.*

Ebenfalls bevorzugt als Mikroorganismen sind Bakterien ausgewählt aus der Gruppe der Familien Bacillaceae, Enterobacteriacae oder Rhizobiaceae. Besonders bevorzugt seien die folgenden Bakterien genannt ausgewählt aus der Gruppe: Bacillaceae wie die Gattung Bacillus z.B die Gattungen und Arten *Bacillus acidocaldarius, Bacillus acidoterrestris, Bacillus alcalophilus, Bacillus amyloliquefaciens, Bacillus amylolyticus, Bacillus brevis, Bacillus cereus, Bacillus circulans, Bacillus coagulans, Bacillus sphaericus subsp. fusiformis, Bacillus galactophilus, Bacillus globisporus, Bacillus globisporus subsp. marinus, Bacillus halophilus, Bacillus lentimorbus, Bacillus lentus, Bacillus licheniformis, Bacillus megaterium, Bacillus polymyxa, Bacillus psychrosaccharolyticus, Bacillus pumilus, Bacillus sphaericus, Bacillus subtilis subsp. spizizenii, Bacillus subtilis subsp. subtilis* oder *Bacillus thuringiensis;* Enterobacteriacae wie die Gattungen Citrobacter, Edwardsiella, Enterobacter, Erwinia, Escherichia, Klebsiella, Salmonella oder Serratia z.B die Gattungen und Arten *Citrobacter amalonaticus, Citrobacter diversus, Citrobacter freundii, Citrobacter genomospecies, Citrobacter gillenii, Citrobacter intermedium, Citrobacter koseri, Citrobacter murliniae, Citrobacter sp., Edwardsiella hoshinae, Edwardsiella ictaluri, Edwardsiella tarda, Erwinia alni, Erwinia amylovora, Erwinia ananatis, Erwinia aphidicola, Erwinia billingiae, Erwinia cacticida, Erwinia cancerogena, Erwinia carnegieana, Erwinia carotovora* subsp. *atroseptica, Erwinia carotovora* subsp. *betavasculorum, Erwinia carotovora* subsp. *odorifera, Erwinia carotovora* subsp. *wasabiae, Erwinia chrysanthemi, Erwinia cypripedii, Erwinia dissolvens, Erwinia herbicola, Erwinia mallotivora, Erwinia milletiae, Erwinia nigrifluens, Erwinia nimipressuralis, Erwinia persicina, Erwinia psidii, Erwinia pyrifoliae, Erwinia quercina, Erwinia rhapontici, Erwinia rubrifaciens, Erwinia salicis, Erwinia stewartii, Erwinia tracheiphila, Erwinia uredovora, Escherichia adecarboxylata, Escherichia anindolica, Escherichia aurescens, Escherichia blattae, Escherichia coli, Escherichia coli var. communior, Escherichia coli-mutabile, Escherichia fergusonii, Escherichia hermannii, Escherichia sp., Escherichia vulneris, Klebsiella aerogenes, Klebsiella edwardsii subsp. atlantae, Klebsiella ornithinolytica, Klebsiella oxytoca, Klebsiella planticola, Klebsiella pneumoniae, Klebsiella pneumoniae subsp. pneumoniae, Klebsiella sp., Klebsiella terrigena, Klebsiella trevisanii, Salmonella abony, Salmonella arizonae, Salmonella bongori, Salmonella choleraesuis subsp. arizonae, Salmonella choleraesuis subsp. bongori, Salmonella choleraesuis subsp. cholereasuis, Salmonella choleraesuis subsp. diarizonae, Salmonella choleraesuis subsp. houtenae, Salmonella choleraesuis subsp. indica, Salmonella choleraesuis subsp. salamae, Salmonella daressalaam, Salmonella enterica subsp. houtenae, Salmonella enterica subsp. salamae, Salmonella enteritidis, Salmonella gallinarum, Salmonella heidelberg, Salmonella panama, Salmonella senftenberg, Salmonella typhimurium, Serratia entomophila, Serratia ficaria, Serratia fonticola, Serratia grimesii, Serratia liquefaciens, Serratia marcescens, Serratia marcescens subsp. marcescens, Serratia marinorubra, Serratia odorifera, Serratia plymouthensis, Serratia plymuthica, Serratia proteamaculans, Serratia proteamaculans subsp. quinovora, Serratia quinivorans* oder *Serratia rubidaea;* Rhizobiaceae wie die Gattungen Agrobacterium, Carbophiius, Chelatobacter, Ensifer, Rhizobium, Sinorhizobium z.B. die Gattungen und Arten *Agrobacterium atlanticum, Agrobacterium ferrugineum, Agrobacterium gelatinovorum, Agrobacterium larrymoorei, Agrobacterium meteori, Agrobacterium radiobacter, Agrobacterium rhizogenes, Agrobacterium rubi, Agrobacterium stellulatum, Agrobacterium tumefaciens, Agrobacterium vitis, Carbophilus carboxidus, Chelatobacter heintzii, Ensifer adhaerens, Ensifer arboris, Ensifer fredii, Ensifer kostiensis, Ensifer kummerowiae, Ensifer medicae, Ensifer meliloti, Ensifer saheli, Ensifer terangae, Ensifer xinjiangensis, Rhizobium ciceri Rhizobium etli, Rhizobium fredii, Rhizobium galegae, Rhizobium gallicum, Rhizobium giardinii, Rhizobium hainanense, Rhizobium huakuii, Rhizobium huautlense, Rhizobium indigoferae, Rhizobium japonicum, Rhizobium leguminosarum, Rhizobium loessense, Rhizobium loti, Rhizobium lupini, Rhizobium mediterraneum, Rhizobium meliloti, Rhizobium mongolense, Rhizobium phaseoli, Rhizobium radiobacter, Rhizobium rhizogenes, Rhizobium rubi, Rhizobium sullae, Rhizobium tianshanense, Rhizobium trifolii, Rhizobium tropici, Rhizobium undicola, Rhizobium vitis, Sinorhizobium adhaerens, Sinorhizobium arboris, Sinorhizobium fredii, Sinorhizobium kostiense, Sinorhizobium kummerowiae, Sinorhizobium medicae, Sinorhizobium meliloti, Sinorhizobium morelense, Sinorhizobium saheli* oder *Sinorhizobium xinjiangense.*

Nutzbare Wirtszelien sind weiterhin genannt in: Goeddel, Gene Expression Technology: Methods in Enzymology 185, Academic Press, San Diego, CA (1990). Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128. Hierzu gehören Pflanzenzellen und bestimmte Gewebe, Organe und Teile von Pflanzen in all ihren Erscheinungsformen, wie Antheren, Fasern, Wurzel haare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Polynukleotide oder Vektoren können mit im Stand der Technik bekannten Transformations- bzw. Transfektionsverfahren in die Wirtszelle eingebracht werden wie zuvor beschrieben. Bedingungen und Medien für die Kultivierung der Wirtszellen sind dem Fachmann ebenfalls bekannt.

Bevorzugt umfasst die erfindungsgemäße Wirtszelle zusätzlich mindestens ein weiteres Enzym, das in die Biosynthese von Lipiden oder Fettsäuren eingebunden ist. Bevorzugte Enzyme sind bereits an anderer Stelle in der Beschreibung genannt. Das Enzym kann endogen in der Wirtszelle vorliegen, d.h. die Wirtszelle exprimiert bereits natürlicherweise ein Gen, das für ein entsprechendes Enzym kodiert. Alternativ kann auch ein heterologes Polynucleotid in die Wirtszelle eingebracht werden, das für das Enzym kodiert. Geeignete Verfahren und Maßnahmen für die Expression eines heterologen Polynucleotids sind im Stand der Technik bekannt und in Zusammenhang mit den efindungsgemäßen Polynukleotiden, Vektoren und Wirtszellen hier beschrieben.

Die Erfindung betrifft auch ein Verfahren zur Herstellung eines Polypeptids mit Elongase Aktivität umfassend die Schritte:
(a) Exprimieren eines erfindungsgemäßen Polynukleotids wie oben definiert in einer Wirtszelle; und
(b) Gewinnen des Polypeptids, das von dem Polynukleotid kodiert wird, aus der Wirtszelle.

Das Polypeptid kann hierbei durch alle gängigen Verfahren zur Proteinreinigung gewonnen werden. Die Verfahren umfassen beispielsweise Affinitätschromatographie, Molsiebchromatographie, Hochdruck Flüssigkeitschromatographie oder auch Proteinpräzipitation ggf. mit spezifischen Antikörpern. Obwohl dies bevorzugt ist, muss das Verfahren nicht notwendigerweise ein reines Präparat des Polypeptids bereitstellen.

Die Erfindung betrifft somit auch ein Polypeptid, das von dem erfindungsgemäßen Polynucleotid kodiert wird oder das erhätlich ist durch das zuvor genannte erfindungsgemäße Verfahren.

Der Begriff "Polypeptid" bezeichnet sowohl ein im Wesentlichen reines Polypeptid als auch ein Polypeptid Präparat, das noch weitere Komponenten oder Verunreinigungen aufweist. Der Begriff wird auch für Fusionsproteine oder Proteinaggregate verwendet, die das erfindungsgemäße Polypeptid und zusätzlich noch weitere Komponenten umfassen. Der Begriff bezeichnet auch chemisch modifizierte Polypeptide. Chemische Modifikationen beinhalten in diesem Zusammenhang künstliche Modifikationen oder natürlich auftretende Modifikationen, z.B posttranslationale Modifikationen wie Phosphorylierung, Myristylierung, Glykosylierung usw.. Die Begriffe Polypeptid, Peptid oder Protein sind austauschbar und werden entsprechend in der Beschreibung und im Stand der Technik verwendet. Die erfindungsgemäßen Polypeptide haben die zuvor genannten biologischen Aktivitäten, also Elongase Aktivitäten, und können die Biosynthese von mehrfach ungesättigten Fettsäuren (PUFAs), vorzugsweise den langkettigen PUFAs (LCPUFAs), wie hier beschrieben beeinflussen.

Von der Erfindung umfasst ist auch ein Antikörper, der das erfindungsgemäße Polypeptid spezifisch erkennt.

Antikörper gegen das erfindungsgemäße Polypeptid können mit bekannten Verfahren hergestellt werden, bei denen gereinigtes Polypeptid oder Fragmente davon mit geeigneten Epitopen als Antigen verwendet werden. Geeignete Epitope können mittels bekannter Algorithmen zur Antigenizitätsbestimmung basierend auf den hier bereitgestellten Aminosäuresequenzen der erfindungsgemäßen Polypeptide ermittelt werden. Die entsprechenden Polypeptide oder Fragmente können dann synthetisiert oder rekombinant gewonnen werden. Nach Immunisierung von geeigneten Tieren, vorzugsweise von Säugern z.B. Hasen, Ratten oder Mäusen, können die Antikörper dann aus dem Serum mit bekannten Verfahren gewonnen werden. Alternativ können monoklonale Antikörper oder Antikörperfragmente mit den bekannte Verfahren bereitgestellt werden; siehe z.B. Harlow and Lane "Antibodies, A Laboratory Manual", CSH Press, Cold Spring Harbor, 1988, oder Köhler und Milstein, Nature 256 (1975), 495, und Galfre, Meth. Enzymol. 73 (1981), 3.

Vorzugsweise handelt es sich bei den Antikörpern um monoklonale oder polyklonale Antikörper, "single chain"-Antikörper oder chimäre Antikörper sowie Fragmenten davon wie Fab, Fv oder scFv. Weitere Antikörper im Sinne der Erfindung sind bispezifische Antikörper, synthetische Antikörper oder deren chemisch modifizierte Derivate.

Die erfindungsgemäßen Antikörper erkennen die erfindungsgemäßen Polypeptide spezifisch, d.h. sie kreuzreagieren nicht in signifikantem Ausmaß mit anderen Proteinen. Dies kann mit im Stand der Technik bekannten Verfahren getestet werden. Die Antikörper können beispielsweise zur Immunopräzipitation, zur Immunhistochemie oder zur Proteinreinigung (z.B. Affinitätschromatographie) eingesetzt werden.

Die Erfindung betrifft ferner einen transgenen, nicht-humanen Organismus, der das Polynucleotid, den Vektor oder die Wirtszelle der vorliegenden Erfindung umfasst Bevorzugt handelt es sich bei dem transgenen, nicht humanen Organismus um ein Tier, eine Pflanze oder einen multizeiluläreren Mikroorganismus.

Unter dem Begriff "transgen" ist zu verstehen, dass ein heterologes Polynucleotid, also ein in dem jeweiligen Organismus nicht natürlicherweise vorkommende Polynucleotid, in den Organismus eingebracht wird. Dies kann entweder durch zufällige Insertion des Polynucleotids oder durch homologe Rekombination erreicht werden. Selbstverständlich kann statt des Polynucleotids auch der erfindungsgemäße Vektor eingebracht werden. Verfahren zum Einbringen von Polynukleotiden oder Vektoren zwecks zufälliger Insertion oder homologer Rekombination sind im Stand der Technik bekannt und auch nachfolgend genauer beschrieben. Wirtszellen, die das Polynucleotid oder den Vektor enthalten, können ebenfalls in einen Organismus eingebracht werden und so einen transgenen Organismus erzeugen. Bei einem solchen Organismus handelt es sich dann aber um einen chimären Organismus bei dem lediglich die Zellen, die sich von den eingebrachten Zellen ableiten, transgen sind, d.h. das heterologe Polynucleotid umfassen.

Bevorzugt sind die transgenen, nicht-humanen Organismen Öl-produzierende Organismen, das heißt solche, die für die Herstellung von Ölen verwendet werden, beispielsweise Pilze wie Rhizopus oder Thraustochytrium, Algen wie Euglena, Nephroselmis, Pseudoscourfielda, Prasinococcus, Scherffelia, Tetraselmis, Mantoniella, Ostreococcus, Crypthecodinium, Phaeodactylum, Diatomeen wie Pytium oder Phytophtora oder Pflanzen.

Als transgene Pflanzen können grundsätzlich alle Pflanzen verwendet werden, d.h. sowohl zweikeimblättrige als auch einkeimblättrige Pflanzen. Vorzugsweise handelt es sich um Ölfruchtpflanzen, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Saflor , Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf. Prinzipiell kommen aber alle Pflanzen in Frage, die in der Lage sind Fettsäuren zu synthetisieren wie alle dikotylen oder monokotylen Pflanzen, Algen oder Moose. Vorteilhaft Pflanzen sind ausgewählt aus der Gruppe der Pflanzenfamilien Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae, Prasinophyceae oder Gemüsepflanzen oder Zierpflanzen wie Tagetes in Betracht.

Besonders bevorzugt seien die folgenden Pflanzen genannt ausgewählt aus der Gruppe: Adelotheciaceae wie die Gattungen Physcomitrella z.B. die Gattung und Arten *Physcomitrella patens,* Anacardiaceae wie die Gattungen Pistacia, Mangifera, Anacardium z.B. die Gattung und Arten *Pistacia vera* [Pistazie], *Mangifer indica* [Mango] oder *Anacardium occidentale* [Cashew], Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis [Garten-Ringelblume], Carthamus tinctorius [Färberdistel, safflower], *Centaurea cyanus* [Kornblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [Salat], *Tagetes lucida, Tagetes erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Borago z.B. die Gattung und Art *Borago officinalis* [Borretsch], Brassicaceae wie die Gattungen Brassica, Camelina, Melanosinapis, Sinapis, Arabadopsis z.B. die Gattungen und Arten *Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides,* Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia comosa* [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima, Beta vulgaris var. Vulgaris, Beta maritima, Beta vulgaris var. perennis, Beta vulgaris var. conditiva* oder *Beta vulgaris var. esculenta* [Zuckerrübe], Crypthecodiniaceae wie die Gattung Crypthecodinium z.B. die Gattung und Art *Cryptecodinium cohnii,* Cucurbitaceae wie die Gattung Cucubita z.B. die Gattungen und Arten *Cucurbita maxima, Cucurbita mixta, Cucurbita pepo* oder *Cucurbita moschata* [Kürbis], Cymbellaceae wie die Gattungen Amphora, Cymbella, Okedenia, Phaeodactylum, Reimeria z.B. die Gattung und Art *Phaeodactylum tricornutum,* Ditrichaceae wie die Gattungen Ditrichaceae, Astomiopsis, Ceratodon, Chrysoblastella, Ditrichum, Distichium, Eccremidium, Lophidion, Philibertiella, Pleuridium, Saelania, Trichodon, Skottsbergia z.B. die Gattungen und Arten *Ceratodon antarcticus, Ceratodon columbiae, Ceratodon heterophyllus, Ceratodon purpurascens, Ceratodon purpureus, Ceratodon purpureus ssp. convolutus, Ceratodon purpureus ssp. stenocarpus, Ceratodon purpureus var. rotundifolius, Ceratodon ratodon, Ceratodon stenocarpus, Chrysoblastella chilensis, Ditrichum ambiguum, Ditrichum brevisetum, Ditrichum crispatissimum, Ditrichum difficile, Ditrichum falcifolium, Ditrichum flexicaule, Ditrichum giganteum, Ditrichum heteromallum, Ditrichum lineare, Ditrichum lineare, Ditrichum montanum, Ditrichum montanum, Ditrichum pallidum, Ditrichum punctulatum, Ditrichum pusillum, Ditrichum pusillum var. tortile, Ditrichum rhynchostegium, Ditrichum schimperi, Ditrichum tortile, Distichium capillaceum, Distichium hagenii, Distichium inclinatum, Distichium macounii, Eccremidium floridanum, Eccremidium whiteleggei, Lophidion strictus, Pleuridium acuminatum, Pleuridium alternifolium, Pleuridium holdridgei, Pleuridium mexicanum, Pleuridium ravenelii, Pleuridium subulatum, Saelania glaucescens, Trichodon borealis, Trichodon cylindricus oder Trichodon cylindricus var. oblongus,* Elaeagnaceae wie die Gattung Elaeagnus z.B. die Gattung und Art *Olea europaea* [Olive], Ericaceae wie die Gattung Kalmia z.B. die Gattungen und Arten *Kalmia latifolia, Kalmia angustifolia, Kalmia microphylla, Kalmia polifolia, Kalmia occidentalis, Cistus chamaerhodendros* oder *Kalmia lucida* [Berglorbeer], Euphorbiaceae wie die Gattungen Manihot, Janipha, Jatropha, Ricinus z.B. die Gattungen und Arten *Manihot utilissima, Janipha manihot,, Jatropha manihot., Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago varia* [Alfalfa] *Glycine max Dolichos soja, Glycine gracilis, Glycine hispida, Phaseolus max, Soja hispida* oder *Soja max* [Sojabohne], Funariaceae wie die Gattungen Aphanorrhegma, Entosthodon, Funaria, Physcomitrella, Physcomitrium z.B. die Gattungen und Arten *Aphanorrhegma serratum, Entosthodon attenuatus, Entosthodon bolanderi, Entosthodon bonplandii, Entosthodon californicus, Entosthodon drummondii, Entosthodon jamesonii, Entosthodon leibergii, Entosthodon neoscoticus, Entosthodon rubrisetus, Entosthodon spathulifolius, Entosthodon tucsoni, Funaria americana, Funaria bolanderi, Funaria calcarea, Funaria californica, Funaria calvescens, Funaria convoluta, Funaria flavicans, Funaria groutiana, Funaria hygrometrica, Funaria hygrometrica var. arctica, Funaria hygrometrica var. calvescens, Funaria hygrometrica var. convoluta, Funaria hygrometrica var. muralis, Funaria hygrometrica var. utahensis, Funaria microstoma, Funaria microstoma var. obtusifolia, Funaria muhlenbergii, Funaria orcuttii, Funaria plano-convexa, Funaria polaris, Funaria ravenelii*, *Funaria rubriseta, Funaria serrata, Funaria sonorae, Funaria sublimbatus, Funaria tucsoni, Physcomitrella californica, Physcomitrella patens, Physcomitrella readeri, Physcomitrium australe, Physcomitrium californicum, Physcomitrium collenchymatum, Physcomitrium coloradense, Physcomitrium cupuliferum, Physcomitrium drummondii, Physcomitrium eurystomum, Physcomitrium flexifolium, Physcomitrium hookeri, Physcomitrium hookeri var. serratum, Physcomitrium immersum, Physcomitrium kellermanii, Physcomitrium megalocarpum, Physcomitrium pyriforme, Physcomitrium pyriforme var. serratum, Physcomitrium rufipes, Physcomitrium sandbergii, Physcomitrium subsphaericum, Physcomitrium washingtoniense,* Geraniaceae wie die Gattungen Pelargonium, Cocos, Oleum z.B. die Gattungen und Arten *Cocos nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea gratissima* oder *Persea persea* [Avocado], Leguminosae wie die Gattung Arachis z.B. die Gattung und Art *Arachis hypogaea* [Erdnuss], Linaceae wie die Gattungen Linum, Adenolinum z.B. die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hirsutum, Gossypium arboreum, Gossypium barbadense, Gossypium herbaceum* oder *Gossypium thurberi* [Baumwolle], Marchantiaceae wie die Gattung Marchantia z.B. die Gattungen und Arten *Marchantia berteroana, Marchantia foliacea, Marchantia macropora,* Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], *Zea mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum,* Proteaceae wie die Gattung Macadamia z.B. die Gattung und Art *Macadamia intergrifolia* [Macadamia], Prasinophyceae wie die Gattungen Nephroselmis, Prasinococcus, Scherffelia, Tetraselmis, Mantoniella, Ostreococcus z.B. die Gattungen und Arten *Nephroselmis olivacea, Prasinococcus capsulatus, Scherffelia dubia*, *Tetraselmis chui, Tetraselmis suecica,* Mantoniella squamata, Ostreococcus tauri, Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Verbascum z.B. die Gattungen und Arten *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* oder *Verbascum thapsus [Königskerze],* Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solanum lycopersicum* [Tomate], Sterculiaceae wie die Gattung Theobroma z.B. die Gattung und Art *Theobroma cacao* [Kakao] oder Theaceae wie die Gattung Camellia z.B. die Gattung und Art *Camellia sinensis* [Tee].

Multizelluläre Mikroorganismen, die als transgene nicht-humane Organismen eingesetzt werden können, sind vorzugsweise Protisten oder Diatomeen ausgewählt aus der Gruppe der Familien Dinophyceae, Turaniellidae oder Oxytrichidae wie die Gattungen und Arten: *Crypthecodinium cohnii, Phaeodactylum tricornutum, Stylonychia mytilus, Stylonychia pustulata, Stylonychia putrina, Stylonychia notophora, Stylonychia* sp., Colpidium campylum oder Colpidium sp.

Die Erfindung betrifft des weiteren ein Verfahren zur Herstellung eines Stoffes, der die Struktur aufweist, die in der folgenden allgemeinen Formel I gezeigt wird 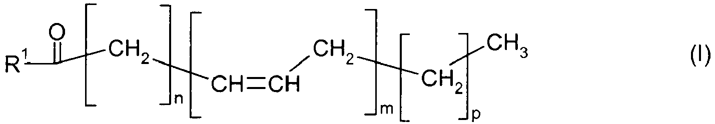 wobei die Variablen und Substiuenten die folgenden sind
- R¹ =: Hydroxyl, Coenzym A (Thioester), Lysophosphatidylcholin, Lysophosphatidylethanolamin, Lysophosphatidylglycerol, Lysodiphosphatidylglycerol, Lysophosphatidylserin, Lysophosphatidylinositol, Sphingo- Base oder ein Radikal der Formel II

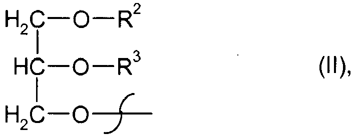

- R² =: Wasserstoff, Llysophosphatidylcholin, Lysophosphatidylethanolamin, Lysophosphatidylglycerol, Llysodiphosphatidylglycerol, Lysophosphatidylserin, Lysophosphatidylinositol oder gesättigtes oder ungesättigtes C₂-C₂₄-Alkylcarbonyl,
- R³ =: Wasserstoff, gesättigtes oder ungesättigtes C₂-C₂₄-Alkylcarbonyl, oder R² und R³ sind unabhängig voneinander ein Radikal der Formel Ia:

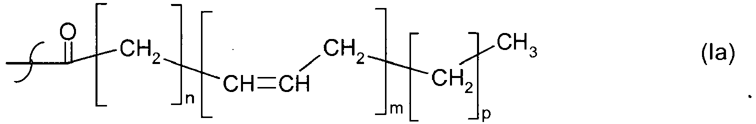

- n =: 2, 3, 4, 5, 6, 7 or 9, m = 2, 3, 4, 5 oder 6 und p = 0 oder 3;
und wobei das Verfahren das Kultivieren von (i) einer erfindungsgemäßen Wirtszelle oder (ii) eines erfindungsgemäßen transgenen, nicht-humanen Organismus unter Bedingungen umfasst, die die Biosynthese des Stoffes erlauben. Vorzugsweise wird dabei der zuvor genannte Stoff in einer Menge von mindestens 1 Gew.-% bezogen auf den Gesamtgehalt der Lipide in der Wirtszelle oder dem transgenen Organismus bereitgestellt.

R¹ bedeutet in der allgemeinen Formel I Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

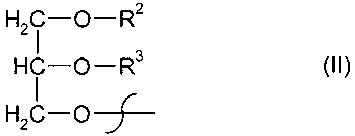

Die oben genannten Reste von R¹ sind immer in Form ihrer Thioester an die Verbindungen der allgemeinen Formel I gebunden.
R² bedeutet in der allgemeinen Formel II Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes C₂-C₂₄-Alkylcarbonyl.

Als Alkylreste seien substituiert oder unsubstituiert, gesättigt oder ungesättigte C₂-C₂₄-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl- genannt, die eine oder mehrere Doppelbindung(en) enthalten. Gesättigte oder ungesättigte C_{1O}-C₂₂-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl-oder n-Tetracosanylcarbonyl-, die eine oder mehrere Doppelbindung(en) enthalten, sind bevorzugt. Besonders bevorzugt sind gesättigte und/oder ungesättigte C₁₀-C₂₂-Alkylcarbonylreste wie C₁₀-Alkylcarbonyl-, C₁₁-Alkylcarbonyl-, C₁₂-Alkylcarbonyl-, C₁₃-Alkylcarbonyl-, C₁₄-Alkylcarbonyl-, C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Ganz besonders bevorzugt sind gesättigte oder ungesättigte C₁₆-C₂₂-Alkylcarbonylreste wie C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

R³ bedeutet in der allgemeinen Formel II Wasserstoff-, gesättigtes oder ungesättigtes C₂-C₂₄-Alkylcarbonyl.

Als Alkylreste seien substituiert oder unsubstituiert, gesättigt oder ungesättigte C₂-C₂₄-Alkylcarbonyl-Ketten wie Ethylcarbonyl-, n-Propylcarbonyl-, n-Butylcarbonyl-, n-Pentylcarbonyl-, n-Hexylcarbonyl-, n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl- genannt, die eine oder mehrere Doppelbindung(en) enthalten. Gesättigte oder ungesättigte C₁₀-C₂₂-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl-oder n-Tetracosanylcarbonyl-, die eine oder mehrere Doppelbindung(en) enthalten, sind bevorzugt. Besonders bevorzugt sind gesättigte und/oder ungesättigte C₁₀-C₂₂-Alkylcarbonylreste wie C₁₀-Alkylcarbonyl-, C₁₁-Alkylcarbonyl-, C₁₂-Alkylcarbonyl-, C₁₃-Alkylcarbonyl-, C₁₄-Alkylcarbonyl-, C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Ganz besonders bevorzugt sind gesättigte oder ungesättigte C₁₆-C₂₂-Alkylcarbonylreste wie C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

Die oben genannten Reste von R¹, R² and R³ können mit Hydroxyl- und/oder Epoxygruppen substituierte sein und/oder können Dreifachbindungen enthalten.

Vorteilhaft enthalten die im erfindungsgemäßen Verfahren hergestellten mehrfach ungesättigten Fettsäuren mindestens zwei, vorteilhaft drei, vier, fünf oder sechs Doppelbindungen. Besonders vorteilhaft enthalten die Fettsäuren vier, fünf oder sechs Doppelbindungen. Im Verfahren hergestellte Fettsäuren haben vorteilhaft 18-, 20- oder 22-C-Atome in der Fettsäurekette, bevorzugt enthalten die Fettsäuren 20 oder 22 Kohlenstoffatome in der Fettsäurekette. Vorteilhaft werden gesättigte Fettsäuren mit den im Verfahren verwendeten Nukleinsäuren wenig oder gar nicht umgesetzt. Unter wenig ist zu verstehen, dass im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese hergestellten Fettsäuren können als einziges Produkt im Verfahren hergestellt werden oder in einem Fettsäuregemisch vorliegen.

Vorteilhaft bedeuten die Substituenten R² oder R³ in den allgemeinen Formeln I und II unabhängig voneinander gesättigtes oder ungesättigtes C₁₈-C₂₂-Alkylcarbonyl-, besonders vorteilhaft bedeuten sie unabhängig voneinander ungesättigtes C₁₈-, C₂₀-oder C₂₂-Alkylcarbonyl- mit mindestens zwei Doppelbindungen.

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen. Die in den Triacylglyceriden gebundenen verschiedenen Fettsäuren lassen sich dabei von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die langkettigen Fettsäuren, besonders bevorzugt sind die langkettigen Fettsäuren LCPUFAs von C₁₈-, C₂₀- und/oder C₂₂-Fettsäuren.

Im erfindungsgemäßen Verfahren werden vorteilhaft Fettsäureester mit mehrfach ungesättigten C₁₈-, C₂₀- und/oder C₂₂-Fettsäuremolekülen mit mindestens zwei Doppelbindungen im Fettsäureester, vorteilhaft mit mindestens drei, vier, fünf oder sechs Doppelbindungen im Fettsäureester, besonders vorteilhaft von mindestens fünf oder sechs Doppelbindungen im Fettsäureester hergestellt und führen vorteilhaft zur Synthese von Linolsäure (=LA, C18:2^{Δ9,12}), γ-Linolensäure (= GLA, C18:3^{Δ6,9,12}), Stearidonsäure (= SDA, C18:4 ^{Δ6,9,12,15}), Dihomo-γ-Linolensäure (= DGLA, 20:3 ^{Δ8,11,14}), ω-3-Eicosatetraensäure (= ETA, C20:4 ^{Δ5,8,11,14}), Arachidonsäure (ARA, C20:4 ^{Δ5,8,11,14}), Eicosapentaensäure (EPA, C20:5^{Δ5,8,11,14,17}), ω-6-Docosapentaensäure (C22:5^{Δ4,7,10,13,16}), ω-6-Docosatetraensäure (C22:4^{Δ,7,10,13,16}), ω-3-Docosapentaensäure (= DPA, C22:5^{Δ7,10,13,16,19}), Docosahexaensäure (= DHA, C22:6^{Δ4,7,10,13,16,19}) oder deren Mischungen, bevorzugt ARA, EPA und/oder DHA. Ganz besonders bevorzugt werden, ω-3-Fettsäuren wie EPA und/oder DHA hergestellt.

Die Fettsäureester mit mehrfach ungesättigten C₁₈-, C₂₀- und/oder C₂₂-Fettsäure-molekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin. Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die Acetyl-Coenzym-A-Ester, die die merhfach ungesättigten Fettsäuren mit mindestens zwei, drei, vier, fünf oder sechs bevorzugt fünf oder sechs Doppelbindungen enthalten, isoliert werden, vorteilhaft werden sie in der Form ihrer Diacylglyceride, Triacylglyceride und/oder in Form des Phosphatidylcholin isoliert, besonders bevorzugt in der Form der Triacylglyceride. Neben diesen Estern sind die mehrfach ungesättigten Fettsäuren auch als freie Fettsäuren oder gebunden in anderen Verbindungen in den Organismen, vorteilhaft den Pflanzen, enthalten. In der Regel liegen die verschiedenen vorgenannten Verbindungen (Fettsäureester und freie Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-%, ganz besonders bevorzugt von mindestens 15 Gew.% bezogen auf die gesamten Fettsäuren in den transgenen Organismen vorteilhaft in einer transgenen Pflanzen hergestellt. Dabei werden vorteilhaft C₁₈- und/oder C₂₀-Fettsäuren und/oder C₂₂-Fettsäuren, die in den Wirtsorganismen vorhanden sind, zu mindestens 10 %, vorteilhaft zu mindestens 20 %, besonders vorteilhaft zu mindestens 30 %, ganz besonders vorteilhaft zu mindestens 40 % in die entsprechenden Produkte wie DPA oder DHA, um nur zwei beispielhaft zu nennen, umgesetzt. Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA), ω-6-Docosapentaensäure oder DHA nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA, EPA oder DHA als Mischungen vor. Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endproduktes betragen. Vorteilhaft werden in einer transgenen Pflanze als Endprodukte nur ARA, EPA oder nur DHA im erfindungsgemäßen Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4, besonders bevorzugt von 1:1:5 hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Buttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21}).

Durch die erfindungsgemäßen Nukleinsäuresequenzen bzw. im erfindungsgemäßen Verfahren verwendeten Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber den nicht transgenen Ausgangsorganismus beispielsweise einer Hefe, einer Alge, einem Pilz oder einer Pflanze wie Arabidopsis oder Lein beim Vergleich in der GC-Analyse (siehe Beispiele) erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Im Prinzip können alle Gene des Fettsäure- oder Lipidstoffwechsels vorteilhaft in Kombination mit den erfinderischen Polynukleotiden (im Sinne dieser Anmeldung soll der Plural den Singular und umgekehrt beinhalten) im Verfahren zur Herstellung mehrfach ungesättigter Fettsäuren verwendet werden. Vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) verwendet. Besonders bevorzugt werden Gene ausgewählt aus der Gruppe der Δ-5-Desaturasen, Δ-6-Desaturasen, Δ-8-Desaturasen, Δ-12-Desaturasen, omega-3-Desaturasen in Kombination mit den erfindungsgemäßen Polynukleotiden verwendet, wobei einzelne Gene oder mehrere Gene in Kombination verwendet werden können. Besonders bevorzugt können, wie bereits erwähnt, in diesem Zusammenhang die Δ-6-Desaturase mit SEQ ID Nr. 9 und 10, die Δ-5-Desaturase mit SEQ ID Nr. 11 und 12, die Δ-8-Desaturase mit SEQ ID Nr. 15 und 16, die Δ-12-Desaturase mit SEQ ID Nr. 17 und 18 und/oder die omega-3-Desaturase mit SEQ ID Nr. 13 und 14 eingesetzt werden. Besonders bevorzugte Kombinationen für die Herstellung von Arachidonsäure sind in Tabelle 7, für die Herstellung von Eicosapentaensäure in Tabelle 8 und für Docosahexaensäure in Tabelle 9 nachfolgend aufgeführt.

Vorteilhaft setzen die im erfindungsgemäßen Verfahren verwendeten Desaturasen ihre jeweiligen Substrate in Form der CoA-Fettsäureester um. Dies führt, wenn vorher ein Elongationsschritt stattgefunden hat, vorteilhaft zu einer erhöhten Produktausbeute. Die jeweiligen Desaturierungsprodukte werden dadurch in höheren Mengen synthetisiert, da der Elongationsschritt in der Regel an den CoA-Fettsäureestern erfolgt, während der Desaturierungsschritt überwiegend an den Phospholipiden oder an den Triglyceriden erfolgt. Eine Ausstauschreaktion, die eine weitere möglicherweise limitierende Enzymreaktion erforderlich machen würde, zwischen den CoA-Fettsäureestern und den Phospholipiden oder Triglyceriden ist somit nicht erforderlich.

Durch die enzymatische Aktivität der im erfindungsgemäßen Verfahren verwendeten Polypeptide können unterschiedlichste mehrfach ungesättigte Fettsäuren im erfindungsgemäßen Verfahren hergestellt werden. Je nach Auswahl der für das erfindungsgemäße Verfahren verwendeten Organismen wie den bevorzugten Pflanzen lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder solche, die sich von C18:3-Fettsäuren ableiten, wie SDA, ETA oder EPA. Liegt in der für das Verfahren verwendeten Pflanze als ungesättigte Fettsäure nur Linolsäure (= LA, C 1 8:2A9, 12) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15}) beispielsweise wie in Lein, so können als Produkte des Verfahrens nur SDA, ETA, EPA und/oder DHA entstehen, die wie oben beschrieben als freie Fettsäuren oder gebunden vorliegen können. Durch Modifikation der Aktivität der an der Synthese beteiligten Enzyme Δ-5-Desaturase, Δ-6-Desaturase, Δ-4-Desaturase, Δ-12-Desaturase, Δ-5-Elongase und/oder Δ-6-Elongase lassen sich gezielt in den vorgenannten Organismen vorteilhaft in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Werden die Δ-5-Desaturase, die Δ-5-Elongase und die Δ-4-Desaturase zusätzlich in die Organismen vorteilhaft in die Pflanze eingebracht, so entstehen zusätzlich ARA, EPA und/oder DHA. Vorteilhaft werden nur ARA, EPA oder DHA oder deren Mischungen synthetisiert, abhängig von den im Organismus bzw. in der Pflanze vorliegenden Fettsäuren, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA, DHA oder deren Mischungen vorteilhaft EPA oder DHA oder deren Mischungen.

Neben der Produktion der Ausgangsfettsäuren für die im erfindungsgemäßen Verfahren verwendeten Polypeptide direkt im Organismus können die Fettsäuren auch von außen gefüttert werden. Aus Kostengründen ist die Produktion im Organismus bevorzugt. Bevorzugte Substrate sind die Linolsäure (C18:2^{Δ9,12}), die γ-Linolensäure (C18:3^{Δ6,9,12}) die Eicosadiensäure (C20:2^{Δ11,14}), die Dihomo-γ-linolensäure (C20:3^{Δ8,11,14}), die Arachidonsäure (C20:4^{Δ5,8,11,14}), die Docosatetraensäure (C22:4^{Δ7,10,13,16}) und die Docosapentaensäure (C22:5^{Δ4,7,10,13,15}).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Öl-produzierenden Organismen wie der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max,* die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Vorteilhaft werden im erfindungsgemäßen Verfahren die vorgenannten Nukleinsäuresequenzen oder deren Derivate oder Homologe eingesetzt, die für Polypeptide codieren, die noch die enzymatische Aktivität der durch Nukleinsäuresequenzen codierten Proteine besitzen. Diese Sequenzen werden einzeln oder in Kombination mit den erfindungsgemäßen Polynukleotiden in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder eines ganzen Organismuses, der die im Verfahren verwendeten Nukleinsäuresequenzen enthält, wobei die Zelle und/oder der Organismus mit einem erfindungsgemäßen Polynukleotiden, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Öle, Lipide oder freien Fettsäuren aus dem Organismus oder aus der Kultur. Bei der Kultur kann es sich beispielsweise um eine Fermentationskultur beispielsweise im Falle der Kultivierung von Mikroorganismen wie z.B. Mortierella, Thalassiosira, Mantoniella, Ostreococcus, Saccharomyces oder Thraustochytrium oder um eine Treibhaus- oder Feldkultur einer Pflanze handeln. Die so hergestellte Zelle oder der so hergestellte Organismus ist vorteilhaft eine Zelle eines 01-produzierenden Organismus, wie einer Ölfruchtpflanze wie beispielsweise Erdnuss, Raps, Canoia, Lein, Hanf, Erdnuss, Soja, Färberdiestel, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

Als Organismen bzw. Wirtszellen für das erfindungsgemäße Verfahren sind solche geeignet, die in der Lage sind Fettsäuren, speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne, Mikroorganismen wie Pilze, beispielsweise die Gattung Mortierella, Thraustochytrium, Saprolegnia, Phytophtora oder Pythium, Bakterien wie die Gattung Escherichia oder Shewanella, Hefen wie die Gattung Saccharomyces, Cyanobakterien, Ciliaten, Algen wie Mantoniella oder Ostreococcus oder Protozoen wie Dinoflagellaten wie Thalassiosira oder Crypthecodinium genannt. Bevorzugt werden Organismen, die natürlicherweise Öle in größeren Mengen synthetisieren können wie Pilze wie Mortierella alpina, Pythium insidiosum, Phytophtora infestans oder Pflanzen wie Soja, Raps, Kokosnuss, Ölpalme, FärberSaflor, Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, FärberSaflor, Sonnenblume, Calendula, Mortierella oder Saccharomyces cerevisiae. Prinzipiell sind als Wirtsorganismen neben den vorgenannten transgenen Organismen auch transgene Tiere vorteilhaft nicht-humane Tiere geeignet beispielsweise C. elegans. Weitere geeignete Wirtszellen und Organismen wurden bereits zuvor ausführlich beschrieben.

Transgene Pflanzen, die die im erfindungsgemäßen Verfahren synthetisierten mehrfach ungesättigten Fettsäuren enthalten, können vorteilhaft direkt vermarktet werden ohne dass die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze ableiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigte Fettsäuren lassen sich durch Ernten der Organismen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch so genanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmes Hexan extrahiert werden. Anschließend wird das Lösungsmittel wieder entfernt. Im Falle von Mikroorganismen werden diese nach Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf desodoriert.

Vorzugsweise sind die durch dieses Verfahren produzierten PUFAs bzw. LCPUFAs C₁₈-, C₂₀- oder C₂₂-Fettsäuremoleküle, vorteilhaft C₂₀- oder C₂₂-Fettsäuremoleküle mit mindestens zwei Doppelbindungen im Fettsäuremolekül, vorzugsweise drei, vier, fünf oder sechs Doppelbindungen. Diese C₁₈-, C₂₀- oder C₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind transgene Pflanzen.

### Beschrieben sind Öle, Lipide oder Fettsäuren oder

Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.
Diese Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vakzensäure, 0,1 bis 1 % Arachinsäure, 7 bis 25% gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester- bzw. Fettsäuregemischen bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,8-C?ctadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c1Dt12c-Octadecatriensäure), Punicinsäure (9c11t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester- bzw. Fettsäuregemischen in der Regel vorteilhaft nur in Spuren vor, das heißt, sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Buttersäure, kein Cholesterin, keine Clupanodonsäure (= Docosapentaensäure, C22:5^{Δ4,8,12,15,24}) sowie keine Nisinsäure (Tetracosahexaensäure, C23:6^{Δ3,8,12,15,18,21}).

### Bevorzugt enthalten die Öle, Lipide oder Fettsäuren mindestens

0,5%, 1%, 2%, 3%, 4% oder 5%, vorteilhaft mindestens 6%, 7%, 8%, 9% oder 10%, besonders vorteilhaft mindestens 11%, 12%, 13%, 14% oder 15% ARA oder mindestens 0,5%, 1%, 2%, 3%, 4% oder 5%, vorteilhaft mindestens 6%, oder 7%, besonders vorteilhaft mindestens 8%, 9% oder 10% EPA und/oder DHA bezogen auf den Gesamtfettsäuregehalt des Produktionsorganismus vorteilhaft einer Pflanze, besonders vorteilhaft einer Ölfruchtpflanze wie Soja, Raps, Kokosnuss, Ölpalme, Färbersafflor, Flachs, Hanf, Rizinus, Calendula, Erdnuss, Kakaobohne, Sonnenblume oder den oben genannten weiteren ein- oder zweikeimblättrigen Ölfruchtpflanzen.

Eine weitere erfindungsgemäße Ausführungsform ist die Verwendung des Öls, Lipids, der Fettsäuren und/oder der Fettsäurezusammensetzung in Futtermitteln, Nahrungsmitteln, Kosmetika oder Pharmazeutika. Die Öle, Lipide,
Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-Linolensäure, Arachidonsäure, α-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigten Fettsäuren mit vorteilhaft mindestens zwei Doppelbindungen, handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens fünf oder sechs Doppelbindungen lassen sich die enthaltenden mehrfach ungesättigten Fettsäuren beispielsweise über eine Alkalibehandlung beispielsweise wässrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die im Verfahren verwendeten Nukleinsäuren können nach Einbringung in einen Organismus vorteilhaft einer Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in "trans", so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Als Substrate der erfindungsgemäßen Polypeptide oder des erfindungsgemäßen Polypeptids des Fettsäure- oder Lipidstoffwechsels, ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) eignen sich bevorzugt C₁₈-, C₂₀ oder C₂₂Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der langkettigen PUFAs müssen die mehrfach
ungesättigten C₁₈-Fettsäuren zunächst durch die enzymatische Aktivität einer Desaturase zunächst desaturiert und anschließend über eine Elongase um mindestens zwei Kohlenstoffatome verlängert werden. Nach einer Elongationsrunde führt diese Enzymaktivität zu C₂₀-Fettsäuren, und nach zwei Elongationsrunden zu C₂₂-Fettsäuren. Die Aktivität der efindungsgemäßen Verfahren verwendeten Desaturasen und Elongasen führt vorzugsweise zu C₁₈-, C₂₀- und/oder C₂₂-Fettsäuren vorteilhaft mit mindestens zwei Doppelbindungen im Fettsäuremolekül, vorzugsweise mit drei, vier, fünf oder sechs Doppelbindungen, besonders bevorzugt zu C₂₀- und/oder C₂₂-Fettsäuren mit mindestens zwei Doppelbindungen im Fettsäuremolekül, vorzugsweise mit drei, vier, fünf oder sechs Doppelbindungen, ganz besonders bevorzugt mit fünf oder sechs Doppelbindungen im Molekül. Nachdem eine erste Desaturierung und die Verlängerung stattfanden, können weitere Desaturierungs- und Elongierungsschritte wie z.B. eine solche Desaturierung in Δ-5- und Δ-4-Position erfolgen. Besonders bevorzugt als Produkte des erfindungsgemäßen Verfahrens sind Dihomo-γ-linolensäure, Arachidonsäure, Eicosapentaensäure, Docosapetaensäure und/oder Docosahexaensäure. Die C₂₀-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanzebeispielsweise in Epidermiszellen oder in den Knollen - gewebespezifisch erfolgen kann.

Werden im erfindungsgemäßen Verfahren als Organismen Mikroorganismus wie Hefen wie Saccharomyces oder Schizosaccharomyces, Pilze wie Mortierella, Aspergillus, Phytophtora, Entomophthora, Mucor oder Thraustochytrium Algen wie Isochrysis, Mantoniella, Ostreococcus, Phaeodactylum oder Crypthecodinium verwendet, so werden diese Organismen vorteilhaft fermentativ angezogen.

Durch die Verwendung der erfindungsgemäßen Nukleinsäuren, die für eine Elongase codieren, können im Verfahren die hergestellten mehrfach ungesättigten Fettsäuren mindestens um 5 %, bevorzugt mindestens um 10 %, besonders bevorzugt mindestens um 20 %, ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Organismen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Werden im erfindungsgemäßen Verfahren als Organismen Mikroorganismen verwendet, so werden sie je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu Beginn der Fermentation vorgelegt oder semikontinuierlich oder kontinuierlich nachgefüttert werden. Die hergestellten mehrfach ungesättigten Fettsäuren können nach dem Fachmann bekannten Verfahren wie oben beschrieben aus den Organismen isoliert werden. Beispielsweise über Extraktion, Destillation, Kristallisation, ggf. Salzfällung und/oder Chromatographie. Die Organismen können dazu vorher noch vorteilhaft aufgeschlossen werden.

Das erfindungsgemäße Verfahren wird, wenn es sich bei den Wirtsorganismen um Mikroorganismen handelt, vorteilhaft bei einer Temperatur zwischen 0°C bis 95° , bevorzugt zwischen 10°C bis 85°C, besonders bevorzugt zwischen 15°C bis 75°C, ganz besonders bevorzugt zwischen 15°C bis 45°C durchgeführt.

Der pH Wert wird dabei vorteilhaft zwischen pH 4 und 12, bevorzugt zwischen pH 6 und 9, besonders bevorzugt zwischen pH 7 und 8 gehalten.

Das erfindungsgemäße Verfahren kann batchweise, semi-batchweise oder kontinuierlich betrieben werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z.B. Sojaöl, Sonnenblumenöl, Erdnussöl und/oder Kokosfett, Fettsäuren wie z.B. Palmitinsäure, Stearinsäure und/oder Linolsäure, Alkohole und/oder Polyalkohole wie z. B. Glycerin, Methanol und/oder Ethanol und/oder organische Säuren wie z.B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger- oder Gas-Form oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natriumhaltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Zitronensäure.

Die erfindungsgemäß zur Kultivierung von Mikroorganismen eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzu gegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder sauerstoffhaltige Gasmischungen, wie z.B. Umgebungsluft, in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere mehrfach ungesättigte Fettsäuren enthaltenden, Fermentationsbrühen haben üblicherweise eine Trockenmasse von 7,5 bis 25 Gew.-%.

Die Fermentationsbrühe kann anschließend weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden. Vorteilhaft wird die Biomasse nach Abtrennung aufgearbeitet.

Die Fermentationsbrühe kann aber auch ohne Zellabtrennung mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann schließlich zur Gewinnung der darin enthaltenen Fettsäuren aufgearbeitet werden.

Die Polynukleotide bzw. Polypeptide der vorliegenden Erfindung, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen vorteilhaft in Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat. Da mehrfach ungesättigte Fettsäuren (= PUFAs) nicht nur einfach in Triacylglycerin sondern auch in Membranlipide eingebaut werden.

Besonders zur Herstellung von PUFAs, beispielsweise Stearidonsäure, Eicosapentaensäure und Docosahexaensäure eignen sich Brasicaceae, Boraginaceen, Primulaceen, oder Linaceen. Besonders vorteilhaft eignet sich Lein (Linum usitatissimum) zur Herstellung von PUFAS mit den erfindungsgemäßen Nukleinsäuresequenzen vorteilhaft, wie beschrieben, in Kombination mit weiteren Desaturasen und Elongasen.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weiteren Elongationen aus den Phospholipide in den FettsäureCoA-Ester-Pool überführt werden. Dies ermöglichen Acyl-CoA:Lysophospholipid-Acyl-transferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mithilfe der im Verfahren verwendeten Desaturasen wie der Δ-15-, Δ-12- und Δ-15-, omega-3-, Δ-12-, Δ-4-, Δ-5-, Δ-6-Desaturasen und/oder der Δ-5- und/oder Δ-6-Elongasen können Arachidonsäure, Eicosapentaensäure, Docosapentaensäure oder Docosahexaensäure vorteilhaft Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden. Mit den genannten Enzymen können C₂₀- und/oder C₂₂-Fettsäuren mit mindestens zwei vorteilhaft mindestens drei, vier, fünf oder sechs Doppelbindungen im Fettsäuremolekül, vorzugsweise C₂₀- oder C₂₂-Fettsäuren mit vorteilhaft vier, fünf oder sechs Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von C₂₀ zu C₂₂-Fettsäuren, zu Fettsäuren wie γ-Linolensäure, Dihomo-γ-linolensäure, Arachidonsäure, Stearidonsäure, Eicosatetraensäure oder Eicosapentaensäure. Substrate der verwendeten Desaturasen und Elongasen im erfindungsgemäßen Verfahren sind C₁₆-, C₁₈- oder C₂₀-Fettsäuren wie zum Beispiel Linolsäure, γ-Linolensäure, α-Linolensäure, Dihomo-γ-linolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die synthetisierten C₂₀- oder C₂₂-Fettsäuren mit mindestens zwei, drei, vier, fünf oder sechs Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten. Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden. Beispielsweise können Katzen Arachidonsäure nicht mehr synthetisieren.

Unter "Phospholipiden" im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin. Die Begriffe "Produktion oder Produktivität" sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Es umfasst auch die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff "Effizienz der Produktion" umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff "Ausbeute oder Produkt/Kohlenstoff-Ausbeute" ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe "Biosynthese oder Biosyntheseweg" sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe "Abbau oder Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle), beispielsweise in einem Mehrschritt- und stark regulierten Prozess. Der Begriff "Stoffwechsel" ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Durch den Einsatz der erfindungsgemäßen Polynukleotide und optional weiterer Polynukleotide, die für Enzyme des Lipid oder Fettsäurestoffwechsels kodieren, in dem erfindungsgemäßen Verfahren können verschiedene vorteilhafte Effekte erzielt werden. So kann die Ausbeute, Produktion und/oder Effizienz der Produktion der mehrfach ungesättigten Fettsäuren in einer Pflanze, bevorzugt in einer Ölfruchtpflanze, oder einem Mikroorganismus beeinflusst werden. Die Anzahl oder Aktivität der erfindungsgemäßen Polypeptide bzw. Polynukleotide kann erhöht werden, so dass größere Mengen der Genprodukte und damit letztlich größere Mengen der Verbindungen der allgemeinen Formel I hergestellt werden. Auch eine de novo Synthese in einem Organismus, dem die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines erfindungsgemäßen Polynucleotids in einen Organismus, allein oder in Kombination mit anderen Genen, in eine Zelle kann nicht nur der Biosynthesefluss zum Endprodukt erhöht, sondern auch die entsprechende Triacylglycerin- Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs weiter gesteigert wird. Durch Optimierung der Aktivität oder Erhöhung der Anzahl einer oder mehrerer erfindungsgemäßer Polynukleotide bzw. Polypeptide, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen insbesondere aus Pflanzen zu steigern. Die im Verfahren gewonnenen Fettsäuren eignen sich als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Aus den zuvor gemachten Ausführungen versteht sich, dass die Erfindung auch ein Verfahren zur Herstellung einer Öl-, Lipid- oder Fettsäurezusammensetzung umfassend die Schritte des erfindungsgemäßen Verfahrens und den weiteren Schritt des Formulierens des Stoffes als Öl-, Lipid- oder Fettsäurezusammensetzung betrifft.

In einer bevorzugten Ausführungsform dieses Verfahren wird die Öl-, Lipid- oder Fettsäurezusammensetzung weiter formuliert zu einem Arzneimittel, zu Kosmetik, zu einem Nahrungsmittel, zu einem Futtermittel, vorzugsweise Fischfutter, oder zu einem Nahrungsergänzungsmittel.

Schließlich betrifft die Erfindung grundsätzlich die Verwendung des Polynucleotids, des Vektors, der Wirtszelle, des Polypeptids oder des transgenen, nicht-humanen Organsismus der vorliegenden Erfindung für die Herstellung einer Öl-, Lipid- oder Fettsäurezusammensetzung. Diese ist dann bevorzugt als Arzneimittel, Kosmetik, Nahrungsmittel, Futtermittel, vorzugsweise Fischfutter, oder Nahrungsergänzungsmittel einzusetzen.

### Figuren

Figur 1: Gaschromatographische Analyse von Hefen, die mit dem Plasmid pYES-D9Elo(Ro) transformiert wurden und ohne Fettsäuren (Figur 1A), mit Linolsäure (18:2Δ9,12, Figur 1B) oder mit Linolensäure (18:3Δ9,12,15, Figur 1C) gefüttert wurden.
Figur 2: Gaschromatographische Analyse von Hefen, die mit dem Plasmid pYES-D5Elo(Eg) transformiert und mit keiner Fettsäure (Figur 2A), mit der Fettsäure 18:3Δ6,9,12 (Figur 2B), mit der Fettsäure 20:4Δ5,8,11,14 (Figur 2C) oder mit der Fettsäure 20:5Δ5,8,11,14,17 (Figur 2D) gefüttert wurden.

### Beispiele

### Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

### Beispiel 3: Lipidextraktion aus Hefen

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der eindeutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (z.B. Sigma), definiert werden.

### Beispiel 4: Klonierung und Charakterisierung von Elongase-Genen aus Pythium irregulare

*Pythium irregulare* ATCC 1095 wurde angezogen wie beschrieben in Hong et al. 2002, Plant Physiology 129:354-62. Dazu wurde eine Kultur von *Pythium irregulare* ATCC 1095 für 6 Tage bei 25°C in 3g/L Hefe Extrakt, 3g/L Malz-Extrakt, 5g/L Peptone, 10g/L Glucose und 0,68g/L Kaliumhydrogencarbonat (pH 6,0, 1 M HCl) angezogen. Zellen wurden durch Filtration geerntet und 3x mit destilliertem Wasser gewaschen und mit flüssigem Stickstoff eingefroren. Gefrorene Zellen wurden mittels Mörser und Pistil zu einem feinen Pulver zermahlen. Aus dem Pulver wurde entsprechend der Herstellerangaben mRNA aus der Gesamt RNA mittels Dynabeads oligo(dT)₂₅ (Dynal Biotech) gewonnen. Eine cDNA Bank wurde entsprechend der Herstellerangaben mittels ZAP-cDNA Gigapack III Gold Cloning Kit (Stratagene) konstruiert und nach Standardmethoden durchsucht (Ausubel et al. 1995). Zur Isolierung von Elongase-Genen aus *Pythium irregulare* wurden verschiedene Primer-Sequenzen verwendet (Tab. 1).

**Tabelle 1: Primersequenzen zur Isolierung von Elongasen aus Pythium irregulare.**

| Primer | Sequenz | SEQ ID Nr. |
|---|---|---|
| F4 | GTGGAGGCCGCCATCCAG | 21 |
| R4 | CCTGCACGTTCATGGTCAC | 22 |
| Forward-M | GCGAGATCTGGTGGAAGAAGTATC | 23 |
| Reverse-M | AGTGTAGCCGTTGCGGTAGG | 24 |
| PFLF | ATTAGACAATGGCGACCGAGATG | 25 |
| PFLR | GCATTCTACACGCTCTTGTTCTTC | 26 |

Die Primerkombination PFLF-PFLR lieferte dabei Sequenzen, die Homologien zu bekannten delta 6-Elongasen zeigen (Tab. 2). Die beiden erhaltenen Sequenzen unterschieden sich allerdings nur auf DNA-Ebene. Die translatierten Polypeptid-Sequenzen zeigten keine Unterschiede. Um die Funktionalität der erhaltenen Sequenzen zu untersuchen, wurde mittels PCR durch die Primerkombination PFLF-PFLR ein DNA-Fragment von D6Elo(Pir)_1 hergestellt und in den Hefe-Expressionsvektor pYES2.1/V5-His-TOPO entsprechend Herstellerangaben kloniert, wobei der Vektor pYES-D6Elo(Pir) entstanden ist.

**Tabelle 2: Elongase-Aminosäureseauenzen aus Pvthium irregulare**

| Gen-Name | SEQ ID Nr. | Länge in Aminosäuren |
|---|---|---|
| D6Elo(Pir)_1 | 2 | 281 |
| D6Elo(Pir)_2 | 4 | 281 |

Die korrespondierende Polynukleotidsequenz von D6Elo(Pir)_1 ist in SEQ ID Nr. 1 gezeigt. Die korrespondierende Polynukleotidsequenz von D6Elo(Pir)_2 ist in SEQ ID Nr. 3 dargestellt.

Das Plasmid pYES-d6Elo(Pir) wurde nach Herstellerangaben in den Hefestamm INVSC-1 (Invitrogen) transformiert und auf Platten mit DOB-U Agar basierend auf Uracil Auxotrophie selektioniert. Positive Kolonien wurden durch PCR identifiziert. Dazu wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten. Parallel wurde der leere Vektor pYES2.1/V5-His-TOPO in der beschriebenen Art und Weise in kompetente Hefezellen des Stamms INVSC-1 transformiert. Hefezellen mit dem Plasmid pYES-d6Elo(Pir) bzw. pYES2.1/V5-His-TOPO wurden 12 h in flüssigem DOB-U Medium bei 28 °C, 200 rpm inkubiert und dann weitere 12 h in Induktionsmedium (DOB-U+2% (w/v) Galaktose+ 2% (w/v) Raffinose) sowie 250 µM von ins Medium zugegebenen Fettsäuren angezogen. Anhand der zugegebenen Fettsäuren lässt sich die Spezifität und Aktivität des zu charakterisierenden Gens bestimmen.

Hefen, die mit den Plasmiden pYES2/V5-His-TOPO und pYES2-D6Elo(Pir) transformiert wurden, wurden folgendermaßen analysiert:
Die Hefezellen aus den Hauptkulturen werden durch Zentrifugation (100 x g, 5 min, 20°C) geerntet und mit 100 mM NaHCO₃, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgt durch zweimalige Extraktion mit Petrolether (PE). Zur Entfernung nicht derivatisierter Fettsäuren werden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt.

Die Bedingungen für die GLC-Analyse sind wie folgt: Die Ofentemperatur wurde von 50°C bis 250°C mit einer Rate von 5°C/min und schließlich 10 min bei 250°C (halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Aktivitäts- und Substratbestimmung von D6Elo(Pir)

Die Substratspezifität der D6Elo(Pir) [SEQ ID Nr. 2] wurde nach Expression und Fütterung verschiedener Fettsäuren ermittelt (Tab. 3). Die gefütterten Substrate sind dabei in großen Mengen in allen transgenen Hefen nachzuweisen. Die transgenen Hefen zeigen die Synthese neuer Fettsäuren, den Produkten der D6Elo(Pir)-Reaktion. Dies bedeutet, dass das Gen D6Elo(Pir) funktional exprimiert wurde.

Tabelle 3 beschreibt die Umsetzung von Fettsäuren durch die in die Hefen eingebrachten Plasmide. Die Umsetzungsraten sind in Prozent Elongationseffizienz für die verschiedenen exogen zugegebenen Fettsäuren angegeben. Als Kontrolle für die Aktivität der Gensequenz D6Elo(Pir) fungiert der Leervektor pYES2.1/V5-His-TOPO. Fettsäuren, die in den Hefen mit dem Vektor pYES-D6Elo(Pir) entstanden sind, aber nicht in Hefen mit dem Vektor pYES2.1/V5-His-TOPO, gehen auf die Aktivität des translatierten Produktes von D6Elo(Pir) zurück. Dabei konnte für die Sequenz D6Elo(Pir) eine delta 6-Elongase-Aktivität festgestellt werden, wobei C18-Δ6-desaturierte Fettsäuren (GLA, SDA) das bevorzugte Substrat darstellen. Geringere Aktitivät konnte für C20-Δ6-desaturierte Fettsäuren (ARA, EPA), sowie C18-Δ9- oder C20-Δ11-desaturierte Fettsäuren festgestellt werden.

Überraschenderweise und im Unterschied zu den bisher beschriebenen Δ6-Elongasen (Mortierella alpina, WO00208401, Physcomitrella patens, WO0159128) konnte auch eine bevorzugte Substratspezifität für die Fettsäure C18Δ11 (cis-Vakzensäure) gefunden werden. Damit konnte das Protein D6Elo(Pir) mit der SEQ ID Nr. 2 als Elongase mit Substratspezifität für Δ6- und Δ11-desaturierte C18-Fettsäuren charakterisiert werden.

**Tabelle 3: Elongationseffizienzen in Prozent für verschiedene gefütterte Fettsäuren. Die Elongationseffizienz errechnet sich aus den Peakflächen der gaschromatographischen Analyse nach der Formel: (Fläche Produkt/Fläche Substrat+Produkt)*100.**

| Gefütterte Fettsäure | pYES-D6Elo(Pir) | pYES2.1/V5-His-TOPO |
|---|---|---|
| 18:1Δ11 | 28,6% | 0,0% |
| 18:2Δ9,12 | 11,1% | 1,1% |
| 18:3Δ9,12,15 | 19,1% | 1,4% |
| 18:3Δ6,9,12 | 27,1% | 1,3% |
| 18:4Δ6,9,12,15 | 38,7% | 1,2% |
| 20:1Δ11 | 17,1% | 6,6% |
| 20:4Δ5,8,11,14 | 6,7% | 0,0% |
| 20:5Δ5,8,11,14,17 | 11,7% | 0,0% |

### Beispiel 5: Klonierung und Charakterisierung eines Elongase-Gens aus Rhizopus oryzae

Durch Suche nach konservierten Bereichen in Proteinsequenzen des Pilzes *Rhizopus oryzae* konnte eine Sequenz mit entsprechenden Motiven für eine Elongase identifiziert werden. Um eine vollständige Sequenz zu erhalten, wurde cDNA mit den Primern RhiEloF (gtttacgatggacattgatcaattgaagc) [SEQ ID Nr. 27] und RhiEloR (gttcctaatccaccttctttgcagc) [SEQ ID Nr. 28] in einer PCR für die Amplifzierung eingesetzt. Dabei konnte das Fragment SEQ ID Nr. 5 erhalten werden (Tab. 4).

**Tabelle 4: Elongase Aminosäuresequenz aus Rhizopus oryzae.**

| Gen-Name | SEQ ID Nr. | Länge in Aminosäuren |
|---|---|---|
| D9Elo(Ro) | 6 | 275 |

Die korrespondierende Polynukleotidsequenz von D9Elo(Ro) ist in SEQ ID Nr. 5 gezeigt.

Zur Charakterisierung der Funktion der Sequenz D9Elo(Ro) aus *Rhizopus oryzae* wurde der offenen Leserahmen der DNA stromabwärts des Galactose-induzierbaren GAL1-Promotors von pYES2.1/V5-His-TOPO (Invitrogen) kloniert, wobei das Plasmid pYES-D9Elo(Ro) erhalten wurde.

Das Plasmid pYES-D9Elo(Ro) wurde nach Herstellerangaben in den Hefestamm INVSC-1 (Invitrogen) transformiert und auf Platten mit DOB-U Agar basierend auf Uracil Auxotrophie selektioniert. Positive Kolonien wurden durch PCR identifiziert. Dazu wurde jeweils mit 1 µl aufgetauten Zellen, 200 µM dNTPs, 2,5 U Taq-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten. Parallel wurde der leere Vektor pYES2.1/V5-His-TOPO in der beschriebenen Art und Weise in kompetente Hefezellen des Stamms INVSC-1 transformiert. Hefezellen mit dem Plasmid pYES-D9Elo(Ro) bzw. pYES2.1/V5-His-TOPO wurden 12 h in flüssigem DOB-U Medium bei 28 °C, 200 rpm inkubiert und dann weitere 12 h in Induktionsmedium (DOB-U+2% (w/v) Galaktose+ 2% (w/v) Raffinose) sowie 250 µM von ins Medium zugegebenen Fettsäuren angezogen. Anhand der zugegebenen Fettsäuren lässt sich die Spezifität und Aktivität des zu charakterisierenden Gens bestimmen.

Hefen, die mit den Plasmiden pYES2/V5-His-TOPO und pYES2-D9Elo(Ro) transformiert wurden, wurden folgendermaßen analysiert:
Die Hefezellen aus den Hauptkulturen werden durch Zentrifugation (100 x g, 5 min, 20°C) geerntet und mit 100 mM NaHCO₃, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgt durch zweimalige Extraktion mit Petrolether (PE). Zur Entfernung nicht derivatisierter Fettsäuren werden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wurde von 50°C bis 250°C mit einer Rate von 5°C/min und schließlich 10 min bei 250°C (halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Aktivitäts- und Substrafbestimmung von D9Elo(Ro)

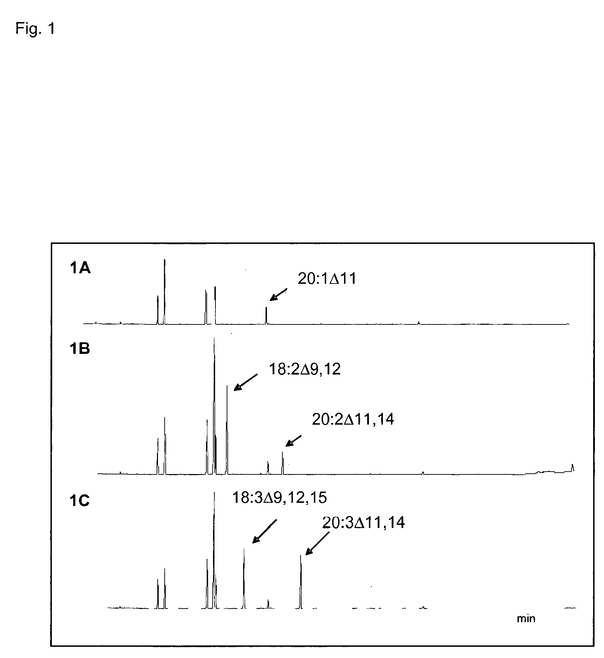

Die Substratspezifität der D9Elo(Ro) [SEQ ID Nr. 6] wurde nach Expression und Fütterung verschiedener Fettsäuren ermittelt (Fig. 1). Figur 1 zeigt die gaschromatographische Analyse von Hefen, die mit dem Plasmid pYES-D9Elo(Ro) transformiert wurden. In Figur 1A wurden Hefen analysiert, die nicht mit exogen zugegebenen Fettsäuren gefüttert wurden. Im Unterschied zur Kontrolle (nicht gezeigt) wurde eine neue Fettsäure in kleinen Mengen gebildet (20:1Δ11). Figur 1B zeigt die Analyse von Hefezellen, die mit dem Plasmid pYES-D9Elo(Ro) transformiert und mit der Fettsäure 18:2Δ9,12 gefüttert wurden. Neben der Fettsäure 18:2Δ9,12, die durch die Fütterung in die Hefe aufgenommen wurde, kann die Synthese von zwei neuen Fettsäuren festgestellt werden: 20:1Δ11 und 20:2Δ11,14. Das Produkt 20:2Δ11,14 geht dabei auf die Elongation von 18:2 zurück. In Figur 1C wurden die Hefezellen nach Transformation mit Plasmid pYES-D9Elo(Ro) und Fütterung mit der Fettsäure 18:3Δ9,12,15 analysiert. Auch hier konnte die Neubildung von zwei Fettsäuren gezeigt werden. Neben der Fettsäure 18:3Δ9,12,15, die durch die Fütterung in die Hefe aufgenommen wurde, wurden zwei neue Fettsäuren gebildet: 20:1Δ11 und in grossen Mengen 20:3Δ11,14,17.

In Tabelle 5 sind die Aktivitäten nach Fütterung mit verschiedenen Fettsäuren zusammengefaßt. Die transgenen Hefen zeigen die Synthese neuer Fettsäuren, den Produkten der D9Elo(Ro)-Reaktion. Dies bedeutet, dass das Gen D9Elo(Ro) funktional exprimiert wurde.

Tabelle 5 zeigt, dass D9Elo(Ro) eine spezifische Aktivität für C18-Δ9-desaturierte Fettsäuren besitzt, wobei sich die höchste Aktivität mit der Fettsäure 18:3Δ9,12,15 (Linolensäure) ergibt. Das Gen D9Elo(Ro) eignet sich damit besonders gut für die Elongation von 18:3Δ9,12,15 zu 20:3Δ11,14,17 (iso-Dihomogammalinolensäure).

**Tabelle 5: Elongationseffizienz in Prozent von verschiedener Fettsäuren durch , D9Elo(Ro) im Vergleich zur Vektorkontrolle pYES2.1/V5-His_TOPO.**

| Gefütterte Fettsäure | D9Elo(Ro) | pYes2.1/V5-His-TOPO |
|---|---|---|
| 16:1Δ9 | 36,6% | 2,4% |
| 18:1Δ9 (Ölsäure) | 8,3% | 0,0% |
| 18:2Δ9,12 (Linolsäure) | 19,3% | 0,0% |
| 18:3Δ9,12,15 (Linolensäure) | 49,3% | 0,0% |

### Beispiel 6: Klonierung und Charakterisierung eines Elongase-Gens aus Euglena gracilis

*Euglena gracilis* wurde angezogen wie beschrieben in Hoffmeister et al. 2005, J Biol Chem 280:4329-38. Zellen wurden durch Filtration geerntet und 3x mit destilliertem Wasser gewaschen und mit flüssigem Stickstoff eingefroren. Gefrorene Zellen wurden mittels Mörser und Pistil zu einem feinen Pulver zermahlen. Aus dem Pulver wurde entsprechend der Herstellerangaben mRNA aus der Gesamt RNA mittels Dynabeads oligo(dT)₂₅ (Dynal Biotech) gewonnen. Eine cDNA Bank wurde entsprechend der Herstellerangaben mittels ZAP-cDNA Gigapack III Gold Cloning Kit (Stratagene) konstruiert und eine EST Sequenzierung durchgeführt. Die Datenbank wurde mittels der Sequenz D6Elo(Pir), [SEQ ID Nr. 1], nach Elongasen durchsucht. Eine Sequenz mit putativen Elongase-Motiven konnte gefunden werden. Zur Isolierung des Elongase-Gens aus *Euglena gracilis* wurden folgende Primer eingesetzt (Tab. 6).

**Tabelle 6: Eingesetzte Primer zur Isolierung von d5Elo(Eg)**

| Primer | Sequenz (5'-3') | SEQ ID Nr. |
|---|---|---|
| E52iF | ggagcttgctacaattcttccatccatg | 29 |
| E52iR | atcccagtgqcatgcacctggta | 30 |
| E52FLF | ctgcacaatggcggatagc | 31 |
| E52FLR | gactcctcagcgtcccatg | 32 |
| E52OFLF | ccatggcggatagcccagtc | 33 |
| E52OFLR | tcagcgtcccatgccgacag | 34 |

Mit dem Primerpaar E52OFLF und E52OFLR konnte die Volllängensequenz D5Elo(Eg) SEQ ID Nr. 7 isoliert werden (Tab. 7). Das entsprechend hergestellte PCR Produkt wurde dann in den Vektor pYES2.1/V5-His-TOPO (Invitrogen) nach Herstellerangaben kloniert. Es wurde das Plasmid pYES-D5Elo(Eg) erhalten.

**Tabelle 7: Elongase Aminosäuresequenz aus Euglena gracilis.**

| Gen-Name | SEQ ID Nr. | Länge in Aminosäuren |
|---|---|---|
| D5Elo(Eg) | 8 | 305 |

Die korrespondierende Polynukleotidsequenz von D5Elo(Eg) ist in SEQ ID Nr. 7 gezeigt.

Das Plasmid pYES-D5Elo(Eg) wurde nach Herstellerangaben in den Hefestamm INVSC-1 (Invitrogen) transformiert und auf Platten mit DOB-U Agar basierend auf Uracil-Auxotrophie selektioniert. Positive Kolonien wurden durch PCR identifiziert. Dazu wurde jeweils mit 1 µl aufgetauten Zellen, 200 µM dNTPs, 2,5 U Taq-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten. Parallel wurde der leere Vektor pYES2.1/V5-His-TOPO in der beschriebenen Art und Weise in kompetente Hefezellen des Stamms INVSC-1 transformiert. Hefezellen mit dem Plasmid pYES-D5Elo(Eg) bzw. pYES2.1/V5-His-TOPO wurden 12 h in flüssigem DOB-U Medium bei 28 °C, 200 rpm inkubiert und dann weitere 12 h in Induktionsmedium (DOB-U+2% (w/v) Galaktose+ 2% (w/v) Raffinose) sowie 250 µM von ins Medium zugegebenen Fettsäuren angezogen. Anhand der zugegebenen Fettsäuren lässt sich die Spezifität und Aktivität des zu charakterisierenden Gens bestimmen.

Hefen, die mit den Plasmiden pYES2/V5-His-TOPO und pYES2-D5Elo(Eg) transformiert wurden, wurden folgendermaßen analysiert:
Die Hefezellen aus den Hauptkulturen werden durch Zentrifugation (100 x g, 5 min, 20°C) geerntet und mit 100 mM NaHCO₃, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgt durch zweimalige Extraktion mit Petrolether (PE). Zur Entfernung nicht derivatisierter Fettsäuren werden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wurde von 50°C bis 250°C mit einer Rate von 5°C/min und schließlich 10 min bei 250°C (halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Aktivität- und Substratbestimmung von d5Elo(Eg)

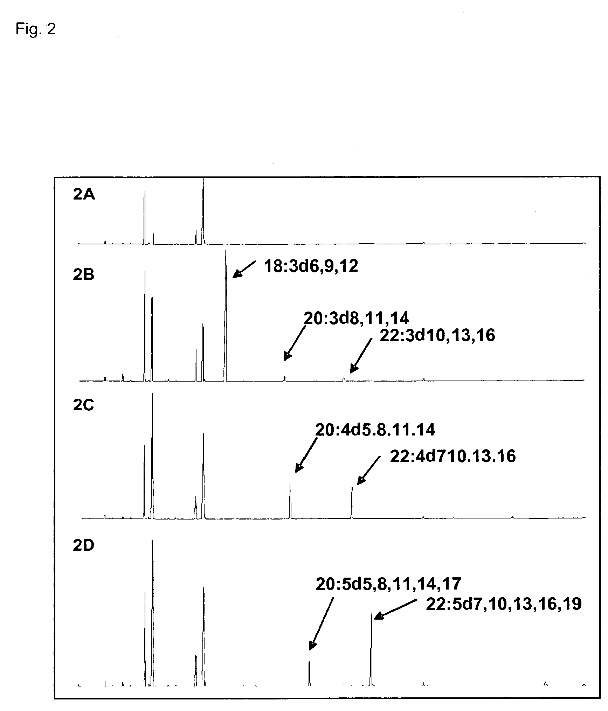

Die Substratspezifität der D5Elo(Eg) [SEQ ID Nr. 8] wurde nach Expression und Fütterung verschiedener Fettsäuren ermittelt (Fig. 2). Figur 2 zeigt die gaschromatographische Analyse von Hefen, die mit dem Plasmid pYES-D5Elo(Eg) transformiert wurden. In Figur 2A wurden Hefen analysiert, die nicht mit exogen zugegebenen Fettsäuren gefüttert wurden. Es wurde keine neue Fettsäure gebildet. Figur 2B zeigt die Analyse von Hefezellen, die mit dem Plasmid pYES-D5Elo(Eg) transformiert und mit der Fettsäure 18:3Δ6,9,12 gefüttert wurden. Neben der Fettsäure 18:3Δ6,9,12, die durch die Fütterung in die Hefe aufgenommen wurde, kann die Synthese von zwei neuen Fettsäuren festgestellt werden: 20:3Δ8,11,14 und 22:3Δ10,13,16. Beide Produkte gehen dabei auf die Elongation von 18:3Δ6,9,12 zurück. In Figur 2C wurden die Hefezellen nach Transformation mit Plasmid pYES-D5Elo(Eg) und Fütterung mit der Fettsäure 20:4Δ5,8,11,14 analysiert. Hier konnte die Neubildung einer Fettsäure gezeigt werden. Neben der Fettsäure 20:4Δ5,8,11,14, die durch die Fütterung in die Hefe aufgenommen wurde, wurde die Fettsäuren 22:4Δ7,10,13,16 neu gebildet. In Figur 2D wurden die Hefezellen nach Transformation mit Plasmid pYES-D5Elo(Eg) und Fütterung mit der Fettsäure 20:5Δ5,8,11,14,17 analysiert. Hier konnte die Neubildung einer Fettsäure gezeigt werden. Neben der Fettsäure 20:5Δ5,8,11,14,17, die durch die Fütterung in die Hefe aufgenommen wurde, wurde die Fettsäuren 22:5Δ7,10,13,16,19 neu gebildet.

In Tabelle 6 sind die Aktivitäten nach Fütterung mit verschiedenen Fettsäuren zusammengefaßt. Die transgenen Hefen zeigen die Synthese neuer Fettsäuren, den Produkten der D5Elo(Eg)-Reaktion. Dies bedeutet, dass das Gen D5Elo(Eg) funktional exprimiert wurde.

**Tabelle 6: Elongationseffizienz in Prozent von verschiedenen Fettsäuren durch D5Elo(Eg) im Vergleich zur Vektorkontrolle pYES2.1/V5-His_TOPO.**

| Gefütterte Fettsäure | D5Elo(EG) | pYes2.1/V5-His-TOPO |
|---|---|---|
| 18:3Δ6,9,12 | 2,9% | 0,9% |
| 20:4Δ5,8,11,14 | 46,1% | 0,0% |
| 20:5Δ5,8,11,14,17 | 78,3% | 0,0% |

Weiterhin zeigt Tabelle 6, dass D5Elo(Eg) eine spezifische Aktivität für C20-Δ5-desaturierte Fettsäuren besitzt, wobei die höchste Aktivität mit der Fettsäure 20:5Δ5,8,11,14,17 (Eicosapentaensäure) erreicht wird. Die Umsetzung von Eicosapentaensäure von D5Elo(Eg) ist dabei deutlich höher als die Umsetzung von anderen Genen mit ähnlicher Enzymaktivität. Das Gen D5Elo(Eg) eignet sich damit besonders gut für die Elongation von 20:5Δ5,8,11,14,17zu 22:5Δ7,10,13,16,19 (Docosapentaensäure). Docosapentaensäure ist die Vorstufe zu Docosahexaensäure (DHA) und ist eine ernährungswissenschaftlich wertvolle ω-3 Fettsäure.

### Beispiel 7: Herstellung transgener Pflanzen zur Produktion von langkettigen mehrfachungesättigten Fettsäuren.

Zur Herstellung von langkettigen mehrfach ungesättigten Fettsäuren in Pflanzen werden die verschiedenen Gene des Stoffwechselweges auf einem binären Vektor kombiniert. Für die Herstellung von Arachidonsäure (20:4Δ5,8,11,14) werden folgende Gene wie in Tabelle 7 beschrieben, kombiniert. Entsprechend werden zur Herstellung der Fettsäure Eicosapentaensäure (20:5Δ5,8,11,14,17) die Gene wie in Tabelle 8 beschrieben, kombiniert. Entsprechend werden zur Herstellung der Fettsäure Docosahexaensäure (22:6Δ4,7,10,13,16,19) die Gene wie in Tabelle 9 beschrieben, kombiniert.

**Tabelle 7: Genkombination zur Herstellung von Arachidonsäure**

| Gene | Aktivität | SEQ ID Nr. |
|---|---|---|
| D6Des(Pir) | Δ6-desaturase | 9 und 10 |
| D6Elo(Pir) | Δ6-elongase | 1 und 2 oder 3 und 4 |
| D5Des(Tc) | Δ5-desaturase | 11 und 12 |
| D12Des(Ps) | Δ12-desaturase | 17 und 18 |

**Tabelle 8: Genkombination zur Herstellung von Eicosapentaensäure**

| Gene | Aktivität | SEQ ID Nr. |
|---|---|---|
| D6Des(Pir) | Δ6-desaturase | 9 und 10 |
| D6Elo(Pir) | Δ6-elongase | 1 und 2 oder 3 und 4 |
| D5Des(Tc) | Δ5-desaturase | 11 und 12 |
| D9Elo(Ro) | Δ9-elongase | 5 und 6 |
| D8Des(Eg) | Δ8-desaturase | 15 und 16 |
| ω3-Des(Pir) | Omega-3-desaturase | 13 und 14 |

**Tabelle 9: Genkombination zur Herstellung von Docosahexaensäure**

| Gene | Aktivität | SEQ ID Nr. |
|---|---|---|
| D6Des(Pir) | Δ6-desaturase | 9 und 10 |
| D6Elo(Pir) | Δ6-elongase | 1 und 2 oder 3 und 4 |
| DSDes(Tc) | Δ5-desaturase | 11 und 12 |
| D9Elo(Ro) | Δ9-elongase | 5 und 6 |
| D8Des(Eg) | Δ8-desaturase | 15 und 16 |
| ω3-Des(Pir) | Omega-3-desaturase | 13 und 14 |
| D5Elo(Eg) | Δ5-elongase | 7 und 8 |
| D4Des(Tc) | Δ4-desaturase | 19 und 20 |

Für die Transformation von Pflanzen werden weiterere Transformationsvektoren auf Basis von pSUN-USP erzeugt. Dazu werden mit folgenden Primerpaaren Notl-Schnittstellen am 5' und 3'-Ende der kodierenden Sequenz eingefügt (siehe folgende Tabelle).

Zusammensetzung des PCR-Ansatzes (50 µL):
5,00 µL Template cDNA
5,00 µL 10x Puffer (Advantage-Polymerase)+ 25mM MgCl₂
5,00 µL 2mM dNTP
1,25 µL je Primer (10 pmol/µL)
0,50 µL Advantage-Polymerase
Die Advantage-Polymerase von Clontech wird eingesetzt.

Reaktionsbedingungen der PCR:
Anlagerungstemperatur: 1 min 55°C
Denaturierungstemperatur: 1 min 94°C
Elongationstemperatur: 2 min 72°C
Anzahl der Zyklen: 35

| Gen | Kodierende Sequenz in bp | Primersequenz (5'-3') | SEQ ID Nr. |
|---|---|---|---|
| D6-Des(Pir) | 846 | Fwd : | 35 |
| | | gcggccgcgccatggtggacctcaagcctgg | |
| | | Rvs : | 36 |
| | | gcggccgttacatcgctgggaactcgg | |
| D5-Des(Tc) | 1320 | Fwd: | 37 |
| | | gcggccgcgccatgggcaagggcagcgaggg | |
| | | Rvs: | 38 |
| | | gcggccgcgcctcagtcctgcttcttggtgtc | |
| D12-Des(Ps) | 1197 | Fwd: | 39 |
| | | gcggccgcgccatggcgatcctgaacccgg | |
| | | Rvs: | 40 |
| | | gcggccgctagagcttgttcttgtaga | |
| O3-Des(Pir) | 1092 | Fwd: | 41 |
| | | gcggccgcgccatggcgacgaaggaggcgtatg | |
| | | Rvs: | 42 |
| | | gcggccgcgccttacgtggacttggtcttgg | |
| D8-Des(Eg) | 1266 | Fwd: | 43 |
| | | gcggccgcgccatgaagtcaaagcgccaagc | |
| | | Rvs: | 44 |
| | | gcggccgcgcccccgcggggaaggctctataa | |
| D6-Elo(Pir) | 846 | Fwd: | 45 |
| | | gcggccgcgccatggcgaccgagatgctgca | |
| | | Rvs: | 46 |
| | | gcggccgctacacgctcttgttcttcttgc | |
| D9-Elo(Ro) | 828 | Fwd: | 47 |
| | | gcggccgcgccatggacattgatcaattgaag | |
| | | Rvs: | 48 |
| | | gcggccgcgcccaatggtgatggtgatgatgac | |
| D5-Elo(Eg) | 918 | Fwd: | 49 |
| | | gcggccgcgccatggcggatagcccagtcatc | |
| | | Rvs: | 50 |
| | | gcggccgcgcctcagcgtcccatgccgacagatc | |
| D4-Des(Tc) | 1560 | Fwd: | 51 |
| | | gcggccgcgccataaccigtcggctacgacga | |
| | | Rvs: | 52 |
| | | gcggccgcgcctcaggcagcgcgctgccagg | |

Die PCR Produkte werden für 4 h bei 37°C mit dem Restriktionsenzym Notl inkubiert. Der Pflanzen-Expressionsvektor pSUN300-USP wird in gleicherweise inkubiert. Anschließend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel purification Kit gemäss Herstellerangaben. Anschliessend werden Vektor und PCR-Produkte ligiert. Dazu wird das Rapid Ligation Kit von Roche verwendet. Die entstandene Plasmide werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des OCS-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCCGGATCTGCTGG CTATGAA-3') [SEQ ID Nr 53].

Das PCR-Fragment wurde mit EcoRI/Sall nachgeschnitten und in den Vektor pSUN300 mit OCS Terminator eingesetzt. Es entstand das Plasmid mit der Bezeichnung pSUN-USP, das für die Pflanzen-Transformation mittels *Agrobacterium tumefaciens* eingesetzt werden kann.

### a) Erzeugung transgener Rapspflanzen (verändert nach Moloney et al., 1992, Plant Cell Reports, 8:238-242)

Zur Erzeugung transgener Rapspflanzen werden binäre Vektoren wie die weiter oben beschrieben pSUN Plasmide mit den entsprechend kombinierten Genen in Agrobacterium tumefaciens C58C1:pGV2260 transformiert (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wird eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 cm²) wurden in einer Petrischale mit einer 1:50 Agrobakterienverdünnung für 5-10 Minuten inkubiert. Es folgt eine 3-tägige Co-Inkubation in Dunkelheit bei 25°C auf 3MS-Medium mit 0,8 % Bacto-Agar. Die Kultivierung wurde nach 3 Tagen mit 16 Stunden Licht / 8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln zum Medium gegeben.

Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Expression der Desaturase- bzw. Elongase-Gene mittels Lipidanalysen untersucht wie beispielhaft in Qiu et al. 2001, J. Biol. Chem. 276, 31561-31566 beschrieben.

### b) Herstellung von transgenen Leinpflanzen

Die Herstellung von transgenen Leinpflanzen können zum Beispiel nach der Methode von Bell et al., 1999, In Vitro Cell. Dev. Biol.-Plant. 35(6):456-465 mittels particle bombartment erzeugt werden. Agrobakterien-vermittelte Transformationen können zum Beispiel nach Mlynarova et al. (1994), Plant Cell Report 13: 282-285 hergestellt werden.

## Patentansprüche

1. Polynucleotid umfassend eine Nukleinsäuresequenz, die ausgewählt ist aus der Gruppe bestehend aus:
(a) Nukleinsäuresequenz wie in einer der SEQ ID No. 1 oder 3 gezeigt;
(b) Nukleinsäuresequenz, die ein Polypeptid kodiert, das eine Aminosäuresequenz wie in einer der SEQ ID No. 2 oder 4 gezeigt aufweist;
(c) Nukleinsäuresequenz, die mindestens 70% identisch zu einer der Nukleinsäuresequenzen aus (a) oder (b) ist und ein Polypeptid mit einer Δ-6-Elongase Aktivität kodiert, wobei das Polypeptid die Fettsäure cis-Vakzensäure (C18:1Δ11), Arachidonsäure (C20:4Δ5,8,11,14) und/oder Eicosapentaensäure (C20:5Δ5,8,11,14,17) elongieren kann; und
(d) Nukleinsäuresequenz für ein Fragment einer Nukleinsäure aus (a), (b) oder (c), wobei das Fragment ein Polypeptid mit einer Δ-6-Elongase Aktivität kodiert, wobei das Polypeptid die Fettsäure cis-Vakzensäure (C18:1Δ11), Arachidonsäure (C20:4Δ5,8,11,14) und/oder Eicosapentaensäure (C20:5Δ5,8,11,14,17) elongieren kann

2. Polynucleotid nach Anspruch 1, wobei das Polynucleotid aus RNA oder DNA besteht.

3. Vektor umfassend das Polynucleotid nach Anspruch 1 oder 2.

4. Vektor nach Anspruch 3, wobei der Vektor ein Expressionsvektor ist.

5. Vektor nach Anspruch 3 oder 4, wobei der Vektor mindestens ein weiteres Polynucleotid umfasst, das ein weiteres Enzym kodiert, das in die Biosynthese von Lipiden oder Fettsäuren eingebunden ist.

6. Wirtszelle umfassend das Polynucleotid nach Anspruch 1 oder 2 oder den Vektor nach einem der Ansprüche 3 bis 5.

7. Wirtszelle nach Anspruch 6, wobei die Wirtszelle zusätzlich mindestens ein weiteres Enzym umfasst, das in die Biosynthese von Lipiden oder Fettsäuren eingebunden ist.

8. Vektor nach Anspruch 5 oder Wirtszelle nach Anspruch 7 wobei das Enzym ausgewählt ist aus der Gruppe bestehend aus: Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n); Lipoxygenase(n), Triacylglycerol-Lipase(n), Allenoxid-Synthase(n), Hydroperoxid-Lyase(n), und Fettsäure-Elongase(n), bevorzugt bestehend aus Δ5-Desaturase(n), Δ6-Desaturase(n), Δ8-Desaturase(n), Δ12-Desaturase(n) und omega-3-Desaturase(n).

9. Verfahren zur Herstellung eines Polypeptids mit Δ-6-Elongase Aktivität umfassend die Schritte:
(a) Exprimieren eines Polynucleotids nach Anspruch 1 oder 2 in einer Wirtszelle; und
(b) Gewinnen des Polypeptids, das von dem Polynucleotid kodiert wird, aus der Wirtszelle.

10. Polypeptid, das von dem Polynucleotid gemäß Anspruch 1 oder 2 kodiert wird oder das erhätlich durch das Verfahren nach Anspruch 9 ist.

11. Antikörper, der das Polypeptid nach Anspruch 10 spezifisch erkennt.

12. Transgener, nicht-humaner Organismus umfassend das Polynucleotid nach Anspruch 1 oder 2, den Vektor nach einem der Ansprüche 3 bis 5 oder die Wirtszelle nach Anspruch 8.

13. Transgener, nicht-humaner Organismus nach Anspruch 12, wobei der Organismus ein Tier, eine Pflanze oder ein multizellulärer Mikroorganismus ist.

14. Verfahren zur Herstellung eines Stoffes, der die Struktur aufweist, die in der folgenden allgemeinen Formel I gezeigt wird 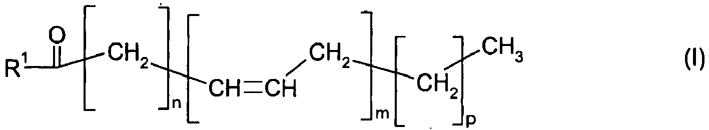 wobei die Variablen und Substituenten die folgenden sind
R¹ = Hydroxyl, Coenzym A (Thioester), Lysophosphatidylcholin, Lysophosphatidylethanolamin, Lysophosphatidylglycerol, Lysodiphosphatidylglycerol, Lysophosphatidylserin, Lysophosphatidylinositol, Sphingo- Base oder ein Radikal der Formel II 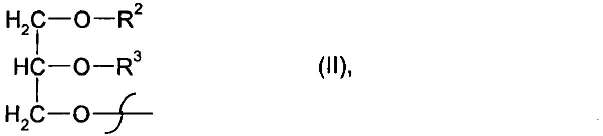
R² = Wasserstoff, Lysophosphatidylcholin, Lysophosphatidylethanolamin, Lysophosphatidylglycerol, Lysodiphosphatidylglycerol, Lysophosphatidylserin, Lysophosphatidylinositol oder gesättigtes oder ungesättigtes C₂-C₂₄-Alkylcarbonyl,
R³ = Wasserstoff, gesättigtes oder ungesättigtes C₂-C₂₄-Alkylcarbonyl, oder R² und R³ sind unabhängig voneinander ein Radikal der Formel la: 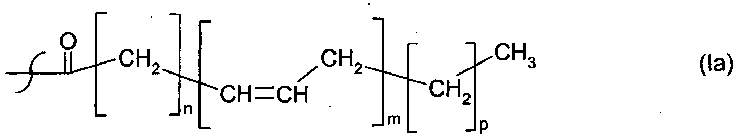
n = 2, 3, 4, 5, 6, 7 or 9, m = 2, 3, 4, 5 oder 6 und p = 0 oder 3;
und
wobei das Verfahren das Kultivieren von (i) einer Wirtszelle nach einem der Ansprüche 6 bis 8 oder (ii) eines transgenen, nicht-humanen Organismus nach Anspruch 12 oder 13 unter Bedingungen umfasst, die die Biosynthese des Stoffes erlauben.

15. Verfahren zur Herstellung einer Öl-, Lipid- oder Fettsäurezusammensetzung umfassend die Schritte des Verfahrens nach Anspruch 14 und den weiteren Schritt des Formulierens des Stoffes als Öl-, Lipid- oder Fettsäurezusammensetzung.

16. Verfahren nach Anspruch 15, wobei die Öl-, Lipid- oder Fettsäurezusammensetzung weiter formuliert wird zu einem Arzneimittel, zu Kosmetik, zu einem Nahrungsmittel, zu einem Futtermittel, vorzugsweise Fischfutter, oder zu einem Nahrungsergänzungsmittel.

17. Verwendung des Polynucleotids nach Anspruch 1 oder 2, des Vektors nach einem der Ansprüche 3 bis 5, der Wirtszelle nach Anspruch 6 oder 7, des Vektors oder der Wirtszelle nach Anspruch 8, des Polypeptids nach Anspruch 10 oder des transgenen, nicht-humanen Organismus nach Anspruch 12 oder 13 für die Herstellung einer Öl-, Lipid- oder Fettsäurezusammensetzung.

18. Verwendung nach Anspruch 17, wobei die Öl-, Lipid- oder Fettsäurezusammensetzung einzusetzen ist als Arzneimittel, Kosmetik, Nahrungsmittel, Futtermittel, vorzugsweise Fischfutter, oder Nahrungsergänzungsmittel.

## Claims

1. A polynucleotide comprising a nucleic acid sequence selected from the group consisting of:
(a) nucleic acid sequence as shown in either of SEQ ID NO. 1 or 3;
(b) nucleic acid sequence which codes for a polypeptide which features an amino acid sequence as shown in either SEQ ID NO. 2 or 4;
(c) nucleic acid sequence which has at least 70% identity to one of the nucleic acid sequences of (a) or (b), and which codes for a polypeptide with Δ-6-elongase activity, the polypeptide being able to elongate the fatty acid cis-vaccenic acid (C18:1Δ11), arachidonic acid (C20;4Δ5, 8, 11, 14) and/or eicosapentaenoic acid (C20: 5Δ5, 8, 11, 14,17); and
(d) nucleic acid sequence for a fragment of a nucleic acid of (a), (b) or (c), where the fragment codes for a polypeptide with Δ-6-elongase activity, the polypeptide being able to elongate the fatty acid cis-vaccenic acid (C18:1Δ11), arachidonic acid (C20:4Δ5, 8, 11, 14) and/or eicosapentaenoic acid (C20:5Δ5, 8, 11, 14, 17).

2. The polynucleotide according to claim 1, wherein the polynucleotide consists of RNA or DNA.

3. A vector comprising the polynucleotide according to claim 1 or 2.

4. The vector according to claim 3, wherein the vector is an expression vector.

5. The vector according to claim 3 or 4, wherein the vector comprises at least one further polynucleotide which codes for a further enzyme which is involved in the biosynthesis of lipids or fatty acids.

6. A host cell comprising the polynucleotide according to claim 1 or 2 or the vector according to any of claims 3 to 5.

7. The host cell according to claim 6, wherein the host cell additionally comprises at least one further enzyme which is involved in the biosynthesis of lipids or fatty acids.

8. The vector according to claim 5 or the host cell according to claim 7, wherein the enzyme is selected from the group consisting of: acyl-CoA dehydrogenase(s), acyl-ACP [= acyl carrier protein] desaturase(s), acyl-ACP thioesterase(s), fatty acid acyltransferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenase(s), lipoxygenase(s), triacylglycerol lipase(s), allene oxide synthase(s), hydroperoxide lyase(s), fatty acid elongase(s), preferably consisting of Δ5-desaturase(s), Δ6-desaturase(s), Δ8-desaturase(s), Δ12-desaturase(s) and omega-3-desaturase(s).

9. A method of generating a polypeptide with Δ-6-elongase activity, comprising the steps:
(a) expressing a polynucleotide according to claim 1 or 2 in a host cell; and
(b) obtaining, from the host cell, the polypeptide which is encoded by the polynucleotide.

10. A polypeptide which is encoded by the polynucleotide according to claim 1 or 2 or which is obtainable by the method according to claim 9.

11. An antibody which specifically recognizes the polypeptide according to claim 10.

12. A transgenic, nonhuman organism comprising the polynucleotide according to claim 1 or 2, the vector according to any of claims 3 to 5 or the host cell according to claim 8.

13. The transgenic, nonhuman organism according to claim 12, wherein the organism is an animal, a plant or a multicellular microorganism.

14. A process for the production of a substance which has the structure shown in the general formula I hereinbelow 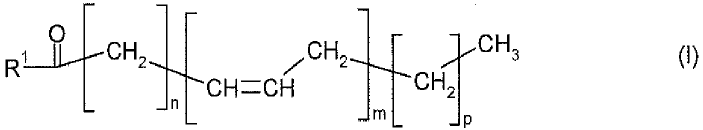 where the variables and substituents are as follows:
R¹ = hydroxyl, coenzyme A (thioester), lysophosphatidylcholine, lysophosphatidylethanolamine, lysophosphatidylglycerol, lysodiphosphatidylglycerol, lysophosphatidylserine, lysophosphatidylinositol, sphingo base or a radical of the formula II 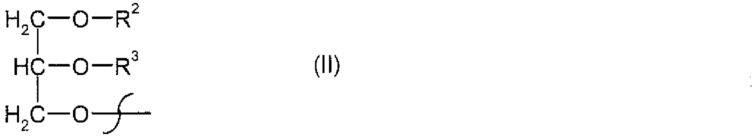
R² = hydrogen, lysophosphatidylcholine, lysophosphatidylethanolamine, lysophosphatidylglycerol, lysodiphosphatidylglycerol, lysophosphatidylserine, lysophosphatidylinositol or saturated or unsaturated C₂-C₂₄-alkylcarbonyl,
R³ = hydrogen, saturated or unsaturated C₂-C₂₄-alkylcarbonyl, or R² and R³ independently of one another are a radical of the formula Ia: 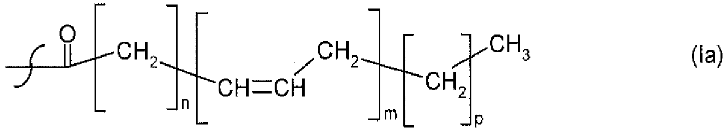
n = 2, 3, 4, 5, 6, 7 or 9, m = 2, 3, 4, 5 or 6 and p = 0 or 3;
and
wherein the process comprises the cultivation of (i) a host cell according to any of claims 6 to 8 or (ii) of a transgenic, nonhuman organism according to claim 12 or 13 under conditions which permit the biosynthesis of the substance.

15. A process for the production of an oil, lipid or fatty acid composition, comprising the steps of the process according to claim 14 and the further step of formulating the substance as an oil, lipid or fatty acid composition.

16. The process according to claim 15, wherein the oil, lipid or fatty acid composition is formulated further to give a drug, a cosmetic product, a foodstuff, a feedstuff, preferably fish food, or a food supplement.

17. The use of the polynucleotide according to claim 1 or 2, of the vector according to any of claims 3 to 5, of the host cell according to claim 6 or 7, of the vector or the host cell according to claim 8, of the polypeptide according to claim 10 or of the transgenic, nonhuman organism according to claim 12 or 13 for the production of an oil, lipid or fatty acid composition.

18. The use according to claim 17, wherein the oil, lipid or fatty acid composition is to be employed as a drug, cosmetic product, foodstuff, feedstuff, preferably fish food, or food supplement.

## Revendications

1. Polynucléotide comprenant une séquence d'acide nucléique qui est choisie dans le groupe consistant en :
(a) la séquence d'acide nucléique telle que présentée dans l'une des séquences SEQ ID NO:1 ou 3 ;
(b) la séquence d'acide nucléique qui code pour un polypeptide qui a une séquence d'acides aminés telle que présentée dans l'une des séquences SEQ ID NO:2 ou 4 ;
(c) la séquence d'acide nucléique qui a une identité d'au moins 70 % avec l'une des séquences d'acides nucléiques de (a) ou (b) et qui code pour un polypeptide ayant une activité de Δ-6-élongase, le polypeptide pouvant allonger l'acide gras acide cis-vaccénique (C18:1 Δ11), l'acide arachidonique (C20:4 Δ5,8,11,14) et/ou l'acide éicosapentaénoïque (C20:5 Δ5,8,11,14,17) ; et
(d) la séquence d'acide nucléique pour un fragment d'un acide nucléique de (a), (b) ou (c), le fragment codant pour un polypeptide ayant une activité de Δ-6-élongase, le polypeptide pouvant allonger l'acide gras acide cis-vaccénique (C18:1 Δ11), l'acide arachidonique (C20:4 Δ5,8,11,14) et/ou l'acide éicosapentaénoïque (C20:5 Δ5,8,11,14,17).

2. Polypeptide selon la revendication 1, le polypeptide étant constitué d'ARN ou d'ADN.

3. Vecteur comprenant le polypeptide selon la revendication 1 ou 2.

4. Vecteur selon la revendication 3, le vecteur étant un vecteur d'expression.

5. Vecteur selon la revendication 3 ou 4, le vecteur comprenant au moins un autre polynucléotide, qui code pour une autre enzyme, qui participe à la biosynthèse de lipides ou d'acides gras.

6. Cellule hôte comprenant le polynucléotide selon la revendication 1 ou 2 ou le vecteur selon l'une des revendications 3 à 5.

7. Cellule hôte selon la revendication 6, la cellule hôte comprenant en outre au moins une autre enzyme, qui participe à la biosynthèse de lipides ou d'acides gras.

8. Vecteur selon la revendication 5 ou cellule hôte selon la revendication 7, l'enzyme étant choisie dans le groupe consistant en la ou les acyl-CoA-déshydrogénases, la ou les acyl-ACP[= protéine porteuse d'acyle]-désaturases, la ou les acyl-ACP-thioestérases, la ou les acide gras-acyl-transférases, la ou les acyl-CoA:lysophospholipide-acyltransférases, la ou les acide gras-synthases, la ou les acide gras-hydroxylases, la ou les acétyl-coenzyme A-carboxylases, la ou les acyl-coenzyme A-oxydases, la ou les acide gras-désaturases, la ou les acide gras-acétylénases, la ou les lipoxygénases, la ou les triacylglycérol-lipases, la ou les oxyde d'allène-synthases, la ou les hydroperoxyde-lyases, et la ou les acide gras-élongases, consistant de préférence en la ou les Δ5-désaturases, la ou les Δ6-désaturases, la ou les Δ8-désaturases, la ou les Δ12-désaturases et la ou les oméga-3-désaturases,

9. Procédé de fabrication d'un polypeptide ayant une activité de Δ-6-élongase, comprenant les étapes :
(a) expression d'un polynucléotide selon la revendication 1 ou 2 dans une cellule hôte ; et
(b) obtention, à partir de la cellule hôte, du polypeptide qui est codé par le polynucléotide.

10. Polypeptide qui est codé par le polypeptide selon la revendication 1 ou 2 ou qui peut être obtenu par le procédé selon la revendication 9.

11. Anticorps qui reconnaît spécifiquement le polypeptide selon la revendication 10.

12. Organisme transgénique non-humain, comprenant le polypeptide selon la revendication 1 ou 2, le peptide selon l'une des revendication 3 à 5 ou la cellule hôte selon la revendication 8.

13. Organisme transgénique non-humain selon la revendication 12, l'organisme étant un animal, une plante ou un microorganisme multicellulaire.

14. Procédé de fabrication d'une substance ayant la structure présentée dans la formule générale I ci-après 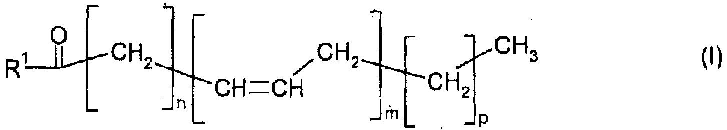 dans laquelle les variables et substituants sont les suivants :
R¹ = hydroxyle, coenzyme A (thioester), lysophosphatidylcholine, lysophosphatidyléthanolamine, lysophosphatidylglycérol, lysodiphosphatidylglycérol, lysophosphatidylsérine, lysophosphatidylinositol, sphingo-base, ou un radical de formule II 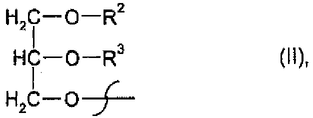
R² = hydrogène, lysophosphatidylcholine, lysophosphatidyléthanolamine, lysophasphatidylglycérol, lysodiphosphatidylglycérol, lysophosphatidylsérine, lysophosphatidylinositol ou alkylcarbonyle en C₂-C₂₄ saturé ou insaturé,
R³ = un atome d'hydrogène, un radical alkylcarbonyle en C₂-C₂₄ saturé ou insaturé, ou R² et R³ représentent chacun indépendamment de l'autre un radical de formule Ia : 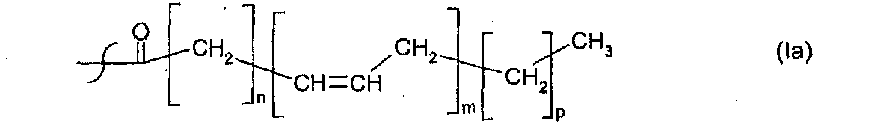
n - 2, 3, 4, 5, 6, 7, ou 9, m = 2, 3, 4, 5 ou 6, et p = 0 ou 3 ;
et
le procédé comprenant la culture (i) d'une cellule hôte selon l'une des revendications 6 à 8 ou (ii) d'un organisme transgénique non-humain selon la revendication 12 ou 13, dans des conditions permettant la biosynthèse de la substance.

15. Procédé de fabrication d'une composition d'huiles, de lipides ou d'acides gras, comprenant les étapes du procédé selon la revendication 14, et l'étape supplémentaire de formulation de la substance en tant que composition d'huiles, de lipides ou d'acides gras.

16. Procédé selon la revendication 15, dans lequel la composition d'huiles, de lipides ou d'acides gras est en outre formulée en un médicament, en un produit cosmétique, en un produit pour l'alimentation humaine, en un produit pour l'alimentation animale, de préférence en un produit pour l'alimentation des poissons, ou en un complément alimentaire.

17. Utilisation du polynucléotide selon la revendication 1 ou 2, du vecteur selon l'une des revendications 3 à 5, de la cellule hôte selon la revendication 6 ou 7, du vecteur ou de la cellule hôte selon la revendication 8, du polypeptide selon la revendication 10 ou de l'organisme transgénique non-humain selon la revendication 12 ou 13, pour la fabrication d'une composition d'huiles, de lipides ou d'acides gras,

18. Utilisation selon la revendication 17, pour laquelle la composition d'huiles, de lipides ou d'acides gras est destinée à être utilisée en tant que médicament, produit cosmétique, produit pour l'alimentation humaine, produit pour l'alimentation animale, de préférence produit pour l'alimentation des poissons, ou complément alimentaire.


### SEQUENCE LISTING

<110> BASF Plant Science GmbH
<120> Elongase und Verfahren zur Herstellung mehrfach ungesättigter Fettsäuren in transgenen Organismen
<130> BPS64972PC
<150> EP 07113547.9
   <151> 2007-07-31
<160> 53
<170> PatentIn version 3.4
<210> 1
   <211> 846
   <212> DNA
   <213> Pythium irregulare
<220>
   <221> CDS
   <222> (1)..(846)
<400> 1
   atg gcg acc gag atg ctg cag agc tac tac gac tgg gct gaa gcc acg      48
   Met Ala Thr Glu Met Leu Gln Ser Tyr Tyr Asp Trp Ala Glu Ala Thr
   1               5                   10                  15
   gag ctc aag ctc ttc gag tgg gtg gac ccg aac ggc gcc tac aag gtg      96
   Glu Leu Lys Leu Phe Glu Trp Val Asp Pro Asn Gly Ala Tyr Lys Val
               20                  25                  30
   cat ccg cag gct gac tac cca ctc gcc aac ttt gcg agc gtg tac gcg     144
   His Pro Gln Ala Asp Tyr Pro Leu Ala Asn Phe Ala Ser Val Tyr Ala
           35                  40                  45
   atc tgc gtc ggt tac ctg ctc ttc gtg atc ttc ggc acg gcg ctc atg     192
   Ile Cys Val Gly Tyr Leu Leu Phe Val Ile Phe Gly Thr Ala Leu Met
       50                  55                  60
   aag gcc ggt gtg ccg gcg ctt aac acc tcg gcg ctc cag ttt gtc tac     240
   Lys Ala Gly Val Pro Ala Leu Asn Thr Ser Ala Leu Gln Phe Val Tyr
   65                  70                  75                  80
   aac ccg ctg cag gtc atc gtc tgc tct tac atg tgc ctc gag gcc gcg     288
   Asn Pro Leu Gln Val Ile Val Cys Ser Tyr Met Cys Leu Glu Ala Ala
                   85                  90                  95
   att cag gcc tac cgc aac ggc tac act gcg gcg ccg tgc aac gac ttc     336
   Ile Gln Ala Tyr Arg Asn Gly Tyr Thr Ala Ala Pro Cys Asn Asp Phe
               100                 105                 110
   aac gcg gcc aag cca gtg atg gca aac gtg ctt tac ctg ttc ttc atc     384
   Asn Ala Ala Lys Pro Val Met Ala Asn Val Leu Tyr Leu Phe Phe Ile
           115                 120                 125
   tcc aaa atc ctc gac ctg tgc gac acg ttc ttc atc atc atg ggc aag     432
   Ser Lys Ile Leu Asp Leu Cys Asp Thr Phe Phe Ile Ile Met Gly Lys
       130                 135                 140
   aag tgg aag cag ctc tcg gta ctg cac gtg tac cac cac ctg acc gtg     480
   Lys Trp Lys Gln Leu Ser Val Leu His Val Tyr His His Leu Thr Val
   145                 150                 155                 160
   ctc ttt ttc tac tac atc gcg tac cgc tgc gct cag gat ggc gat atc     528
   Leu Phe Phe Tyr Tyr Ile Ala Tyr Arg Cys Ala Gln Asp Gly Asp Ile
                       165                     170                     175
   tac gtc tcg atc gtg ctc aac ggc ttc gtg cac aca atc atg tat acg     576
   Tyr Val Ser Ile Val Leu Asn Gly Phe Val His Thr Ile Met Tyr Thr
                   180                     185                     190
   tac tac ttt gtg agc tcg cac acg cgc gag atc tgg tgg aag aag tat     624
   Tyr Tyr Phe Val Ser Ser His Thr Arg Glu Ile Trp Trp Lys Lys Tyr
               195                     200                     205
   ctc acg tcc atg cag ctc atc cag ttc gtg ctc atg aac gtg cag ggc     672
   Leu Thr Ser Met Gln Leu Ile Gln Phe Val Leu Met Asn Val Gln Gly
       210                     215                     220
   tac atg acg tac gcg cgc gag tgc ccg ggc atg ccg cgc aag gta ccg     720
   Tyr Met Thr Tyr Ala Arg Glu Cys Pro Gly Met Pro Arg Lys Val Pro
   225                     230                     235                     240
   atc ctc tac ttg ggc tac gtg cag tcg ctc ttc tgg ctc ttc atg aac     768
   Ile Leu Tyr Leu Gly Tyr Val Gln Ser Leu Phe Trp Leu Phe Met Asn
                   245                     250                     255
   ttc tac atc cgt gcc tat gtg ctc ggc ggc ggt gcc cgg aag cct gtg     816
   Phe Tyr Ile Arg Ala Tyr Val Leu Gly Gly Gly Ala Arg Lys Pro Val
               260                     265                     270
   gca gcc ggc aag aag aac aag agc gtg tag                             846
   Ala Ala Gly Lys Lys Asn Lys Ser Val
           275                     280
<210> 2
   <211> 281
   <212> PRT
   <213> Pythium irregulare
<400> 2
   Met Ala Thr Glu Met Leu Gln Ser Tyr Tyr Asp Trp Ala Glu Ala Thr
   1               5                   10                  15
   Glu Leu Lys Leu Phe Glu Trp Val Asp Pro Asn Gly Ala Tyr Lys Val
               20                  25                  30
   His Pro Gln Ala Asp Tyr Pro Leu Ala Asn Phe Ala Ser Val Tyr Ala
           35                  40                  45
   Ile Cys Val Gly Tyr Leu Leu Phe Val Ile Phe Gly Thr Ala Leu Met
       50                  55                  60
   Lys Ala Gly Val Pro Ala Leu Asn Thr Ser Ala Leu Gln Phe Val Tyr
   65                  70                  75                  80
   Asn Pro Leu Gln Val Ile Val Cys Ser Tyr Met Cys Leu Glu Ala Ala
                   85                  90                  95
   Ile Gln Ala Tyr Arg Asn Gly Tyr Thr Ala Ala Pro Cys Asn Asp Phe
               100                 105                 110
   Asn Ala Ala Lys Pro Val Met Ala Asn Val Leu Tyr Leu Phe Phe Ile
           115                 120                 125
   Ser Lys Ile Leu Asp Leu Cys Asp Thr Phe Phe Ile Ile Met Gly Lys
       130                 135                 140
   Lys Trp Lys Gln Leu Ser Val Leu His Val Tyr His His Leu Thr Val
   145                 150                 155                 160
   Leu Phe Phe Tyr Tyr Ile Ala Tyr Arg Cys Ala Gln Asp Gly Asp Ile
                   165                 170                 175
   Tyr Val Ser Ile Val Leu Asn Gly Phe Val His Thr Ile Met Tyr Thr
               180                 185                 190
   Tyr Tyr Phe Val Ser Ser His Thr Arg Glu Ile Trp Trp Lys Lys Tyr
           195                 200                 205
   Leu Thr Ser Met Gln Leu Ile Gln Phe Val Leu Met Asn Val Gln Gly
       210                 215                 220
   Tyr Met Thr Tyr Ala Arg Glu Cys Pro Gly Met Pro Arg Lys Val Pro
   225                 230                 235                 240
   Ile Leu Tyr Leu Gly Tyr Val Gln Ser Leu Phe Trp Leu Phe Met Asn
                   245                 250                 255
   Phe Tyr Ile Arg Ala Tyr Val Leu Gly Gly Gly Ala Arg Lys Pro Val
               260                 265                 270
   Ala Ala Gly Lys Lys Asn Lys Ser Val
           275                 280
<210> 3
   <211> 846
   <212> DNA
   <213> Pythium irregulare
<220>
   <221> CDS
   <222> (1)..(846)
<400> 3
   atg gcg acc gag atg ctg cag agc tac tac gac tgg gct gaa gcc acg        48
   Met Ala Thr Glu Met Leu Gln Ser Tyr Tyr Asp Trp Ala Glu Ala Thr
   1               5                   10                  15
   gag ctc aag ctc ttc gag tgg gtg gac ccg aac ggc gcc tac aag gtg        96
   Glu Leu Lys Leu Phe Glu Trp Val Asp Pro Asn Gly Ala Tyr Lys Val
               20                  25                  30
   cat ccg cag gct gac tac cca ctc gcc aac ttt gcg agc gtg tac gcg       144
   His Pro Gln Ala Asp Tyr Pro Leu Ala Asn Phe Ala Ser Val Tyr Ala
           35                  40                  45
   atc tgc gtc ggt tac ctg ctc ttc gtg atc ttc ggc acg gcg ctc atg       192
   Ile Cys Val Gly Tyr Leu Leu Phe Val Ile Phe Gly Thr Ala Leu Met
       50                  55                  60
   aag gcc ggc gtg ccg gcg ctt aac acc tcg gcg ctc cag ttt gtc tac       240
   Lys Ala Gly Val Pro Ala Leu Asn Thr Ser Ala Leu Gln Phe Val Tyr
   65                  70                  75                  80
   aac ccg ctg cag gtc atc gtc tgc tct tac atg tgc ctc gag gcc gcg      288
   Asn Pro Leu Gln Val Ile Val Cys Ser Tyr Met Cys Leu Glu Ala Ala
                   85                  90                  95
   att cag gcc tac cgc aac ggc tac act gcg gcg ccg tgc aac gac ttc      336
   Ile Gln Ala Tyr Arg Asn Gly Tyr Thr Ala Ala Pro Cys Asn Asp Phe
               100                 105                 110
   aac gcg gcc aag ccg gtg atg gca aac gtg ctt tac ctg ttc ttc atc      384
   Asn Ala Ala Lys Pro Val Met Ala Asn Val Leu Tyr Leu Phe Phe Ile
           115                 120                 125
   tcc aag atc ctc gac ctg tgc gac acg ttc ttc atc atc atg ggc aag      432
   Ser Lys Ile Leu Asp Leu Cys Asp Thr Phe Phe Ile Ile Met Gly Lys
       130                 135                 140
   aag tgg aag cag ctc tcg gta ctg cac gtg tac cac cac ctg acc gtg      480
   Lys Trp Lys Gln Leu Ser Val Leu His Val Tyr His His Leu Thr Val
   145                 150                 155                 160
   ctc ttt ttc tac tac atc gcg tac cgc tgc gct cag gat ggc gat atc      528
   Leu Phe Phe Tyr Tyr Ile Ala Tyr Arg Cys Ala Gln Asp Gly Asp Ile
                   165                 170                 175
   tac gtc tcg atc gta ctc aac ggc ttc gtg cac aca atc atg tac acg      576
   Tyr Val Ser Ile Val Leu Asn Gly Phe Val His Thr Ile Met Tyr Thr
               180                 185                 190
   tac tac ttt gtg agc tcg cac acg cgc gag atc tgg tgg aag aag tat      624
   Tyr Tyr Phe Val Ser Ser His Thr Arg Glu Ile Trp Trp Lys Lys Tyr
           195                 200                 205
   ctc acg tcc atg cag ctc atc cag ttc gta ctc atg aac gtg cag ggc      672
   Leu Thr Ser Met Gln Leu Ile Gln Phe Val Leu Met Asn Val Gln Gly
       210                 215                 220
   tac atg acg tac gcg cgc gag tgc ccg ggc atg ccg cgc aaa gta ccg      720
   Tyr Met Thr Tyr Ala Arg Glu Cys Pro Gly Met Pro Arg Lys Val Pro
   225                 230                 235                 240
   atc ctc tac ttg ggc tac gtg cag tcg ctc ttc tgg ctc ttc atg aac      768
   Ile Leu Tyr Leu Gly Tyr Val Gln Ser Leu Phe Trp Leu Phe Met Asn
                   245                 250                 255
   ttc tac atc cgt gcc tat gtg ctc ggc ggc ggt gcc cgg aag cct gtt      816
   Phe Tyr Ile Arg Ala Tyr Val Leu Gly Gly Gly Ala Arg Lys Pro Val
               260                 265                 270
   gca gcc ggc aag aag aac aag agc gtg tag                              846
   Ala Ala Gly Lys Lys Asn Lys Ser Val
           275                 280
<210> 4
   <211> 281
   <212> PRT
   <213> Pythium irregulare
<400> 4
   Met Ala Thr Glu Met Leu Gln Ser Tyr Tyr Asp Trp Ala Glu Ala Thr
   1               5                   10                  15
   Glu Leu Lys Leu Phe Glu Trp Val Asp Pro Asn Gly Ala Tyr Lys Val
               20                  25                  30
   His Pro Gln Ala Asp Tyr Pro Leu Ala Asn Phe Ala Ser Val Tyr Ala
           35                  40                  45
   Ile Cys Val Gly Tyr Leu Leu Phe Val Ile Phe Gly Thr Ala Leu Met
       50                  55                  60
   Lys Ala Gly Val Pro Ala Leu Asn Thr Ser Ala Leu Gln Phe Val Tyr
   65                  70                  75                  80
   Asn Pro Leu Gln Val Ile Val Cys Ser Tyr Met Cys Leu Glu Ala Ala
                   85                  90                  95
   Ile Gln Ala Tyr Arg Asn Gly Tyr Thr Ala Ala Pro Cys Asn Asp Phe
               100                 105                 110
   Asn Ala Ala Lys Pro Val Met Ala Asn Val Leu Tyr Leu Phe Phe Ile
           115                 120                 125
   Ser Lys Ile Leu Asp Leu Cys Asp Thr Phe Phe Ile Ile Met Gly Lys
       130                 135                 140
   Lys Trp Lys Gln Leu Ser Val Leu His Val Tyr His His Leu Thr Val
   145                 150                 155                 160
   Leu Phe Phe Tyr Tyr Ile Ala Tyr Arg Cys Ala Gln Asp Gly Asp Ile
                   165                 170                 175
   Tyr Val Ser Ile Val Leu Asn Gly Phe Val His Thr Ile Met Tyr Thr
               180                 185                 190
   Tyr Tyr Phe Val Ser Ser His Thr Arg Glu Ile Trp Trp Lys Lys Tyr
           195                 200                 205
   Leu Thr Ser Met Gln Leu Ile Gln Phe Val Leu Met Asn Val Gln Gly
       210                 215                 220
   Tyr Met Thr Tyr Ala Arg Glu Cys Pro Gly Met Pro Arg Lys Val Pro
   225                 230                 235                 240
   Ile Leu Tyr Leu Gly Tyr Val Gln Ser Leu Phe Trp Leu Phe Met Asn
                   245                 250                 255
   Phe Tyr Ile Arg Ala Tyr Val Leu Gly Gly Gly Ala Arg Lys Pro Val
               260                 265                 270
   Ala Ala Gly Lys Lys Asn Lys Ser Val
           275                 280
<210> 5
   <211> 828
   <212> DNA
   <213> Rhizopus oryzae
<220>
   <221> CDS
   <222> (1)..(828)
<400> 5
   atg gac att gat caa ttg aag cca atc act aac att cca tac cca gaa      48
   Met Asp Ile Asp Gln Leu Lys Pro Ile Thr Asn Ile Pro Tyr Pro Glu
   1               5                   10                  15
   tat tat gaa ttc ttt atg gat tgg aaa acg ccc gta gtg att gcc act      96
   Tyr Tyr Glu Phe Phe Met Asp Trp Lys Thr Pro Val Val Ile Ala Thr
               20                  25                  30
   ctt tac gct gtc aca att cat ctt tta aat cct agt cgt gac aca gcc      144
   Leu Tyr Ala Val Thr Ile His Leu Leu Asn Pro Ser Arg Asp Thr Ala
           35                  40                  45
   aag tta tct cga gtg gag gct aaa aac cgt ggt gtg aat aat gcg tca      192
   Lys Leu Ser Arg Val Glu Ala Lys Asn Arg Gly Val Asn Asn Ala Ser
       50                  55                  60
   agt agc agt aag ctg ttc acg acc ttt gtc ttt ctt cac aac ctg ttc      240
   Ser Ser Ser Lys Leu Phe Thr Thr Phe Val Phe Leu His Asn Leu Phe
   65                  70                  75                  80
   ttg tcc atc tat tct ggt gtc aca ttc gta aat atg gtt caa gcc tta      288
   Leu Ser Ile Tyr Ser Gly Val Thr Phe Val Asn Met Val Gln Ala Leu
                   85                  90                  95
   cac agg tta ttc aac aat tac tct gta cat gat gct tat tgt gat gtg      336
   His Arg Leu Phe Asn Asn Tyr Ser Val His Asp Ala Tyr Cys Asp Val
               100                 105                 110
   gat gga aca ttt tgg gac gaa gca ttg ggt tac tgg gga tat tta ttt      384
   Asp Gly Thr Phe Trp Asp Glu Ala Leu Gly Tyr Trp Gly Tyr Leu Phe
           115                 120                 125
   tat ctt tct aaa ttc tat gaa gtg gtt gac act gcc atc att ctc atc      432
   Tyr Leu Ser Lys Phe Tyr Glu Val Val Asp Thr Ala Ile Ile Leu Ile
       130                 135                 140
   aag ggt cgt cgc tca tct ctt ttg caa acc tat cat cac tcg ggt gcc      480
   Lys Gly Arg Arg Ser Ser Leu Leu Gln Thr Tyr His His Ser Gly Ala
   145                 150                 155                 160
   atg att act atg tgg tcc ggt att cgt tac aaa gct caa ccc att tgg      528
   Met Ile Thr Met Trp Ser Gly Ile Arg Tyr Lys Ala Gln Pro Ile Trp
                   165                 170                 175
   atc ttt gtc gtc ttt aac tca ctc att cat tct atc atg tac atg tac      576
   Ile Phe Val Val Phe Asn Ser Leu Ile His Ser Ile Met Tyr Met Tyr
               180                 185                 190
   tat gct ttt act tcc atc ggt ctt cat ccc ccc ggt aag cgt tat ttg      624
   Tyr Ala Phe Thr Ser Ile Gly Leu His Pro Pro Gly Lys Arg Tyr Leu
           195                 200                 205
   acc tcc atg caa atc tca caa ttc ttg gtg ggt atg tcg act gct atc      672
   Thr Ser Met Gln Ile Ser Gln Phe Leu Val Gly Met Ser Thr Ala Ile
       210                 215                 220
   agc tat ttg ttt gtg cct gat tgt tta caa acc cct ggt caa cgt ttt      720
   Ser Tyr Leu Phe Val Pro Asp Cys Leu Gln Thr Pro Gly Gln Arg Phe
   225                 230                 235                 240
   gct gtt ggt ttg aac att gct tat ctt tta cct ttg acc tat ctt ttt      768
   Ala Val Gly Leu Asn Ile Ala Tyr Leu Leu Pro Leu Thr Tyr Leu Phe
   245 250 255
   gtt gac ttt gca cgc aaa act tat gga aag aga aag gct gct gca aag 816
   Val Asp Phe Ala Arg Lys Thr Tyr Gly Lys Arg Lys Ala Ala Ala Lys
   260 265 270
   aag gtg gat tag 828
   Lys Val Asp
   275
<210> 6
   <211> 275
   <212> PRT
   <213> Rhizopus oryzae
<400> 6
   Met Asp Ile Asp Gln Leu Lys Pro Ile Thr Asn Ile Pro Tyr Pro Glu
   1               5                   10                  15
   Tyr Tyr Glu Phe Phe Met Asp Trp Lys Thr Pro Val Val Ile Ala Thr
               20                  25                  30
   Leu Tyr Ala Val Thr Ile His Leu Leu Asn Pro Ser Arg Asp Thr Ala
           35                  40                  45
   Lys Leu Ser Arg Val Glu Ala Lys Asn Arg Gly Val Asn Asn Ala Ser
       50                  55                  60
   Ser Ser Ser Lys Leu Phe Thr Thr Phe Val Phe Leu His Asn Leu Phe
   65                  70                  75                  80
   Leu Ser Ile Tyr Ser Gly Val Thr Phe Val Asn Met Val Gln Ala Leu
                   85                  90                  95
   His Arg Leu Phe Asn Asn Tyr Ser Val His Asp Ala Tyr Cys Asp Val
               100                 105                 110
   Asp Gly Thr Phe Trp Asp Glu Ala Leu Gly Tyr Trp Gly Tyr Leu Phe
           115                 120                 125
   Tyr Leu Ser Lys Phe Tyr Glu Val Val Asp Thr Ala Ile Ile Leu Ile
       130                 135                 140
   Lys Gly Arg Arg Ser Ser Leu Leu Gln Thr Tyr His His Ser Gly Ala
   145                 150                 155                 160
   Met Ile Thr Met Trp Ser Gly Ile Arg Tyr Lys Ala Gln Pro Ile Trp
                   165                 170                 175
   Ile Phe Val Val Phe Asn Ser Leu Ile His Ser Ile Met Tyr Met Tyr
               180                 185                 190
   Tyr Ala Phe Thr Ser Ile Gly Leu His Pro Pro Gly Lys Arg Tyr Leu
           195                 200                 205
   Thr Ser Met Gln Ile Ser Gln Phe Leu Val Gly Met Ser Thr Ala Ile
       210                 215                 220
   Ser Tyr Leu Phe Val Pro Asp Cys Leu Gln Thr Pro Gly Gln Arg Phe
   225                 230                 235                 240
   Ala Val Gly Leu Asn Ile Ala Tyr Leu Leu Pro Leu Thr Tyr Leu Phe
                   245                 250                 255
   Val Asp Phe Ala Arg Lys Thr Tyr Gly Lys Arg Lys Ala Ala Ala Lys
               260                 265                 270
   Lys Val Asp
           275
<210> 7
   <211> 918
   <212> DNA
   <213> Euglena gracilis
<220>
   <221> CDS
   <222> (1)..(918)
<400> 7
   atg gcg gat agc cca gtc atc aac ctc agc acc atg tgg aaa ccc ctt      48
   Met Ala Asp Ser Pro Val Ile Asn Leu Ser Thr Met Trp Lys Pro Leu
   1               5                   10                  15
   tca ctg atg gct ttg gac ctt gcc att ttg gga cat gtc tgg aag cag      96
   Ser Leu Met Ala Leu Asp Leu Ala Ile Leu Gly His Val Trp Lys Gln
               20                  25                  30
   gca caa cag gag ggc agc att tcg gcc tat gct gat tct gtt tgg act     144
   Ala Gln Gln Glu Gly Ser Ile Ser Ala Tyr Ala Asp Ser Val Trp Thr
           35                  40                  45
   cct ctc att atg tcc gtt tta tac tta tca atg atc ttc gtg ggg tgc     192
   Pro Leu Ile Met Ser Val Leu Tyr Leu Ser Met Ile Phe Val Gly Cys
       50                  55                  60
   cgc tgg atg aag aac cgt gaa ccc ttt gag atc aaa aca tac atg ttt     240
   Arg Trp Met Lys Asn Arg Glu Pro Phe Glu Ile Lys Thr Tyr Met Phe
   65                  70                  75                  80
   gct tat aac ctg tat cag acc ttg atg aac ctt tgc atc gtg ttg gga     288
   Ala Tyr Asn Leu Tyr Gln Thr Leu Met Asn Leu Cys Ile Val Leu Gly
                   85                  90                  95
   ttc ttg tac cag gtg cat gcc act ggg atg cgc ttt tgg gga agt ggt     336
   Phe Leu Tyr Gln Val His Ala Thr Gly Met Arg Phe Trp Gly Ser Gly
               100                 105                 110
   gtc gac cga agc ccg aaa ggt ttg ggc att ggc ttc ttc att tat gcc     384
   Val Asp Arg Ser Pro Lys Gly Leu Gly Ile Gly Phe Phe Ile Tyr Ala
           115                 120                 125
   cac tac cac aac aag tat gtg gaa tat ttt gat aca ctt ttt atg gtg     432
   His Tyr His Asn Lys Tyr Val Glu Tyr Phe Asp Thr Leu Phe Met Val
       130                 135                 140
   ctg cga aag aag aac aac cag att tct ttc ctt cac gtg tat cat cat      480
   Leu Arg Lys Lys Asn Asn Gln Ile Ser Phe Leu His Val Tyr His His
   145                 150                 155                 160
   gcc ctg ttg aca tgg gct tgg ttt gct gtt gtg tat ttc gca cct gga      528
   Ala Leu Leu Thr Trp Ala Trp Phe Ala Val Val Tyr Phe Ala Pro Gly
                   165                 170                 175
   ggt gat ggc tgg ttt gga gct tgc tac aat tct tcc atc cat gtc ctg      576
   Gly Asp Gly Trp Phe Gly Ala Cys Tyr Asn Ser Ser Ile His Val Leu
               180                 185                 190
   atg tac tct tac tac ttg ctt gca act ttt ggc atc agt tgc cca tgg      624
   Met Tyr Ser Tyr Tyr Leu Leu Ala Thr Phe Gly Ile Ser Cys Pro Trp
           195                 200                 205
   aag aag atc ttg aca cag ctc cag atg gtt caa ttc tgt ttc tgt ttt      672
   Lys Lys Ile Leu Thr Gln Leu Gln Met Val Gln Phe Cys Phe Cys Phe
       210                 215                 220
   aca cat tcc att tat gtg tgg att tgc ggg tca gag atc tac cca cgg      720
   Thr His Ser Ile Tyr Val Trp Ile Cys Gly Ser Glu Ile Tyr Pro Arg
   225                 230                 235                 240
   cct ctg act gct ttg cag tcg ttc gtg atg gtc aat atg ttg gtg ctg      768
   Pro Leu Thr Ala Leu Gln Ser Phe Val Met Val Asn Met Leu Val Leu
                   245                 250                 255
   ttt ggc aat ttc tat gtc aag caa tac tcc caa aag aac ggc aag ccg      816
   Phe Gly Asn Phe Tyr Val Lys Gln Tyr Ser Gln Lys Asn Gly Lys Pro
               260                 265                 270
   gag aac gga gcc acc cct gag aac gga gcg aag ccg caa cct tgc gag      864
   Glu Asn Gly Ala Thr Pro Glu Asn Gly Ala Lys Pro Gln Pro Cys Glu
           275                 280                 285
   aac ggc acg gtg gaa aag cga gag gcg ccc cga tct gtc ggc atg gga      912
   Asn Gly Thr Val Glu Lys Arg Glu Ala Pro Arg Ser Val Gly Met Gly
       290                 295                 300
   cgc tga                                                              918
   Arg
   305
<210> 8
   <211> 305
   <212> PRT
   <213> Euglena gracilis
<400> 8
   Met Ala Asp Ser Pro Val Ile Asn Leu Ser Thr Met Trp Lys Pro Leu
   1               5                   10                  15
   Ser Leu Met Ala Leu Asp Leu Ala Ile Leu Gly His Val Trp Lys Gln
               20                  25                  30
   Ala Gln Gln Glu Gly Ser Ile Ser Ala Tyr Ala Asp Ser Val Trp Thr
           35                  40                  45
   Pro Leu Ile Met Ser Val Leu Tyr Leu Ser Met Ile Phe Val Gly Cys
       50                  55                  60
   Arg Trp Met Lys Asn Arg Glu Pro Phe Glu Ile Lys Thr Tyr Met Phe
   65                  70                  75                  80
   Ala Tyr Asn Leu Tyr Gln Thr Leu Met Asn Leu Cys Ile Val Leu Gly
                   85                  90                  95
   Phe Leu Tyr Gln Val His Ala Thr Gly Met Arg Phe Trp Gly Ser Gly
               100                 105                 110
   Val Asp Arg Ser Pro Lys Gly Leu Gly Ile Gly Phe Phe Ile Tyr Ala
           115                 120                 125
   His Tyr His Asn Lys Tyr Val Glu Tyr Phe Asp Thr Leu Phe Met Val
       130                 135                 140
   Leu Arg Lys Lys Asn Asn Gln Ile Ser Phe Leu His Val Tyr His His
   145                 150                 155                 160
   Ala Leu Leu Thr Trp Ala Trp Phe Ala Val Val Tyr Phe Ala Pro Gly
                   165                 170                 175
   Gly Asp Gly Trp Phe Gly Ala Cys Tyr Asn Ser Ser Ile His Val Leu
               180                 185                 190
   Met Tyr Ser Tyr Tyr Leu Leu Ala Thr Phe Gly Ile Ser Cys Pro Trp
           195                 200                 205
   Lys Lys Ile Leu Thr Gln Leu Gln Met Val Gln Phe Cys Phe Cys Phe
       210                 215                 220
   Thr His Ser Ile Tyr Val Trp Ile Cys Gly Ser Glu Ile Tyr Pro Arg
   225                 230                 235                 240
   Pro Leu Thr Ala Leu Gln Ser Phe Val Met Val Asn Met Leu Val Leu
                   245                 250                 255
   Phe Gly Asn Phe Tyr Val Lys Gln Tyr Ser Gln Lys Asn Gly Lys Pro
               260                 265                 270
   Glu Asn Gly Ala Thr Pro Glu Asn Gly Ala Lys Pro Gln Pro Cys Glu
           275                 280                 285
   Asn Gly Thr Val Glu Lys Arg Glu Ala Pro Arg Ser Val Gly Met Gly
       290                 295                 300
   Arg
   305
<210> 9
   <211> 1380
   <212> DNA
   <213> Pythium irregulare
<220>
   <221> CDS
   <222> (1)..(1380)
<400> 9
   atg gtg gac ctc aag cct gga gtg aag cgc ctg gtg agc tgg aag gag      48
   Met Val Asp Leu Lys Pro Gly Val Lys Arg Leu Val Ser Trp Lys Glu
   1               5                   10                  15
   atc cgc gag cac gcg acg ccc gcg acc gcg tgg atc gtg att cac cac      96
   Ile Arg Glu His Ala Thr Pro Ala Thr Ala Trp Ile Val Ile His His
               20                  25                  30
   aag gtc tac gac atc tcc aag tgg gac tcg cac ccg ggt ggc tcc gtg     144
   Lys Val Tyr Asp Ile Ser Lys Trp Asp Ser His Pro Gly Gly Ser Val
           35                  40                  45
   atg ctc acg cag gcc ggc gag gac gcc acg gac gcc ttc gcg gtc ttc     192
   Met Leu Thr Gln Ala Gly Glu Asp Ala Thr Asp Ala Phe Ala Val Phe
       50                  55                  60
   cac ccg tcc tcg gcg ctc aag ctg ctc gag cag ttc tac gtc ggc gac     240
   His Pro Ser Ser Ala Leu Lys Leu Leu Glu Gln Phe Tyr Val Gly Asp
   65                  70                  75                  80
   gtg gac gaa acc tcc aag gcc gag atc gag ggg gag ccg gcg agc gac     288
   Val Asp Glu Thr Ser Lys Ala Glu Ile Glu Gly Glu Pro Ala Ser Asp
                   85                  90                  95
   gag gag cgc gcg cgc cgc gag cgc atc aac gag ttc atc gcg tcc tac     336
   Glu Glu Arg Ala Arg Arg Glu Arg Ile Asn Glu Phe Ile Ala Ser Tyr
               100                 105                 110
   cgt cgt ctg cgc gtc aag gtc aag ggc atg ggg ctc tac gac gcc agc     384
   Arg Arg Leu Arg Val Lys Val Lys Gly Met Gly Leu Tyr Asp Ala Ser
           115                 120                 125
   gcg ctc tac tac gcg tgg aag ctc gtg agc acg ttc ggc atc gcg gtg     432
   Ala Leu Tyr Tyr Ala Trp Lys Leu Val Ser Thr Phe Gly Ile Ala Val
       130                 135                 140
   ctc tcg atg gcg atc tgc ttc ttc ttc aac agt ttc gcc atg tac atg     480
   Leu Ser Met Ala Ile Cys Phe Phe Phe Asn Ser Phe Ala Met Tyr Met
   145                 150                 155                 160
   gtc gcc ggc gtg att atg ggg ctc ttc tac cag cag tcc gga tgg ctg     528
   Val Ala Gly Val Ile Met Gly Leu Phe Tyr Gln Gln Ser Gly Trp Leu
                   165                 170                 175
   gcg cac gac ttc ttg cac aac cag gtg tgc gag aac cgc acg ctc ggc     576
   Ala His Asp Phe Leu His Asn Gln Val Cys Glu Asn Arg Thr Leu Gly
               180                 185                 190
   aac ctt atc ggc tgc ctc gtg ggc aac gcc tgg cag ggc ttc agc gtg     624
   Asn Leu Ile Gly Cys Leu Val Gly Asn Ala Trp Gln Gly Phe Ser Val
           195                 200                 205
   cag tgg tgg aag aac aag cac aac ctg cac cac gcg gtg ccg aac ctg     672
   Gln Trp Trp Lys Asn Lys His Asn Leu His His Ala Val Pro Asn Leu
       210                 215                 220
   cac agc gcc aag gac gag ggc ttc atc ggc gac ccg gac atc gac acc     720
   His Ser Ala Lys Asp Glu Gly Phe Ile Gly Asp Pro Asp Ile Asp Thr
   225                 230                 235                 240
   atg ccg ctg ctg gcg tgg tct aag gag atg gcg cgc aag gcg ttc gag     768
   Met Pro Leu Leu Ala Trp Ser Lys Glu Met Ala Arg Lys Ala Phe Glu
                   245                 250                 255
   tcg gcg cac ggc ccg ttc ttc atc cgc aac cag gcg ttc cta tac ttc      816
   Ser Ala His Gly Pro Phe Phe Ile Arg Asn Gln Ala Phe Leu Tyr Phe
               260                 265                 270
   ccg ctg ctg ctg ctc gcg cgc ctg agc tgg ctc gcg cag tcg ttc ttc      864
   Pro Leu Leu Leu Leu Ala Arg Leu Ser Trp Leu Ala Gln Ser Phe Phe
           275                     280                 285
   tac gtg ttc acc gag ttc tcg ttc ggc atc ttc gac aag gtc gag ttc      912
   Tyr Val Phe Thr Glu Phe Ser Phe Gly Ile Phe Asp Lys Val Glu Phe
       290                     295                 300
   gac gga ccg gag aag gcg ggt ctg atc gtg cac tac atc tgg cag ctc      960
   Asp Gly Pro Glu Lys Ala Gly Leu Ile Val His Tyr Ile Trp Gln Leu
   305                     310                 315                 320
   gcg atc ccg tac ttc tgc aac atg agc ctg ttt gag ggc gtg gca tac     1008
   Ala Ile Pro Tyr Phe Cys Asn Met Ser Leu Phe Glu Gly Val Ala Tyr
                   325                 330                 335
   ttc ctc atg ggc cag gcg tcc tgc ggc ttg ctc ctg gcg ctg gtg ttc     1056
   Phe Leu Met Gly Gln Ala Ser Cys Gly Leu Leu Leu Ala Leu Val Phe
               340                 345                 350
   agt att ggc cac aac ggc atg tcg gtg tac gag cgc gaa acc aag ccg     1104
   Ser Ile Gly His Asn Gly Met Ser Val Tyr Glu Arg Glu Thr Lys Pro
           355                 360                 365
   gac ttc tgg cag ctg cag gtg acc acg acg cgc aac atc cgc gcg tcg     1152
   Asp Phe Trp Gln Leu Gln Val Thr Thr Thr Arg Asn Ile Arg Ala Ser
       370                 375                 380
   gta ttc atg gac tgg ttc acc ggt ggc ttg aac tac cag atc gac cat     1200
   Val Phe Met Asp Trp Phe Thr Gly Gly Leu Asn Tyr Gln Ile Asp His
   385                 390                 395                 400
   cac ctg ttc ccg ctc gtg ccg cgc cac aac ttg cca aag gtc aac gtg     1248
   His Leu Phe Pro Leu Val Pro Arg His Asn Leu Pro Lys Val Asn Val
                   405                 410                 415
   ctc atc aag tcg cta tgc aag gag ttc gac atc ccg ttc cac gag acc     1296
   Leu Ile Lys Ser Leu Cys Lys Glu Phe Asp Ile Pro Phe His Glu Thr
               420                 425                 430
   ggc ttc tgg gag ggc atc tac gag gtc gtg gac cac ctg gcg gac atc     1344
   Gly Phe Trp Glu Gly Ile Tyr Glu Val Val Asp His Leu Ala Asp Ile
           435                 440                 445
   agc aag gaa ttc atc acc gag ttc cca gcg atg taa                     1380
   Ser Lys Glu Phe Ile Thr Glu Phe Pro Ala Met
       450                 455
<210> 10
   <211> 459
   <212> PRT
   <213> Pythium irregulare
<400> 10
   Met Val Asp Leu Lys Pro Gly Val Lys Arg Leu Val Ser Trp Lys Glu
   1               5                   10                  15
   Ile Arg Glu His Ala Thr Pro Ala Thr Ala Trp Ile Val Ile His His
               20                  25                  30
   Lys Val Tyr Asp Ile Ser Lys Trp Asp Ser His Pro Gly Gly Ser Val
   35 40 45
   Met Leu Thr Gln Ala Gly Glu Asp Ala Thr Asp Ala Phe Ala Val Phe
   50 55 60
   His Pro Ser Ser Ala Leu Lys Leu Leu Glu Gln Phe Tyr Val Gly Asp
   65 70 75 80
   Val Asp Glu Thr Ser Lys Ala Glu Ile Glu Gly Glu Pro Ala Ser Asp
   85 90 95
   Glu Glu Arg Ala Arg Arg Glu Arg Ile Asn Glu Phe Ile Ala Ser Tyr
   100 105 110
   Arg Arg Leu Arg Val Lys Val Lys Gly Met Gly Leu Tyr Asp Ala Ser
   115 120 125
   Ala Leu Tyr Tyr Ala Trp Lys Leu Val Ser Thr Phe Gly Ile Ala Val
   130 135 140
   Leu Ser Met Ala Ile Cys Phe Phe Phe Asn Ser Phe Ala Met Tyr Met
   145 150 155 160
   Val Ala Gly Val Ile Met Gly Leu Phe Tyr Gln Gln Ser Gly Trp Leu
   165 170 175
   Ala His Asp Phe Leu His Asn Gln Val Cys Glu Asn Arg Thr Leu Gly
   180 185 190
   Asn Leu Ile Gly Cys Leu Val Gly Asn Ala Trp Gln Gly Phe Ser Val
   195 200 205
   Gln Trp Trp Lys Asn Lys His Asn Leu His His Ala Val Pro Asn Leu
   210 215 220
   His Ser Ala Lys Asp Glu Gly Phe Ile Gly Asp Pro Asp Ile Asp Thr
   225 230 235 240
   Met Pro Leu Leu Ala Trp Ser Lys Glu Met Ala Arg Lys Ala Phe Glu
   245 250 255
   Ser Ala His Gly Pro Phe Phe Ile Arg Asn Gln Ala Phe Leu Tyr Phe
   260 265 270
   Pro Leu Leu Leu Leu Ala Arg Leu Ser Trp Leu Ala Gln Ser Phe Phe
   275 280 285
   Tyr Val Phe Thr Glu Phe Ser Phe Gly Ile Phe Asp Lys Val Glu Phe
   290 295 300
   Asp Gly Pro Glu Lys Ala Gly Leu Ile Val His Tyr Ile Trp Gln Leu
   305 310 315 320
   Ala Ile Pro Tyr Phe Cys Asn Met Ser Leu Phe Glu Gly Val Ala Tyr
   325 330 335
   Phe Leu Met Gly Gln Ala Ser Cys Gly Leu Leu Leu Ala Leu Val Phe
   340 345 350
   Ser Ile Gly His Asn Gly Met Ser Val Tyr Glu Arg Glu Thr Lys Pro
   355 360 365
   Asp Phe Trp Gln Leu Gln Val Thr Thr Thr Arg Asn Ile Arg Ala Ser
   370 375 380
   Val Phe Met Asp Trp Phe Thr Gly Gly Leu Asn Tyr Gln Ile Asp His
   385 390 395 400
   His Leu Phe Pro Leu Val Pro Arg His Asn Leu Pro Lys Val Asn Val
   405 410 415
   Leu Ile Lys Ser Leu Cys Lys Glu Phe Asp Ile Pro Phe His Glu Thr
   420 425 430
   Gly Phe Trp Glu Gly Ile Tyr Glu Val Val Asp His Leu Ala Asp Ile
   435 440 445
   Ser Lys Glu Phe Ile Thr Glu Phe Pro Ala Met
   450 455
<210> 11
   <211> 1320
   <212> DNA
   <213> Thraustochytrium ssp.
<220>
   <221> CDS
   <222> (1)..(1320)
<400> 11
   atg ggc aag ggc agc gag ggc cgc agc gcg gcg cgc gag atg acg gcc      48
   Met Gly Lys Gly Ser Glu Gly Arg Ser Ala Ala Arg Glu Met Thr Ala
   1               5                   10                  15
   gag gcg aac ggc gac aag cgg aaa acg att ctg atc gag ggc gtc ctg      96
   Glu Ala Asn Gly Asp Lys Arg Lys Thr Ile Leu Ile Glu Gly Val Leu
               20                  25                  30
   tac gac gcg acg aac ttt aag cac ccg ggc ggt tcg atc atc aac ttc     144
   Tyr Asp Ala Thr Asn Phe Lys His Pro Gly Gly Ser Ile Ile Asn Phe
           35                  40                  45
   ttg acc gag ggc gag gcc ggc gtg gac gcg acg cag gcg tac cgc gag     192
   Leu Thr Glu Gly Glu Ala Gly Val Asp Ala Thr Gln Ala Tyr Arg Glu
       50                  55                  60
   ttt cat cag cgg tcc ggc aag gcc gac aag tac ctc aag tcg ctg ccg     240
   Phe His Gln Arg Ser Gly Lys Ala Asp Lys Tyr Leu Lys Ser Leu Pro
   65                  70                  75                  80
   aag ctg gat gcg tcc aag gtg gag tcg cgg ttc tcg gcc aaa gag cag      288
   Lys Leu Asp Ala Ser Lys Val Glu Ser Arg Phe Ser Ala Lys Glu Gln
                   85                  90                  95
   gcg cgg cgc gac gcc atg acg cgc gac tac gcg gcc ttt cgc gag gag      336
   Ala Arg Arg Asp Ala Met Thr Arg Asp Tyr Ala Ala Phe Arg Glu Glu
               100                 105                 110
   ctc gtc gcc gag ggg tac ttt gac ccg tcg atc ccg cac atg att tac      384
   Leu Val Ala Glu Gly Tyr Phe Asp Pro Ser Ile Pro His Met Ile Tyr
           115                 120                 125
   cgc gtc gtg gag atc gtg gcg ctc ttc gcg ctc tcg ttc tgg ctc atg      432
   Arg Val Val Glu Ile Val Ala Leu Phe Ala Leu Ser Phe Trp Leu Met
       130                 135                 140
   tcc aag gcc tcg ccc acc tcg ctc gtg ctg ggc gtg gtg atg aac ggc      480
   Ser Lys Ala Ser Pro Thr Ser Leu Val Leu Gly Val Val Met Asn Gly
   145                 150                 155                 160
   att gcg cag ggc cgc tgc ggc tgg gtc atg cac gag atg ggc cac ggg      528
   Ile Ala Gln Gly Arg Cys Gly Trp Val Met His Glu Met Gly His Gly
                   165                 170                 175
   tcg ttc acg ggc gtc atc tgg ctc gac gac cgg atg tgc gag ttc ttc      576
   Ser Phe Thr Gly Val Ile Trp Leu Asp Asp Arg Met Cys Glu Phe Phe
               180                 185                 190
   tac ggc gtc ggc tgc ggc atg agc ggg cac tac tgg aag aac cag cac      624
   Tyr Gly Val Gly Cys Gly Met Ser Gly His Tyr Trp Lys Asn Gln His
           195                 200                 205
   agc aag cac cac gcc gcg ccc aac cgc ctc gag cac gat gtc gat ctc      672
   Ser Lys His His Ala Ala Pro Asn Arg Leu Glu His Asp Val Asp Leu
       210                 215                 220
   aac acg ctg ccc ctg gtc gcc ttt aac gag cgc gtc gtg cgc aag gtc      720
   Asn Thr Leu Pro Leu Val Ala Phe Asn Glu Arg Val Val Arg Lys Val
   225                 230                 235                 240
   aag ccg gga tcg ctg ctg gcg ctc tgg ctg cgc gtg cag gcg tac ctc      768
   Lys Pro Gly Ser Leu Leu Ala Leu Trp Leu Arg Val Gln Ala Tyr Leu
                   245                 250                 255
   ttt gcg ccc gtc tcg tgc ctg ctc atc ggc ctt ggc tgg acg ctc tac      816
   Phe Ala Pro Val Ser Cys Leu Leu Ile Gly Leu Gly Trp Thr Leu Tyr
               260                 265                 270
   ctg cac ccg cgc tac atg ctg cgc acc aag cgg cac atg gag ttc gtc      864
   Leu His Pro Arg Tyr Met Leu Arg Thr Lys Arg His Met Glu Phe Val
           275                 280                 285
   tgg atc ttc gcg cgc tac att ggc tgg ttc tcg ctc atg ggc gct ctc      912
   Trp Ile Phe Ala Arg Tyr Ile Gly Trp Phe Ser Leu Met Gly Ala Leu
       290                 295                 300
   ggc tac tcg ccg ggc acc tcg gtc ggg atg tac ctg tgc tcg ttc ggc      960
   Gly Tyr Ser Pro Gly Thr Ser Val Gly Met Tyr Leu Cys Ser Phe Gly
   305                 310                 315                 320
   ctc ggc tgc att tac att ttc ctg cag ttc gcc gtc agc cac acg cac     1008
   Leu Gly Cys Ile Tyr Ile Phe Leu Gln Phe Ala Val Ser His Thr His
                   325                 330                 335
   ctg ccg gtg acc aac ccg gag gac cag ctg cac tgg ctc gag tac gcg     1056
   Leu Pro Val Thr Asn Pro Glu Asp Gln Leu His Trp Leu Glu Tyr Ala
               340                 345                 350
   gcc gac cac acg gtg aac att agc acc aag tcc tgg ctc gtc acg tgg     1104
   Ala Asp His Thr Val Asn Ile Ser Thr Lys Ser Trp Leu Val Thr Trp
           355                 360                 365
   tgg atg tcg aac ctg aac ttt cag atc gag cac cac ctc ttc ccc acg      1152
   Trp Met Ser Asn Leu Asn Phe Gln Ile Glu His His Leu Phe Pro Thr
       370                 375                 380
   gcg ccg cag ttc cgc ttc aag gaa atc agt cct cgc gtc gag gcc ctc      1200
   Ala Pro Gln Phe Arg Phe Lys Glu Ile Ser Pro Arg Val Glu Ala Leu
   385                 390                 395                 400
   ttc aag cgc cac aac ctc ccg tac tac gac ctg ccc tac acg agc gcg      1248
   Phe Lys Arg His Asn Leu Pro Tyr Tyr Asp Leu Pro Tyr Thr Ser Ala
                   405                 410                 415
   gtc tcg acc acc ttt gcc aat ctt tat tcc gtc ggc cac tcg gtc ggc      1296
   Val Ser Thr Thr Phe Ala Asn Leu Tyr Ser Val Gly His Ser Val Gly
               420                 425                 430
   gcc gac acc aag aag cag gac tga                                      1320
   Ala Asp Thr Lys Lys Gln Asp
           435
<210> 12
   <211> 439
   <212> PRT
   <213> Thraustochytrium ssp.
<400> 12
   Met Gly Lys Gly Ser Glu Gly Arg Ser Ala Ala Arg Glu Met Thr Ala
   1               5                   10                  15
   Glu Ala Asn Gly Asp Lys Arg Lys Thr Ile Leu Ile Glu Gly Val Leu
               20                  25                  30
   Tyr Asp Ala Thr Asn Phe Lys His Pro Gly Gly Ser Ile Ile Asn Phe
           35                  40                  45
   Leu Thr Glu Gly Glu Ala Gly Val Asp Ala Thr Gln Ala Tyr Arg Glu
       50                  55                  60
   Phe His Gln Arg Ser Gly Lys Ala Asp Lys Tyr Leu Lys Ser Leu Pro
   65                  70                  75                  80
   Lys Leu Asp Ala Ser Lys Val Glu Ser Arg Phe Ser Ala Lys Glu Gln
                   85                  90                  95
   Ala Arg Arg Asp Ala Met Thr Arg Asp Tyr Ala Ala Phe Arg Glu Glu
               100                 105                 110
   Leu Val Ala Glu Gly Tyr Phe Asp Pro Ser Ile Pro His Met Ile Tyr
           115                 120                 125
   Arg Val Val Glu Ile Val Ala Leu Phe Ala Leu Ser Phe Trp Leu Met
       130                 135                 140
   Ser Lys Ala Ser Pro Thr Ser Leu Val Leu Gly Val Val Met Asn Gly
   145                 150                 155                 160
   Ile Ala Gln Gly Arg Cys Gly Trp Val Met His Glu Met Gly His Gly
   165 170 175
   Ser Phe Thr Gly Val Ile Trp Leu Asp Asp Arg Met Cys Glu Phe Phe
   180 185 190
   Tyr Gly Val Gly Cys Gly Met Ser Gly His Tyr Trp Lys Asn Gln His
   195 200 205
   Ser Lys His His Ala Ala Pro Asn Arg Leu Glu His Asp Val Asp Leu
   210 215 220
   Asn Thr Leu Pro Leu Val Ala Phe Asn Glu Arg Val Val Arg Lys Val
   225 230 235 240
   Lys Pro Gly Ser Leu Leu Ala Leu Trp Leu Arg Val Gln Ala Tyr Leu
   245 250 255
   Phe Ala Pro Val Ser Cys Leu Leu Ile Gly Leu Gly Trp Thr Leu Tyr
   260 265 270
   Leu His Pro Arg Tyr Met Leu Arg Thr Lys Arg His Met Glu Phe Val
   275 280 285
   Trp Ile Phe Ala Arg Tyr Ile Gly Trp Phe Ser Leu Met Gly Ala Leu
   290 295 300
   Gly Tyr Ser Pro Gly Thr Ser Val Gly Met Tyr Leu Cys Ser Phe Gly
   305 310 315 320
   Leu Gly Cys Ile Tyr Ile Phe Leu Gln Phe Ala Val Ser His Thr His
   325 330 335
   Leu Pro Val Thr Asn Pro Glu Asp Gln Leu His Trp Leu Glu Tyr Ala
   340 345 350
   Ala Asp His Thr Val Asn Ile Ser Thr Lys Ser Trp Leu Val Thr Trp
   355 360 365
   Trp Met Ser Asn Leu Asn Phe Gln Ile Glu His His Leu Phe Pro Thr
   370 375 380
   Ala Pro Gln Phe Arg Phe Lys Glu Ile Ser Pro Arg Val Glu Ala Leu
   385 390 395 400
   Phe Lys Arg His Asn Leu Pro Tyr Tyr Asp Leu Pro Tyr Thr Ser Ala
   405 410 415
   Val Ser Thr Thr Phe Ala Asn Leu Tyr Ser Val Gly His Ser Val Gly
   420 425 430
   Ala Asp Thr Lys Lys Gln Asp
   435
<210> 13
   <211> 1086
   <212> DNA
   <213> Phytophtora infestans
<220>
   <221> CDS
   <222> (1)..(1086)
<400> 13
   atg gcg acg aag gag gcg tat gtg ttc ccc act ctg acg gag atc aag      48
   Met Ala Thr Lys Glu Ala Tyr Val Phe Pro Thr Leu Thr Glu Ile Lys
   1               5                   10                  15
   cgg tcg cta cct aaa gac tgt ttc gag gct tcg gtg cct ctg tcg ctc      96
   Arg Ser Leu Pro Lys Asp Cys Phe Glu Ala Ser Val Pro Leu Ser Leu
               20                  25                  30
   tac tac acc gtg cgt tgt ctg gtg atc gcg gtg gct cta acc ttc ggt      144
   Tyr Tyr Thr Val Arg Cys Leu Val Ile Ala Val Ala Leu Thr Phe Gly
           35                  40                  45
   ctc aac tac gct cgc gct ctg ccc gag gtc gag agc ttc tgg gct ctg      192
   Leu Asn Tyr Ala Arg Ala Leu Pro Glu Val Glu Ser Phe Trp Ala Leu
       50                  55                  60
   gac gcc gca ctc tgc acg ggc tac atc ttg ctg cag ggc atc gtg ttc      240
   Asp Ala Ala Leu Cys Thr Gly Tyr Ile Leu Leu Gln Gly Ile Val Phe
   65                  70                  75                  80
   tgg ggc ttc ttc acg gtg ggc cac gat gcc ggc cac ggc gcc ttc tcg      288
   Trp Gly Phe Phe Thr Val Gly His Asp Ala Gly His Gly Ala Phe Ser
                   85                  90                  95
   cgc tac cac ctg ctt aac ttc gtg gtg ggc act ttc atg cac tcg ctc      336
   Arg Tyr His Leu Leu Asn Phe Val Val Gly Thr Phe Met His Ser Leu
               100                 105                 110
   atc ctc acg ccc ttc gag tcg tgg aag ctc acg cac cgt cac cac cac      384
   Ile Leu Thr Pro Phe Glu Ser Trp Lys Leu Thr His Arg His His His
           115                 120                 125
   aag aac acg ggc aac att gac cgt gac gag gtc ttc tac ccg caa cgc      432
   Lys Asn Thr Gly Asn Ile Asp Arg Asp Glu Val Phe Tyr Pro Gln Arg
       130                 135                 140
   aag gcc gac gac cac ccg ctg tct cgc aac ctg att ctg gcg ctc ggg      480
   Lys Ala Asp Asp His Pro Leu Ser Arg Asn Leu Ile Leu Ala Leu Gly
   145                 150                 155                 160
   gca gcg tgg ctc gcc tat ttg gtc gag ggc ttc cct cct cgt aag gtc      528
   Ala Ala Trp Leu Ala Tyr Leu Val Glu Gly Phe Pro Pro Arg Lys Val
                   165                 170                 175
   aac cac ttc aac ccg ttc gag cct ctg ttc gtg cgt cag gtg tca gct      576
   Asn His Phe Asn Pro Phe Glu Pro Leu Phe Val Arg Gln Val Ser Ala
               180                 185                 190
   gtg gta atc tct ctt ctc gcc cac ttc ttc gtg gcc gga ctc tcc atc      624
   Val Val Ile Ser Leu Leu Ala His Phe Phe Val Ala Gly Leu Ser Ile
           195                 200                 205
   tat ctg agc ctc cag ctg ggc ctt aag acg atg gca atc tac tac tat      672
   Tyr Leu Ser Leu Gln Leu Gly Leu Lys Thr Met Ala Ile Tyr Tyr Tyr
       210                 215                 220
   gga cct gtt ttt gtg ttc ggc agc atg ctg gtc att acc acc ttc cta      720
   Gly Pro Val Phe Val Phe Gly Ser Met Leu Val Ile Thr Thr Phe Leu
   225                 230                 235                 240
   cac cac aat gat gag gag acc cca tgg tac gcc gac tcg gag tgg acg      768
   His His Asn Asp Glu Glu Thr Pro Trp Tyr Ala Asp Ser Glu Trp Thr
                   245                 250                 255
   tac gtc aag ggc aac ctc tcg tcc gtg gac cga tcg tac ggc gcg ctc      816
   Tyr Val Lys Gly Asn Leu Ser Ser Val Asp Arg Ser Tyr Gly Ala Leu
               260                 265                 270
   att gac aac ctg agc cac aac atc ggc acg cac cag atc cac cac ctt      864
   Ile Asp Asn Leu Ser His Asn Ile Gly Thr His Gln Ile His His Leu
           275                 280                 285
   ttc cct atc att ccg cac tac aaa ctc aag aaa gcc act gcg gcc ttc      912
   Phe Pro Ile Ile Pro His Tyr Lys Leu Lys Lys Ala Thr Ala Ala Phe
       290                 295                 300
   cac cag gct ttc cct gag ctc gtg cgc aag agc gac gag cca att atc      960
   His Gln Ala Phe Pro Glu Leu Val Arg Lys Ser Asp Glu Pro Ile Ile
   305                 310                 315                 320
   aag gct ttc ttc cgg gtt gga cgt ctc tac gca aac tac ggc gtt gtg     1008
   Lys Ala Phe Phe Arg Val Gly Arg Leu Tyr Ala Asn Tyr Gly Val Val
                   325                 330                 335
   gac cag gag gcg aag ctc ttc acg cta aag gaa gcc aag gcg gcg acc     1056
   Asp Gln Glu Ala Lys Leu Phe Thr Leu Lys Glu Ala Lys Ala Ala Thr
               340                 345                 350
   gag gcg gcg gcc aag acc aag tcc acg taa                             1086
   Glu Ala Ala Ala Lys Thr Lys Ser Thr
           355                 360
<210> 14
   <211> 361
   <212> PRT
   <213> Phytophtora infestans
<400> 14
   Met Ala Thr Lys Glu Ala Tyr Val Phe Pro Thr Leu Thr Glu Ile Lys
   1               5                   10                  15
   Arg Ser Leu Pro Lys Asp Cys Phe Glu Ala Ser Val Pro Leu Ser Leu
               20                  25                  30
   Tyr Tyr Thr Val Arg Cys Leu Val Ile Ala Val Ala Leu Thr Phe Gly
           35                  40                  45
   Leu Asn Tyr Ala Arg Ala Leu Pro Glu Val Glu Ser Phe Trp Ala Leu
       50                  55                  60
   Asp Ala Ala Leu Cys Thr Gly Tyr Ile Leu Leu Gln Gly Ile Val Phe
   65                  70                  75                  80
   Trp Gly Phe Phe Thr Val Gly His Asp Ala Gly His Gly Ala Phe Ser
                   85                  90                  95
   Arg Tyr His Leu Leu Asn Phe Val Val Gly Thr Phe Met His Ser Leu
               100                 105                 110
   Ile Leu Thr Pro Phe Glu Ser Trp Lys Leu Thr His Arg His His His
           115                 120                 125
   Lys Asn Thr Gly Asn Ile Asp Arg Asp Glu Val Phe Tyr Pro Gln Arg
       130                 135                 140
   Lys Ala Asp Asp His Pro Leu Ser Arg Asn Leu Ile Leu Ala Leu Gly
   145                 150                 155                 160
   Ala Ala Trp Leu Ala Tyr Leu Val Glu Gly Phe Pro Pro Arg Lys Val
                   165                 170                 175
   Asn His Phe Asn Pro Phe Glu Pro Leu Phe Val Arg Gln Val Ser Ala
               180                 185                 190
   Val Val Ile Ser Leu Leu Ala His Phe Phe Val Ala Gly Leu Ser Ile
           195                 200                 205
   Tyr Leu Ser Leu Gln Leu Gly Leu Lys Thr Met Ala Ile Tyr Tyr Tyr
       210                 215                 220
   Gly Pro Val Phe Val Phe Gly Ser Met Leu Val Ile Thr Thr Phe Leu
   225                 230                 235                 240
   His His Asn Asp Glu Glu Thr Pro Trp Tyr Ala Asp Ser Glu Trp Thr
                   245                 250                 255
   Tyr Val Lys Gly Asn Leu Ser Ser Val Asp Arg Ser Tyr Gly Ala Leu
               260                 265                 270
   Ile Asp Asn Leu Ser His Asn Ile Gly Thr His Gln Ile His His Leu
           275                 280                 285
   Phe Pro Ile Ile Pro His Tyr Lys Leu Lys Lys Ala Thr Ala Ala Phe
       290                 295                 300
   His Gln Ala Phe Pro Glu Leu Val Arg Lys Ser Asp Glu Pro Ile Ile
   305                 310                 315                 320
   Lys Ala Phe Phe Arg Val Gly Arg Leu Tyr Ala Asn Tyr Gly Val Val
                   325                 330                 335
   Asp Gln Glu Ala Lys Leu Phe Thr Leu Lys Glu Ala Lys Ala Ala Thr
               340                 345                 350
   Glu Ala Ala Ala Lys Thr Lys Ser Thr
           355                 360
<210> 15
   <211> 1266
   <212> DNA
   <213> Euglena gracilis
<220>
   <221> CDS
   <222> (1)..(1266)
<400> 15
   atg aag tca aag cgc caa gcg ctt ccc ctt aca att gat gga aca aca 48
   Met Lys Ser Lys Arg Gln Ala Leu Pro Leu Thr Ile Asp Gly Thr Thr
   1 5 10 15
   tat gat gtg tct gcc tgg gtc aat ttc cac cct ggt ggt gcg gaa att 96
   Tyr Asp Val Ser Ala Trp Val Asn Phe His Pro Gly Gly Ala Glu Ile
   20 25 30
   ata gag aat tac caa gga agg gat gcc act gat gcc ttc atg gtt atg 144
   Ile Glu Asn Tyr Gln Gly Arg Asp Ala Thr Asp Ala Phe Met Val Met
   35 40 45
   cac tct caa gaa gcc ttc gac aag ctc aag cgc atg ccc aaa atc aat 192
   His Ser Gln Glu Ala Phe Asp Lys Leu Lys Arg Met Pro Lys Ile Asn
   50 55 60
   ccc agt tct gag ttg cca ccc cag gct gca gtg aat gaa gct caa gag 240
   Pro Ser Ser Glu Leu Pro Pro Gln Ala Ala Val Asn Glu Ala Gln Glu
   65 70 75 80
   gat ttc cgg aag ctc cga gaa gag ttg atc gca act ggc atg ttt gat 288
   Asp Phe Arg Lys Leu Arg Glu Glu Leu Ile Ala Thr Gly Met Phe Asp
   85 90 95
   gcc tcc ccc ctc tgg tac tca tac aaa atc agc acc aca ctg ggc ctt 336
   Ala Ser Pro Leu Trp Tyr Ser Tyr Lys Ile Ser Thr Thr Leu Gly Leu
   100 105 110
   gga gtg ctg ggt tat ttc ctg atg gtt cag tat cag atg tat ttc att 384
   Gly Val Leu Gly Tyr Phe Leu Met Val Gln Tyr Gln Met Tyr Phe Ile
   115 120 125
   ggg gca gtg ttg ctt ggg atg cac tat caa cag atg ggc tgg ctt tct 432
   Gly Ala Val Leu Leu Gly Met His Tyr Gln Gln Met Gly Trp Leu Ser
   130 135 140
   cat gac att tgc cac cac cag act ttc aag aac cgg aac tgg aac aac 480
   His Asp Ile Cys His His Gln Thr Phe Lys Asn Arg Asn Trp Asn Asn
   145 150 155 160
   ctc gtg gga ctg gta ttt ggc aat ggt ctg caa ggt ttt tcc gtg aca 528
   Leu Val Gly Leu Val Phe Gly Asn Gly Leu Gln Gly Phe Ser Val Thr
   165 170 175
   tgc tgg aag gac aga cac aat gca cat cat tcg gca acc aat gtt caa 576
   Cys Trp Lys Asp Arg His Asn Ala His His Ser Ala Thr Asn Val Gln
   180 185 190
   ggg cac gac cct gat att gac aac ctc ccc ctc tta gcc tgg tct gag 624
   Gly His Asp Pro Asp Ile Asp Asn Leu Pro Leu Leu Ala Trp Ser Glu
   195 200 205
   gat gac gtc aca cgg gcg tca ccg att tcc cgc aag ctc att cag ttc 672
   Asp Asp Val Thr Arg Ala Ser Pro Ile Ser Arg Lys Leu Ile Gln Phe
   210 215 220
   cag cag tat tat ttc ttg gtc atc tgt atc ttg ttg cgg ttc att tgg 720
   Gln Gln Tyr Tyr Phe Leu Val Ile Cys Ile Leu Leu Arg Phe Ile Trp
   225                 230                 235                 240
   tgt ttc cag agc gtg ttg acc gtg cgc agt ctg aag gac aga gat aac      768
   Cys Phe Gln Ser Val Leu Thr Val Arg Ser Leu Lys Asp Arg Asp Asn
                   245                 250                 255
   caa ttc tat cgc tct cag tat aag aag gag gcc att ggc ctc gcc ctg      816
   Gln Phe Tyr Arg Ser Gln Tyr Lys Lys Glu Ala Ile Gly Leu Ala Leu
               260                 265                 270
   cat tgg aca ttg aag gcc ctg ttc cac tta ttc ttt atg ccc agc atc      864
   His Trp Thr Leu Lys Ala Leu Phe His Leu Phe Phe Met Pro Ser Ile
           275                 280                 285
   ctc aca tcg ctg ttg gta ttt ttc gtt tcg gag ctg gtt ggc ggc ttc      912
   Leu Thr Ser Leu Leu Val Phe Phe Val Ser Glu Leu Val Gly Gly Phe
       290                 295                 300
   ggc att gcg atc gtg gtg ttc atg aac cac tac cca ctg gag aag atc      960
   Gly Ile Ala Ile Val Val Phe Met Asn His Tyr Pro Leu Glu Lys Ile
   305                 310                 315                 320
   ggg gac tcg gtc tgg gat ggc cat gga ttc tcg gtt ggc cag atc cat     1008
   Gly Asp Ser Val Trp Asp Gly His Gly Phe Ser Val Gly Gln Ile His
                   325                 330                 335
   gag acc atg aac att cgg cga ggg att atc aca gat tgg ttt ttc gga     1056
   Glu Thr Met Asn Ile Arg Arg Gly Ile Ile Thr Asp Trp Phe Phe Gly
               340                 345                 350
   ggc ttg aac tac cag atc gag cac cat ttg tgg ccg acc ctc cct cgc     1104
   Gly Leu Asn Tyr Gln Ile Glu His His Leu Trp Pro Thr Leu Pro Arg
           355                 360                 365
   cac aac ctg aca gcg gtt agc tac cag gtg gaa cag ctg tgc cag aag     1152
   His Asn Leu Thr Ala Val Ser Tyr Gln Val Glu Gln Leu Cys Gln Lys
       370                 375                 380
   cac aac ctg ccg tat cgg aac ccg ctg ccc cat gaa ggg ttg gtc atc     1200
   His Asn Leu Pro Tyr Arg Asn Pro Leu Pro His Glu Gly Leu Val Ile
   385                 390                 395                 400
   ctg ctg cgc tat ctg gcg gtg ttc gcc cgg atg gcg gag aag caa ccc     1248
   Leu Leu Arg Tyr Leu Ala Val Phe Ala Arg Met Ala Glu Lys Gln Pro
                   405                 410                 415
   gcg ggg aag gct cta taa                                              1266
   Ala Gly Lys Ala Leu
               420
<210> 16
   <211> 421
   <212> PRT
   <213> Euglena gracilis
<400> 16
   Met Lys Ser Lys Arg Gln Ala Leu Pro Leu Thr Ile Asp Gly Thr Thr
   1               5                   10                  15
   Tyr Asp Val Ser Ala Trp Val Asn Phe His Pro Gly Gly Ala Glu Ile
               20                  25                  30
   Ile Glu Asn Tyr Gln Gly Arg Asp Ala Thr Asp Ala Phe Met Val Met
           35                  40                  45
   His Ser Gln Glu Ala Phe Asp Lys Leu Lys Arg Met Pro Lys Ile Asn
       50                  55                  60
   Pro Ser Ser Glu Leu Pro Pro Gln Ala Ala Val Asn Glu Ala Gln Glu
   65                  70                  75                  80
   Asp Phe Arg Lys Leu Arg Glu Glu Leu Ile Ala Thr Gly Met Phe Asp
                   85                  90                  95
   Ala Ser Pro Leu Trp Tyr Ser Tyr Lys Ile Ser Thr Thr Leu Gly Leu
               100                 105                 110
   Gly Val Leu Gly Tyr Phe Leu Met Val Gln Tyr Gln Met Tyr Phe Ile
           115                 120                 125
   Gly Ala Val Leu Leu Gly Met His Tyr Gln Gln Met Gly Trp Leu Ser
       130                 135                 140
   His Asp Ile Cys His His Gln Thr Phe Lys Asn Arg Asn Trp Asn Asn
   145                 150                 155                 160
   Leu Val Gly Leu Val Phe Gly Asn Gly Leu Gln Gly Phe Ser Val Thr
                   165                 170                 175
   Cys Trp Lys Asp Arg His Asn Ala His His Ser Ala Thr Asn Val Gln
               180                 185                 190
   Gly His Asp Pro Asp Ile Asp Asn Leu Pro Leu Leu Ala Trp Ser Glu
           195                 200                 205
   Asp Asp Val Thr Arg Ala Ser Pro Ile Ser Arg Lys Leu Ile Gln Phe
       210                 215                 220
   Gln Gln Tyr Tyr Phe Leu Val Ile Cys Ile Leu Leu Arg Phe Ile Trp
   225                 230                 235                 240
   Cys Phe Gln Ser Val Leu Thr Val Arg Ser Leu Lys Asp Arg Asp Asn
                   245                 250                 255
   Gln Phe Tyr Arg Ser Gln Tyr Lys Lys Glu Ala Ile Gly Leu Ala Leu
               260                 265                 270
   His Trp Thr Leu Lys Ala Leu Phe His Leu Phe Phe Met Pro Ser Ile
           275                 280                 285
   Leu Thr Ser Leu Leu Val Phe Phe Val Ser Glu Leu Val Gly Gly Phe
       290                 295                 300
   Gly Ile Ala Ile Val Val Phe Met Asn His Tyr Pro Leu Glu Lys Ile
   305                 310                 315                 320
   Gly Asp Ser Val Trp Asp Gly His Gly Phe Ser Val Gly Gln Ile His
                   325                 330                 335
   Glu Thr Met Asn Ile Arg Arg Gly Ile Ile Thr Asp Trp Phe Phe Gly
               340                 345                 350
   Gly Leu Asn Tyr Gln Ile Glu His His Leu Trp Pro Thr Leu Pro Arg
           355                 360                 365
   His Asn Leu Thr Ala Val Ser Tyr Gln Val Glu Gln Leu Cys Gln Lys
       370                 375                 380
   His Asn Leu Pro Tyr Arg Asn Pro Leu Pro His Glu Gly Leu Val Ile
   385                 390                 395                 400
   Leu Leu Arg Tyr Leu Ala Val Phe Ala Arg Met Ala Glu Lys Gln Pro
                   405                 410                 415
   Ala Gly Lys Ala Leu
               420
<210> 17
   <211> 1197
   <212> DNA
   <213> Phytophtora sojae
<220>
   <221> CDS
   <222> (1)..(1197)
<400> 17
   atg gcg atc ctg aac ccg gag gcc gac tcg gcc gcc aat ctg gcc acc      48
   Met Ala Ile Leu Asn Pro Glu Ala Asp Ser Ala Ala Asn Leu Ala Thr
   1               5                   10                  15
   gac agc gag gcc aag cag cgc cag ctc gcg gag gcc ggc tac acg cac      96
   Asp Ser Glu Ala Lys Gln Arg Gln Leu Ala Glu Ala Gly Tyr Thr His
               20                  25                  30
   gtg gag ggc gcg ccg gcg cca ctg ccg ctg gag ctg ccg cac ttc tcg     144
   Val Glu Gly Ala Pro Ala Pro Leu Pro Leu Glu Leu Pro His Phe Ser
           35                  40                  45
   ctg cgc gac ctg cgc gcc gcc atc ccc aag cac tgc ttc gag cgc tcg     192
   Leu Arg Asp Leu Arg Ala Ala Ile Pro Lys His Cys Phe Glu Arg Ser
       50                  55                  60
   ttc gtc acg tcc acg tac tac atg atc aag aac gtg ctc acg tgc gcc     240
   Phe Val Thr Ser Thr Tyr Tyr Met Ile Lys Asn Val Leu Thr Cys Ala
   65                  70                  75                  80
   gcg ctc ttc tac gcg gcc acc ttc atc gac cgc gcg ggc gcc gcc gcc     288
   Ala Leu Phe Tyr Ala Ala Thr Phe Ile Asp Arg Ala Gly Ala Ala Ala
                   85                  90                  95
   tac gtg ctg tgg ccc gtg tac tgg ttc ttc cag ggc agc tac ctc acg     336
   Tyr Val Leu Trp Pro Val Tyr Trp Phe Phe Gln Gly Ser Tyr Leu Thr
               100                 105                 110
   ggc gtc tgg gtc atc gcg cac gag tgt ggc cac cag gcc tac tgc tcg     384
   Gly Val Trp Val Ile Ala His Glu Cys Gly His Gln Ala Tyr Cys Ser
   115 120 125
   agc gag gtc gtc aac aac ctc atc ggc ctc gta ctg cac tcg gcg ctg 432
   Ser Glu Val Val Asn Asn Leu Ile Gly Leu Val Leu His Ser Ala Leu
   130 135 140
   ctt gtg ccg tac cac agc tgg cgc atc tcg cac cgc aag cac cac tcc 480
   Leu Val Pro Tyr His Ser Trp Arg Ile Ser His Arg Lys His His Ser
   145 150 155 160
   aac acg ggc agc tgc gag aac gac gag gtg ttc gtg ccc gtg acc cgc 528
   Asn Thr Gly Ser Cys Glu Asn Asp Glu Val Phe Val Pro Val Thr Arg
   165 170 175
   tcg gtg ctc gcc agc tcc tgg aac gag acg ctc gag gac tcg ccg ctc 576
   Ser Val Leu Ala Ser Ser Trp Asn Glu Thr Leu Glu Asp Ser Pro Leu
   180 185 190
   tac cag ctc tac cgc atc gtg tac atg ctg gtc gtg ggc tgg atg ccc 624
   Tyr Gln Leu Tyr Arg Ile Val Tyr Met Leu Val Val Gly Trp Met Pro
   195 200 205
   ggc tac ctc ttc ttc aac gcc acg ggc ccg acc aag tac tgg ggc aag 672
   Gly Tyr Leu Phe Phe Asn Ala Thr Gly Pro Thr Lys Tyr Trp Gly Lys
   210 215 220
   tcg cgc agc cac ttc aac ccg tac tcg gcc atc tac gcc gac cgc gag 720
   Ser Arg Ser His Phe Asn Pro Tyr Ser Ala Ile Tyr Ala Asp Arg Glu
   225 230 235 240
   cgc tgg atg atc gtg ctg agc gac atc ttc ctc gtg gcc atg ctg gcc 768
   Arg Trp Met Ile Val Leu Ser Asp Ile Phe Leu Val Ala Met Leu Ala
   245 250 255
   gtg ctg gcc gcg ctc gtg cac acc ttc tcc ttc aac acc atg gtc aag 816
   Val Leu Ala Ala Leu Val His Thr Phe Ser Phe Asn Thr Met Val Lys
   260 265 270
   ttc tac gtc gtg ccc tac ttc atc gtc aac gcc tac ctc gtg ctt atc 864
   Phe Tyr Val Val Pro Tyr Phe Ile Val Asn Ala Tyr Leu Val Leu Ile
   275 280 285
   acg tac ctg cag cac acg gac acg tac atc ccg cac ttc cgc gag ggc 912
   Thr Tyr Leu Gln His Thr Asp Thr Tyr Ile Pro His Phe Arg Glu Gly
   290 295 300
   gag tgg aac tgg ctg cgc ggc gcg ctt tgc acg gtg gac cgg tcg ttc 960
   Glu Trp Asn Trp Leu Arg Gly Ala Leu Cys Thr Val Asp Arg Ser Phe
   305 310 315 320
   ggc ccg ttc ctc gac tcg gtg gtg cac cgc atc gtg gac acg cac gtg 1008
   Gly Pro Phe Leu Asp Ser Val Val His Arg Ile Val Asp Thr His Val
   325 330 335
   tgc cac cac atc ttc tcc aag atg ccg ttc tac cac tgc gag gag gcc 1056
   Cys His His Ile Phe Ser Lys Met Pro Phe Tyr His Cys Glu Glu Ala
   340 345 350
   acg aac gcc atc aag ccg ctg ctg ggc aag ttc tac ctc aag gac acg 1104
   Thr Asn Ala Ile Lys Pro Leu Leu Gly Lys Phe Tyr Leu Lys Asp Thr
   355 360 365
   acg ccc gtg ccc gtc gcg ctc tgg cgg tcc tac acg cac tgc aag ttc 1152
   Thr Pro Val Pro Val Ala Leu Trp Arg Ser Tyr Thr His Cys Lys Phe
   370 375 380
   gtc gag gac gac ggc aag gtc gtc ttc tac aag aac aag ctc taa 1197
   Val Glu Asp Asp Gly Lys Val Val Phe Tyr Lys Asn Lys Leu
   385 390 395
<210> 18
   <211> 398
   <212> PRT
   <213> Phytophtora sojae
<400> 18
   Met Ala Ile Leu Asn Pro Glu Ala Asp Ser Ala Ala Asn Leu Ala Thr
   1 5 10 15
   Asp Ser Glu Ala Lys Gln Arg Gln Leu Ala Glu Ala Gly Tyr Thr His
   20 25 30
   Val Glu Gly Ala Pro Ala Pro Leu Pro Leu Glu Leu Pro His Phe Ser
   35 40 45
   Leu Arg Asp Leu Arg Ala Ala Ile Pro Lys His Cys Phe Glu Arg Ser
   50 55 60
   Phe Val Thr Ser Thr Tyr Tyr Met Ile Lys Asn Val Leu Thr Cys Ala
   65 70 75 80
   Ala Leu Phe Tyr Ala Ala Thr Phe Ile Asp Arg Ala Gly Ala Ala Ala
   85 90 95
   Tyr Val Leu Trp Pro Val Tyr Trp Phe Phe Gln Gly Ser Tyr Leu Thr
   100 105 110
   Gly Val Trp Val Ile Ala His Glu Cys Gly His Gln Ala Tyr Cys Ser
   115 120 125
   Ser Glu Val Val Asn Asn Leu Ile Gly Leu Val Leu His Ser Ala Leu
   130 135 140
   Leu Val Pro Tyr His Ser Trp Arg Ile Ser His Arg Lys His His Ser
   145 150 155 160
   Asn Thr Gly Ser Cys Glu Asn Asp Glu Val Phe Val Pro Val Thr Arg
   165 170 175
   Ser Val Leu Ala Ser Ser Trp Asn Glu Thr Leu Glu Asp Ser Pro Leu
   180 185 190
   Tyr Gln Leu Tyr Arg Ile Val Tyr Met Leu Val Val Gly Trp Met Pro
   195 200 205
   Gly Tyr Leu Phe Phe Asn Ala Thr Gly Pro Thr Lys Tyr Trp Gly Lys
   210 215 220
   Ser Arg Ser His Phe Asn Pro Tyr Ser Ala Ile Tyr Ala Asp Arg Glu
   225 230 235 240
   Arg Trp Met Ile Val Leu Ser Asp Ile Phe Leu Val Ala Met Leu Ala
                   245                 250                 255
   Val Leu Ala Ala Leu Val His Thr Phe Ser Phe Asn Thr Met Val Lys
               260                 265                 270
   Phe Tyr Val Val Pro Tyr Phe Ile Val Asn Ala Tyr Leu Val Leu Ile
           275                 280                 285
   Thr Tyr Leu Gln His Thr Asp Thr Tyr Ile Pro His Phe Arg Glu Gly
       290                 295                 300
   Glu Trp Asn Trp Leu Arg Gly Ala Leu Cys Thr Val Asp Arg Ser Phe
   305                 310                 315                 320
   Gly Pro Phe Leu Asp Ser Val Val His Arg Ile Val Asp Thr His Val
                   325                 330                 335
   Cys His His Ile Phe Ser Lys Met Pro Phe Tyr His Cys Glu Glu Ala
               340                 345                 350
   Thr Asn Ala Ile Lys Pro Leu Leu Gly Lys Phe Tyr Leu Lys Asp Thr
           355                 360                 365
   Thr Pro Val Pro Val Ala Leu Trp Arg Ser Tyr Thr His Cys Lys Phe
       370                 375                 380
   Val Glu Asp Asp Gly Lys Val Val Phe Tyr Lys Asn Lys Leu
   385                 390                 395
<210> 19
   <211> 1560
   <212> DNA
   <213> Thraustochytrium ssp.
<220>
   <221> CDS
   <222> (1)..(1560)
<400> 19
   atg acg gtc ggc tac gac gag gag atc ccg ttc gag cag gtc cgc gcg      48
   Met Thr Val Gly Tyr Asp Glu Glu Ile Pro Phe Glu Gln Val Arg Ala
   1               5                   10                  15
   cac aac aag ccg gat gac gcc tgg tgc gcg atc cac ggg cac gtg tac      96
   His Asn Lys Pro Asp Asp Ala Trp Cys Ala Ile His Gly His Val Tyr
               20                  25                  30
   gat gtg acc aag ttc gcg agc gtg cac ccg ggc ggc gac att atc ctg     144
   Asp Val Thr Lys Phe Ala Ser Val His Pro Gly Gly Asp Ile Ile Leu
           35                  40                  45
   ctg gcc gca ggc aag gag gcc acc gtg ctg tac gag act tac cat gtg     192
   Leu Ala Ala Gly Lys Glu Ala Thr Val Leu Tyr Glu Thr Tyr His Val
       50                  55                  60
   cgg ggc gtc tcg gac gcg gtg ctg cgc aag tac cgc atc ggc aag ctg     240
   Arg Gly Val Ser Asp Ala Val Leu Arg Lys Tyr Arg Ile Gly Lys Leu
   65 70 75 80
   ccg gac ggc caa ggc ggc gcg aac gag aag gaa aag cgg acg ctc tcg 288
   Pro Asp Gly Gln Gly Gly Ala Asn Glu Lys Glu Lys Arg Thr Leu Ser
   85 90 95
   ggc ctc tcg tcg gcc tcg tac tac acg tgg aac agc gac ttt tac agg 336
   Gly Leu Ser Ser Ala Ser Tyr Tyr Thr Trp Asn Ser Asp Phe Tyr Arg
   100 105 110
   gta atg cgc gag cgc gtc gtg gct cgg ctc aag gag cgc ggc aag gcc 384
   Val Met Arg Glu Arg Val Val Ala Arg Leu Lys Glu Arg Gly Lys Ala
   115 120 125
   cgc cgc gga ggc tac gag ctc tgg atc aag gcg ttc ctg ctg ctc gtc 432
   Arg Arg Gly Gly Tyr Glu Leu Trp Ile Lys Ala Phe Leu Leu Leu Val
   130 135 140
   ggc ttc tgg agc tcg ctg tac tgg atg tgc acg ctg gac ccc tcg ttc 480
   Gly Phe Trp Ser Ser Leu Tyr Trp Met Cys Thr Leu Asp Pro Ser Phe
   145 150 155 160
   ggg gcc atc ctg gcc gcc atg tcg ctg ggc gtc ttt gcc gcc ttt gtg 528
   Gly Ala Ile Leu Ala Ala Met Ser Leu Gly Val Phe Ala Ala Phe Val
   165 170 175
   ggc acg tgc atc cag cac gac ggc aac cac ggc gcc ttt gcc cag tcg 576
   Gly Thr Cys Ile Gln His Asp Gly Asn His Gly Ala Phe Ala Gln Ser
   180 185 190
   cga tgg gtc aac aag gtt gcc ggg tgg acg ctc gac atg atc ggc gcc 624
   Arg Trp Val Asn Lys Val Ala Gly Trp Thr Leu Asp Met Ile Gly Ala
   195 200 205
   agc ggc atg acg tgg gag ttc cag cac gtc ctg ggc cac cat ccg tac 672
   Ser Gly Met Thr Trp Glu Phe Gln His Val Leu Gly His His Pro Tyr
   210 215 220
   acg aac ctg atc gag gag gag aac ggc ctg caa aag gtg agc ggc aag 720
   Thr Asn Leu Ile Glu Glu Glu Asn Gly Leu Gln Lys Val Ser Gly Lys
   225 230 235 240
   aag atg gac acc aag ctg gcc gac cag gag agc gat ccg gac gtc ttt 768
   Lys Met Asp Thr Lys Leu Ala Asp Gln Glu Ser Asp Pro Asp Val Phe
   245 250 255
   tcc acg tac ccg atg atg cgc ctg cac ccg tgg cac cag aag cgc tgg 816
   Ser Thr Tyr Pro Met Met Arg Leu His Pro Trp His Gln Lys Arg Trp
   260 265 270
   tac cac cgt ttc cag cac att tac ggc ccc ttc atc ttt ggc ttc atg 864
   Tyr His Arg Phe Gln His Ile Tyr Gly Pro Phe Ile Phe Gly Phe Met
   275 280 285
   acc atc aac aag gtg gtc acg cag gac gtc ggt gtg gtg ctc cgc aag 912
   Thr Ile Asn Lys Val Val Thr Gln Asp Val Gly Val Val Leu Arg Lys
   290 295 300
   cgg ctc ttc cag att gac gcc gag tgc cgg tac gcg agc cca atg tac 960
   Arg Leu Phe Gln Ile Asp Ala Glu Cys Arg Tyr Ala Ser Pro Met Tyr
   305 310 315 320
   gtg gcg cgt ttc tgg atc atg aag gcg ctc acg gtg ctc tac atg gtg 1008
   Val Ala Arg Phe Trp Ile Met Lys Ala Leu Thr Val Leu Tyr Met Val
   325 330 335
   gcc ctg ccg tgc tac atg cag ggc ccg tgg cac ggc ctc aag ctg ttc 1056
   Ala Leu Pro Cys Tyr Met Gln Gly Pro Trp His Gly Leu Lys Leu Phe
   340 345 350
   gcg atc gcg cac ttt acg tgc ggc gag gtg ctc gca acc atg ttc att 1104
   Ala Ile Ala His Phe Thr Cys Gly Glu Val Leu Ala Thr Met Phe Ile
   355 360 365
   gtg aac cac atc atc gag ggc gtc tcg tac gct tcc aag gac gcg gtc 1152
   Val Asn His Ile Ile Glu Gly Val Ser Tyr Ala Ser Lys Asp Ala Val
   370 375 380
   aag ggc acg atg gcg ccg ccg aag acg atg cac ggc gtg acg ccc atg 1200
   Lys Gly Thr Met Ala Pro Pro Lys Thr Met His Gly Val Thr Pro Met
   385 390 395 400
   aac aac acg cgc aag gag gtg gag gcg gag gcg tcc aag tct ggc gcc 1248
   Asn Asn Thr Arg Lys Glu Val Glu Ala Glu Ala Ser Lys Ser Gly Ala
   405 410 415
   gtg gtc aag tca gtc ccg ctc gac gac tgg gcc gcc gtc cag tgc cag 1296
   Val Val Lys Ser Val Pro Leu Asp Asp Trp Ala Ala Val Gln Cys Gln
   420 425 430
   acc tcg gtg aac tgg agc gtc ggc tcg tgg ttc tgg aat cac ttt tcc 1344
   Thr Ser Val Asn Trp Ser Val Gly Ser Trp Phe Trp Asn His Phe Ser
   435 440 445
   ggc ggc ctc aac cac cag att gag cac cac ctg ttc ccc ggg ctc agc 1392
   Gly Gly Leu Asn His Gln Ile Glu His His Leu Phe Pro Gly Leu Ser
   450 455 460
   cac gag acg tac tac cac atc cag gac gtc gtt cag tcc acc tgc gcc 1440
   His Glu Thr Tyr Tyr His Ile Gln Asp Val Val Gln Ser Thr Cys Ala
   465 470 475 480
   gag tac ggc gtc ccg tac cag cac gag cct tcg ctc tgg acc gcg tac 1488
   Glu Tyr Gly Val Pro Tyr Gln His Glu Pro Ser Leu Trp Thr Ala Tyr
   485 490 495
   tgg aag atg ctc gag cac ctc cgt cag ctc ggc aat gag gag acc cac 1536
   Trp Lys Met Leu Glu His Leu Arg Gln Leu Gly Asn Glu Glu Thr His
   500 505 510
   gag tcc tgg cag cgc gct gcc tga 1560
   Glu Ser Trp Gln Arg Ala Ala
   515
<210> 20
   <211> 519
   <212> PRT
   <213> Thraustochytrium ssp.
<400> 20
   Met Thr Val Gly Tyr Asp Glu Glu Ile Pro Phe Glu Gln Val Arg Ala
   1               5                   10                  15
   His Asn Lys Pro Asp Asp Ala Trp Cys Ala Ile His Gly His Val Tyr
               20                  25                  30
   Asp Val Thr Lys Phe Ala Ser Val His Pro Gly Gly Asp Ile Ile Leu
           35                  40                  45
   Leu Ala Ala Gly Lys Glu Ala Thr Val Leu Tyr Glu Thr Tyr His Val
       50                  55                  60
   Arg Gly Val Ser Asp Ala Val Leu Arg Lys Tyr Arg Ile Gly Lys Leu
   65                  70                  75                  80
   Pro Asp Gly Gln Gly Gly Ala Asn Glu Lys Glu Lys Arg Thr Leu Ser
                   85                  90                  95
   Gly Leu Ser Ser Ala Ser Tyr Tyr Thr Trp Asn Ser Asp Phe Tyr Arg
               100                 105                 110
   Val Met Arg Glu Arg Val Val Ala Arg Leu Lys Glu Arg Gly Lys Ala
           115                 120                 125
   Arg Arg Gly Gly Tyr Glu Leu Trp Ile Lys Ala Phe Leu Leu Leu Val
       130                 135                 140
   Gly Phe Trp Ser Ser Leu Tyr Trp Met Cys Thr Leu Asp Pro Ser Phe
   145                 150                 155                 160
   Gly Ala Ile Leu Ala Ala Met Ser Leu Gly Val Phe Ala Ala Phe Val
                   165                 170                 175
   Gly Thr Cys Ile Gln His Asp Gly Asn His Gly Ala Phe Ala Gln Ser
               180                 185                 190
   Arg Trp Val Asn Lys Val Ala Gly Trp Thr Leu Asp Met Ile Gly Ala
           195                 200                 205
   Ser Gly Met Thr Trp Glu Phe Gln His Val Leu Gly His His Pro Tyr
       210                 215                 220
   Thr Asn Leu Ile Glu Glu Glu Asn Gly Leu Gln Lys Val Ser Gly Lys
   225                 230                 235                 240
   Lys Met Asp Thr Lys Leu Ala Asp Gln Glu Ser Asp Pro Asp Val Phe
                   245                 250                 255
   Ser Thr Tyr Pro Met Met Arg Leu His Pro Trp His Gln Lys Arg Trp
               260                 265                 270
   Tyr His Arg Phe Gln His Ile Tyr Gly Pro Phe Ile Phe Gly Phe Met
           275                 280                 285
   Thr Ile Asn Lys Val Val Thr Gln Asp Val Gly Val Val Leu Arg Lys
       290                 295                 300
   Arg Leu Phe Gln Ile Asp Ala Glu Cys Arg Tyr Ala Ser Pro Met Tyr
   305                 310                 315                 320
   Val Ala Arg Phe Trp Ile Met Lys Ala Leu Thr Val Leu Tyr Met Val
                   325                 330                 335
   Ala Leu Pro Cys Tyr Met Gln Gly Pro Trp His Gly Leu Lys Leu Phe
               340                 345                 350
   Ala Ile Ala His Phe Thr Cys Gly Glu Val Leu Ala Thr Met Phe Ile
           355                 360                 365
   Val Asn His Ile Ile Glu Gly Val Ser Tyr Ala Ser Lys Asp Ala Val
       370                 375                 380
   Lys Gly Thr Met Ala Pro Pro Lys Thr Met His Gly Val Thr Pro Met
   385                 390                 395                 400
   Asn Asn Thr Arg Lys Glu Val Glu Ala Glu Ala Ser Lys Ser Gly Ala
                   405                 410                 415
   Val Val Lys Ser Val Pro Leu Asp Asp Trp Ala Ala Val Gln Cys Gln
               420                 425                 430
   Thr Ser Val Asn Trp Ser Val Gly Ser Trp Phe Trp Asn His Phe Ser
           435                 440                 445
   Gly Gly Leu Asn His Gln Ile Glu His His Leu Phe Pro Gly Leu Ser
       450                 455                 460
   His Glu Thr Tyr Tyr His Ile Gln Asp Val Val Gln Ser Thr Cys Ala
   465                 470                 475                 480
   Glu Tyr Gly Val Pro Tyr Gln His Glu Pro Ser Leu Trp Thr Ala Tyr
                   485                 490                 495
   Trp Lys Met Leu Glu His Leu Arg Gln Leu Gly Asn Glu Glu Thr His
               500                 505                 510
   Glu Ser Trp Gln Arg Ala Ala
           515
<210> 21
   <211> 18
   <212> DNA
   <213> artificial sequnce
<220>
   <223> primer sequence
<400> 21
   gtggaggccg ccatccag 18
<210> 22
   <211> 19
   <212> DNA
   <213> artificial sequence
<220>
   <223> primer sequnce
<400> 22
   cctgcacgtt catggtcac 19
<210> 23
   <211> 24
   <212> DNA
   <213> artificial sequence
<220>
   <223> primer sequence
<400> 23
   gcgagatctg gtggaagaag tatc 24
<210> 24
   <211> 20
   <212> DNA
   <213> artificial sequence
<220>
   <223> primer sequence
<400> 24
   agtgtagccg ttgcggtagg 20
<210> 25
   <211> 23
   <212> DNA
   <213> artificial sequence
<220>
   <223> primer sequence
<400> 25
   attagacaat ggcgaccgag atg 23
<210> 26
   <211> 24
   <212> DNA
   <213> artificial sequence
<220>
   <223> primer sequence
<400> 26
   gcattctaca cgctcttgtt cttc 24
<210> 27
   <211> 29
   <212> DNA
   <213> artificial sequence
<220>
   <223> primer sequence
<400> 27
   gtttacgatg gacattgatc aattgaagc 29
<210> 28
   <211> 25
   <212> DNA
   <213> artificial sequence
<220>
   <223> primer sequence
<400> 28
   gttcctaatc caccttcttt gcagc 25
<210> 29
   <211> 28
   <212> DNA
   <213> artificial sequence
<220>
   <223> primer sequence
<400> 29
   ggagcttgct acaattcttc catccatg 28
<210> 30
   <211> 23
   <212> DNA
   <213> artificial sequence
<220>
   <223> primer sequence
<400> 30
   atcccagtgg catgcacctg gta 23
<210> 31
   <211> 19
   <212> DNA
   <213> artificial sequence
<220>
   <223> primer sequence
<400> 31
   ctgcacaatg gcggatagc 19
<210> 32
   <211> 19
   <212> DNA
   <213> artificial sequence
<220>
   <223> primer sequence
<400> 32
   gactcctcag cgtcccatg 19
<210> 33
   <211> 20
   <212> DNA
   <213> artificial sequence
<220>
   <223> primer sequence
<400> 33
   ccatggcgga tagcccagtc 20
<210> 34
   <211> 20
   <212> DNA
   <213> artificial sequence
<220>
   <223> primer sequence
<400> 34
   tcagcgtccc atgccgacag 20
<210> 35
   <211> 31
   <212> DNA
   <213> artificial sequence
<220>
   <223> primer sequence
<400> 35
   gcggccgcgc catggtggac ctcaagcctg g 31
<210> 36
   <211> 27
   <212> DNA
   <213> artificial sequence
<220>
   <223> primer sequence
<400> 36
   gcggccgtta catcgctggg aactcgg 27
<210> 37
   <211> 31
   <212> DNA
   <213> artificial sequene
<220>
   <223> primer sequence
<400> 37
   gcggccgcgc catgggcaag ggcagcgagg g 31
<210> 38
   <211> 32
   <212> DNA
   <213> artificial sequence
<220>
   <223> primer sequence
<400> 38
   gcggccgcgc ctcagtcctg cttcttggtg tc 32
<210> 39
   <211> 30
   <212> DNA
   <213> artificial sequence
<220>
   <223> primer sequence
<400> 39
   gcggccgcgc catggcgatc ctgaacccgg 30
<210> 40
   <211> 27
   <212> DNA
   <213> artificial sequence
<220>
   <223> primer sequence
<400> 40
   gcggccgcta gagcttgttc ttgtaga 27
<210> 41
   <211> 33
   <212> DNA
   <213> artificial sequence
<220>
   <223> primer sequence
<400> 41
   gcggccgcgc catggcgacg aaggaggcgt atg 33
<210> 42
   <211> 31
   <212> DNA
   <213> artificial sequence
<220>
   <223> primer sequence
<400> 42
   gcggccgcgc cttacgtgga cttggtcttg g 31
<210> 43
   <211> 31
   <212> DNA
   <213> artificial sequence
<220>
   <223> primer sequence
<400> 43
   gcggccgcgc catgaagtca aagcgccaag c 31
<210> 44
   <211> 32
   <212> DNA
   <213> artificial sequence
<220>
   <223> primer sequence
<400> 44
   gcggccgcgc ccccgcgggg aaggctctat aa 32
<210> 45
   <211> 31
   <212> DNA
   <213> artificial sequence
<220>
   <223> primer sequence
<400> 45
   gcggccgcgc catggcgacc gagatgctgc a 31
<210> 46
   <211> 30
   <212> DNA
   <213> artificial sequence
<220>
   <223> primer sequence
<400> 46
   gcggccgcta cacgctcttg ttcttcttgc 30
<210> 47
   <211> 32
   <212> DNA
   <213> artificial sequence
<220>
   <223> primer sequence
<400> 47
   gcggccgcgc catggacatt gatcaattga ag 32
<210> 48
   <211> 33
   <212> DNA
   <213> artificial sequence
<220>
   <223> primer sequence
<400> 48
   gcggccgcgc ccaatggtga tggtgatgat gac 33
<210> 49
   <211> 32
   <212> DNA
   <213> artificial sequence
<220>
   <223> primer sequence
<400> 49
   gcggccgcgc catggcggat agcccagtca tc 32
<210> 50
   <211> 34
   <212> DNA
   <213> artificial sequence
<220>
   <223> primer sequence
<400> 50
   gcggccgcgc ctcagcgtcc catgccgaca gatc 34
<210> 51
   <211> 31
   <212> DNA
   <213> artificial sequence
<220>
   <223> primer sequence
<400> 51
   gcggccgcgc catgacggtc ggctacgacg a 31
<210> 52
   <211> 31
   <212> DNA
   <213> artificial sequence
<220>
   <223> primer sequence
<400> 52
   gcggccgcgc ctcaggcagc gcgctgccag g 31
<210> 53
   <211> 60
   <212> DNA
   <213> artificial sequence
<220>
   <223> primer sequence
<400> 53
   gtcgacccgc ggactagtgg gccctctaga cccgggggat ccggatctgc tggctatgaa 60